# EUROPEAN PATENT SPECIFICATION

(11) **EP 2 922 866 B1**
(45) Date of publication and mention of the grant of the patent: **23.01.2019**
(21) Application number: 13792398.3
(22) Date of filing: 20.11.2013
(51) Int. Cl.: C12Q 1/37, C07K 14/47

(54) **MEANS AND METHODS FOR DETERMINATION OF BOTULINUM NEUROTOXIN BIOLOGICAL ACTIVITY**
MITTEL UND VERFAHREN ZUR BESTIMMUNG DER BIOLOGISCHEN BOTULINNEUROTOXINAKTIVITÄT
MOYENS ET PROCÉDÉS DE DÉTERMINATION DE L'ACTIVITÉ BIOLOGIQUE DE LA NEUROTOXINE BOTULIQUE

(30) Priority: 21.11.2012 EP 12193560; 21.11.2012 US 201261729048 P
(43) Date of publication of application: 30.09.2015
(73) Proprietor: Merz Pharma GmbH & Co. KGaA, 60318 Frankfurt am Main (DE)
(72) Inventor: EISELE, Karl-Heinz, 60385 Frankfurt am Main (DE); FINK, Klaus, 50968 Köln (DE)
(74) Representative: Herzog, Fiesser & Partner Patentanwälte PartG mbB
(86) International application number: PCT/EP2013/074276
(87) International publication number: WO 2014/079878

(56) References cited:
- WO-A1-2012/123370
- WO-A2-2005/076785
- SABINE PELLETT ET AL: "Comparison of the primary rat spinal cord cell (RSC) assay and the mouse bioassay for botulinum neurotoxin type A potency determination", JOURNAL OF PHARMACOLOGICAL AND TOXICOLOGICAL METHODS, vol. 61, no. 3, 1 May 2010 (2010-05-01), pages 304-310, XP055049479, ISSN: 1056-8719, DOI: 10.1016/j.vascn.2010.01.003 cited in the application

### SEQUENCE LISTING

<110> Merz Pharma GmbH & Co. KGaA
<120> Means and methods for determination of Botulinum neurotoxin biological activity
<130> MP10485PC
<160> 38
<170> PatentIn version 3.3
<210> 1
   <211> 3258
   <212> DNA
   <213> artificial
<220>
   <223> SV2-SNAP25-tTA (2) (pcDNA3.1)
<400> 1
   gctagcgcca ccatggaaga ctcttacaag gataggactt cactgatgaa gggtgccaag     60
   gacattgcca gagaggtgaa gaaacaaaca gtaaagaagg tgaatcaagc tgtggaccga    120
   gcccaggatg aatacaccca gaggtcctac agtcggttcc aagatgaaga agatgatgat    180
   gactactacc cggctggaga aacctataat ggtgaggcca acgatgacga aggctcaagt    240
   gaagccactg aggggcatga tgaagatgat gagatctatg agggggagta tcagggcatc    300
   cccagtatga accaagcgaa ggacagcatc gtgtcagtgg ggcagcccaa gggcgatgag    360
   tacaaggacc gacgggagct ggaatcagaa aggagagctg acgaggaaga gttagcccag    420
   cagtatgagc tgataatcca agaatgcggt catggtcgtt ttcagtgggc ccttttcttc   480
   gtcctgggca tggctcttat ggcagacggt gtagaggtgt ttgtcgttgg cttcgtgtta    540
   cccagtgctg agacagacct ctgcatccca aattcaggat ctggatggct aggcagcata    600
   gtgtacctcg ggatgatggt gggggcgttc ttctggggag gactggcaga caaagtggga    660
   aggaaacagt ctcttctgat ttgcatgtct gtcaacggat tctttgcctt cctttcttca    720
   tttgtccaag gttatggctt ctttctcttc tgtcgcttac tttctggatt cgggattgga    780
   ggagccatac ccactgtgtt ctcgtacttt gctgaagtcc tggcccggga aaagcggggc    840
   gaacacttga gctggctctg catgttctgg atgatcggtg gcatctacgc ctctgccatg    900
   gcctgggcca tcatcccgca ctacgggtgg agcttcagca tgggatcggc ctaccagttt    960
   cacagttggc gtgtgtttgt catcgtctgt gcactcccct gtgtctcctc cgtggtggcc   1020
   ctcacattca tgcctgaaag cccacgattc ttgttggagg ttggaaaaca tgatgaagct   1080
   tggatgattc tgaagttaat tcatgacacc aacatgagag cccggggtca gcctgagaag   1140
   gtcttcacgg taaacaaaat aaaaactcct aaacaaatag atgagctgat tgaaattgag   1200
   agtgacacag gaacatggta taggaggtgt tttgttcgga tccgcaccga gctgtacgga   1260
   atttggttga cttttatgag atgtttcaac tacccagtca gggataatac aataaagctt 1320
   acaattgttt ggttcaccct gtcctttggg tactatggat tatccgtttg gttccctgat 1380
   gtcattaaac ctctgcagtc cgatgaatat gcattgctaa ccagaaatgt ggagagagat 1440
   aaatatgcaa atttcactat taactttaca atggaaaatc agattcatac tggaatggaa 1500
   tacgacaatg gcagattcat aggggtcaag ttcaaatctg taactttcaa agactctgtt 1560
   tttaagtcct gcacctttga ggatgtaact tcagtgaaca cctacttcaa gaactgcaca 1620
   tttattgaca ctgtttttga caacacagat tttgagccat ataaattcat tgacagtgaa 1680
   tttaaaaact gctcgttttt tcacaacaag acgggatgtc agattacctt tgatgatgac 1740
   tatagtgcct actggattta ttttgtcaac tttctgggga cattggcagt attgccaggg 1800
   aacattgtgt ctgctctgct gatggacaga attgggcgct taacaatgct aggtggctct 1860
   atggtgcttt cggggatcag ctgtttcttc ctttggttcg gcaccagtga atccatgatg 1920
   ataggcatgc tgtgtctgta caatggattg accatctcag cctggaactc tcttgacgtg 1980
   gtcactgtgg aactgtaccc cacagaccgg agggcaacag gctttggctt cttaaatgcg 2040
   ctatgcaagg cagcagccgt cctgggaaac ttaatatttg gctctctggt cagcatcacc 2100
   aaatcaatcc ccatcctgct ggcttctact gtgctcgtgt gtggaggact cgttgggctg 2160
   tgcctgcctg acacacgatc aagtgatgct tacaaaaaag cctggggcaa taatcaggac 2220
   ggagtggtgg ccagccagcc tgctcgtgta gtggacgaac gggagcagat ggccatcagt 2280
   ggcggcttca tccgcagggt aacaaatgat gcccgagaaa atgaaatgga tgaaaaccta 2340
   gagcaggtga gcggcatcat cgggaacctc cgtcacatgg ccctggatat gggcaatgag 2400
   atcgatacac agaatcgcca gatcgacagg atcatggaga aggctgattc caacaaaacc 2460
   agaattgatg aggccaacca acgtgcaaca aagatgctgg gaagtggttc tagactggac 2520
   aagagcaaag tcataaactc tgctctggaa ttactcaatg gagtcggtat cgaaggcctg 2580
   acgacaagga aactcgctca aaagctggga gttgagcagc ctaccctgta ctggcacgtg 2640
   aagaacaagc gggccctgct cgatgccctg ccaatcgaga tgctggacag gcatcatacc 2700
   cactcctgcc ccctggaagg cgagtcatgg caagactttc tgcggaacaa cgccaagtca 2760
   taccgctgtg ctctcctctc acatcgcgac ggggctaaag tgcatctcgg cacccgccca 2820
   acagagaaac agtacgaaac cctggaaaat cagctcgcgt tcctgtgtca gcaaggcttc 2880
   tccctggaga acgcactgta cgctctgtcc gccgtgggcc actttacact gggctgcgta 2940
   ttggaggaac aggagcatca agtagcaaaa gaggaaagag agacacctac caccgattct 3000
   atgcccccac ttctgaaaca agcaattgag ctgttcgacc ggcagggagc cgaacctgcc 3060
   ttccttttcg gcctggaact aatcatatgt ggcctggaga aacagctaaa gtgcgaaagc 3120
   ggcgggccga ccgacgccct tgacgatttt gacttagaca tgctcccagc cgatgccctt 3180
   gacgactttg accttgatat gctgcctgct gacgctcttg acgattttga ccttgacatg 3240
   ctccccgggt aactcgag 3258
<210> 2
   <211> 1079
   <212> PRT
   <213> artificial
<220>
   <223> SV2-SNAP25-tTA (2) (pcDNA3.1)
<400> 2
   Met Glu Asp Ser Tyr Lys Asp Arg Thr Ser Leu Met Lys Gly Ala Lys
   1               5                   10                  15
   Asp Ile Ala Arg Glu Val Lys Lys Gln Thr Val Lys Lys Val Asn Gln
               20                  25                  30
   Ala Val Asp Arg Ala Gln Asp Glu Tyr Thr Gln Arg Ser Tyr Ser Arg
           35                  40                  45
   Phe Gln Asp Glu Glu Asp Asp Asp Asp Tyr Tyr Pro Ala Gly Glu Thr
       50                  55                  60
   Tyr Asn Gly Glu Ala Asn Asp Asp Glu Gly Ser Ser Glu Ala Thr Glu
   65                  70                  75                  80
   Gly His Asp Glu Asp Asp Glu Ile Tyr Glu Gly Glu Tyr Gln Gly Ile
                   85                  90                  95
   Pro Ser Met Asn Gln Ala Lys Asp Ser Ile Val Ser Val Gly Gln Pro
               100                 105                 110
   Lys Gly Asp Glu Tyr Lys Asp Arg Arg Glu Leu Glu Ser Glu Arg Arg
           115                 120                 125
   Ala Asp Glu Glu Glu Leu Ala Gln Gln Tyr Glu Leu Ile Ile Gln Glu
       130                 135                 140
   Cys Gly His Gly Arg Phe Gln Trp Ala Leu Phe Phe Val Leu Gly Met
   145                 150                 155                 160
   Ala Leu Met Ala Asp Gly Val Glu Val Phe Val Val Gly Phe Val Leu
                   165                 170                 175
   Pro Ser Ala Glu Thr Asp Leu Cys Ile Pro Asn Ser Gly Ser Gly Trp
               180                 185                 190
   Leu Gly Ser Ile Val Tyr Leu Gly Met Met Val Gly Ala Phe Phe Trp
           195                 200                 205
   Gly Gly Leu Ala Asp Lys Val Gly Arg Lys Gln Ser Leu Leu Ile Cys
       210                 215                 220
   Met Ser Val Asn Gly Phe Phe Ala Phe Leu Ser Ser Phe Val Gln Gly
   225                 230                 235                 240
   Tyr Gly Phe Phe Leu Phe Cys Arg Leu Leu Ser Gly Phe Gly Ile Gly
                   245                 250                 255
   Gly Ala Ile Pro Thr Val Phe Ser Tyr Phe Ala Glu Val Leu Ala Arg
               260                 265                 270
   Glu Lys Arg Gly Glu His Leu Ser Trp Leu Cys Met Phe Trp Met Ile
           275                 280                 285
   Gly Gly Ile Tyr Ala Ser Ala Met Ala Trp Ala Ile Ile Pro His Tyr
       290                 295                 300
   Gly Trp Ser Phe Ser Met Gly Ser Ala Tyr Gln Phe His Ser Trp Arg
   305                 310                 315                 320
   Val Phe Val Ile Val Cys Ala Leu Pro Cys Val Ser Ser Val Val Ala
                   325                 330                 335
   Leu Thr Phe Met Pro Glu Ser Pro Arg Phe Leu Leu Glu Val Gly Lys
               340                 345                 350
   His Asp Glu Ala Trp Met Ile Leu Lys Leu Ile His Asp Thr Asn Met
           355                 360                 365
   Arg Ala Arg Gly Gln Pro Glu Lys Val Phe Thr Val Asn Lys Ile Lys
       370                 375                 380
   Thr Pro Lys Gln Ile Asp Glu Leu Ile Glu Ile Glu Ser Asp Thr Gly
   385                 390                 395                 400
   Thr Trp Tyr Arg Arg Cys Phe Val Arg Ile Arg Thr Glu Leu Tyr Gly
                   405                 410                 415
   Ile Trp Leu Thr Phe Met Arg Cys Phe Asn Tyr Pro Val Arg Asp Asn
               420                 425                 430
   Thr Ile Lys Leu Thr Ile Val Trp Phe Thr Leu Ser Phe Gly Tyr Tyr
           435                 440                 445
   Gly Leu Ser Val Trp Phe Pro Asp Val Ile Lys Pro Leu Gln Ser Asp
       450                 455                 460
   Glu Tyr Ala Leu Leu Thr Arg Asn Val Glu Arg Asp Lys Tyr Ala Asn
   465                 470                 475                 480
   Phe Thr Ile Asn Phe Thr Met Glu Asn Gln Ile His Thr Gly Met Glu
                   485                 490                 495
   Tyr Asp Asn Gly Arg Phe Ile Gly Val Lys Phe Lys Ser Val Thr Phe
               500                 505                 510
   Lys Asp Ser Val Phe Lys Ser Cys Thr Phe Glu Asp Val Thr Ser Val
           515                 520                 525
   Asn Thr Tyr Phe Lys Asn Cys Thr Phe Ile Asp Thr Val Phe Asp Asn
       530                 535                 540
   Thr Asp Phe Glu Pro Tyr Lys Phe Ile Asp Ser Glu Phe Lys Asn Cys
   545                 550                 555                 560
   Ser Phe Phe His Asn Lys Thr Gly Cys Gln Ile Thr Phe Asp Asp Asp
                   565                 570                 575
   Tyr Ser Ala Tyr Trp Ile Tyr Phe Val Asn Phe Leu Gly Thr Leu Ala
               580                 585                 590
   Val Leu Pro Gly Asn Ile Val Ser Ala Leu Leu Met Asp Arg Ile Gly
           595                 600                 605
   Arg Leu Thr Met Leu Gly Gly Ser Met Val Leu Ser Gly Ile Ser Cys
       610                 615                 620
   Phe Phe Leu Trp Phe Gly Thr Ser Glu Ser Met Met Ile Gly Met Leu
   625                 630                 635                 640
   Cys Leu Tyr Asn Gly Leu Thr Ile Ser Ala Trp Asn Ser Leu Asp Val
                   645                 650                 655
   Val Thr Val Glu Leu Tyr Pro Thr Asp Arg Arg Ala Thr Gly Phe Gly
               660                 665                 670
   Phe Leu Asn Ala Leu Cys Lys Ala Ala Ala Val Leu Gly Asn Leu Ile
           675                 680                 685
   Phe Gly Ser Leu Val Ser Ile Thr Lys Ser Ile Pro Ile Leu Leu Ala
       690                 695                 700
   Ser Thr Val Leu Val Cys Gly Gly Leu Val Gly Leu Cys Leu Pro Asp
   705                 710                 715                 720
   Thr Arg Ser Ser Asp Ala Tyr Lys Lys Ala Trp Gly Asn Asn Gln Asp
                   725                 730                 735
   Gly Val Val Ala Ser Gln Pro Ala Arg Val Val Asp Glu Arg Glu Gln
               740                 745                 750
   Met Ala Ile Ser Gly Gly Phe Ile Arg Arg Val Thr Asn Asp Ala Arg
           755                 760                 765
   Glu Asn Glu Met Asp Glu Asn Leu Glu Gln Val Ser Gly Ile Ile Gly
       770                 775                 780
   Asn Leu Arg His Met Ala Leu Asp Met Gly Asn Glu Ile Asp Thr Gln
   785                 790                 795                 800
   Asn Arg Gln Ile Asp Arg Ile Met Glu Lys Ala Asp Ser Asn Lys Thr
                   805                 810                 815
   Arg Ile Asp Glu Ala Asn Gln Arg Ala Thr Lys Met Leu Gly Ser Gly
               820                 825                 830
   Ser Arg Leu Asp Lys Ser Lys Val Ile Asn Ser Ala Leu Glu Leu Leu
           835                 840                 845
   Asn Gly Val Gly Ile Glu Gly Leu Thr Thr Arg Lys Leu Ala Gln Lys
       850                 855                 860
   Leu Gly Val Glu Gln Pro Thr Leu Tyr Trp His Val Lys Asn Lys Arg
   865                 870                 875                 880
   Ala Leu Leu Asp Ala Leu Pro Ile Glu Met Leu Asp Arg His His Thr
                   885                 890                 895
   His Ser Cys Pro Leu Glu Gly Glu Ser Trp Gln Asp Phe Leu Arg Asn
               900                 905                 910
   Asn Ala Lys Ser Tyr Arg Cys Ala Leu Leu Ser His Arg Asp Gly Ala
           915                 920                 925
   Lys Val His Leu Gly Thr Arg Pro Thr Glu Lys Gln Tyr Glu Thr Leu
       930                 935                 940
   Glu Asn Gln Leu Ala Phe Leu Cys Gln Gln Gly Phe Ser Leu Glu Asn
   945                 950                 955                 960
   Ala Leu Tyr Ala Leu Ser Ala Val Gly His Phe Thr Leu Gly Cys Val
                   965                 970                 975
   Leu Glu Glu Gln Glu His Gln Val Ala Lys Glu Glu Arg Glu Thr Pro
               980                 985                 990
   Thr Thr Asp Ser Met Pro Pro Leu  Leu Lys Gln Ala Ile  Glu Leu Phe
           995                 1000                1005
   Asp Arg  Gln Gly Ala Glu Pro  Ala Phe Leu Phe Gly  Leu Glu Leu
       1010                1015                1020
   Ile Ile  Cys Gly Leu Glu Lys  Gln Leu Lys Cys Glu  Ser Gly Gly
       1025                1030                1035
   Pro Thr  Asp Ala Leu Asp Asp  Phe Asp Leu Asp Met  Leu Pro Ala
       1040                1045                1050
   Asp Ala  Leu Asp Asp Phe Asp  Leu Asp Met Leu Pro  Ala Asp Ala
       1055                1060                1065
   Leu Asp  Asp Phe Asp Leu Asp  Met Leu Pro Gly
       1070                1075
<210> 3
   <211> 2814
   <212> DNA
   <213> artificial
<220>
   <223> SV2-SNAP25-tTA (3) (pCMV-Tet3G)
<400> 3
   gaattcacca tggaagactc ttacaaggat aggacttcac tgatgaaggg tgccaaggac      60
   attgccagag aggtgaagaa acaaacagta aagaaggtga atcaagctgt ggaccgagcc     120
   caggatgaat acacccagag gtcctacagt cggttccaag atgaagaaga tgatgatgac     180
   tactacccgg ctggagaaac ctataatggt gaggccaacg atgacgaagg ctcaagtgaa     240
   gccactgagg ggcatgatga agatgatgag atctatgagg gggagtatca gggcatcccc     300
   agtatgaacc aagcgaagga cagcatcgtg tcagtggggc agcccaaggg cgatgagtac     360
   aaggaccgac gggagctgga atcagaaagg agagctgacg aggaagagtt agcccagcag     420
   tatgagctga taatccaaga atgcggtcat ggtcgttttc agtgggccct tttcttcgtc     480
   ctgggcatgg ctcttatggc agacggtgta gaggtgtttg tcgttggctt cgtgttaccc     540
   agtgctgaga cagacctctg catcccaaat tcaggatctg gatggctagg cagcatagtg 600
   tacctcggga tgatggtggg ggcgttcttc tggggaggac tggcagacaa agtgggaagg 660
   aaacagtctc ttctgatttg catgtctgtc aacggattct ttgccttcct ttcttcattt 720
   gtccaaggtt atggcttctt tctcttctgt cgcttacttt ctggattcgg gattggagga 780
   gccataccca ctgtgttctc gtactttgct gaagtcctgg cccgggaaaa gcggggcgaa 840
   cacttgagct ggctctgcat gttctggatg atcggtggca tctacgcctc tgccatggcc 900
   tgggccatca tcccgcacta cgggtggagc ttcagcatgg gatcggccta ccagtttcac 960
   agttggcgtg tgtttgtcat cgtctgtgca ctcccctgtg tctcctccgt ggtggccctc 1020
   acattcatgc ctgaaagccc acgattcttg ttggaggttg gaaaacatga tgaagcttgg 1080
   atgattctga agttaattca tgacaccaac atgagagccc ggggtcagcc tgagaaggtc 1140
   ttcacggtaa acaaaataaa aactcctaaa caaatagatg agctgattga aattgagagt 1200
   gacacaggaa catggtatag gaggtgtttt gttcggatcc gcaccgagct gtacggaatt 1260
   tggttgactt ttatgagatg tttcaactac ccagtcaggg ataatacaat aaagcttaca 1320
   attgtttggt tcaccctgtc ctttgggtac tatggattat ccgtttggtt ccctgatgtc 1380
   attaaacctc tgcagtccga tgaatatgca ttgctaacca gaaatgtgga gagagataaa 1440
   tatgcaaatt tcactattaa ctttacaatg gaaaatcaga ttcatactgg aatggaatac 1500
   gacaatggca gattcatagg ggtcaagttc aaatctgtaa ctttcaaaga ctctgttttt 1560
   aagtcctgca cctttgagga tgtaacttca gtgaacacct acttcaagaa ctgcacattt 1620
   attgacactg tttttgacaa cacagatttt gagccatata aattcattga cagtgaattt 1680
   aaaaactgct cgttttttca caacaagacg ggatgtcaga ttacctttga tgatgactat 1740
   agtgcctact ggatttattt tgtcaacttt ctggggacat tggcagtatt gccagggaac 1800
   attgtgtctg ctctgctgat ggacagaatt gggcgcttaa caatgctagg tggctctatg 1860
   gtgctttcgg ggatcagctg tttcttcctt tggttcggca ccagtgaatc catgatgata 1920
   ggcatgctgt gtctgtacaa tggattgacc atctcagcct ggaactctct tgacgtggtc 1980
   actgtggaac tgtaccccac agaccggagg gcaacaggct ttggcttctt aaatgcgcta 2040
   tgcaaggcag cagccgtcct gggaaactta atatttggct ctctggtcag catcaccaaa 2100
   tcaatcccca tcctgctggc ttctactgtg ctcgtgtgtg gaggactcgt tgggctgtgc 2160
   ctgcctgaca cacgaaccca ggttctgatg gccgaagacg cagacatgcg caatgagctg 2220
   gaggagatgc agcgaagggc tgaccagttg gctgatgagt cgctggaaag cacccgtcgt 2280
   atgctgcaac tggttgaaga gagtaaagat gctggtatca ggactttggt tatgttggat 2340
   gaacaaggag aacaactgga acgcattgag gaagggatgg accaaatcaa taaggacatg 2400
   aaagaagcag aaaagaattt gacggaccta ggaaaattct gcgggctttg tgtgtgtccc   2460
   tgtaacaagc ttaaatcaag tgatgcttac aaaaaagcct ggggcaataa tcaggacgga   2520
   gtggtggcca gccagcctgc tcgtgtagtg gacgaacggg agcagatggc catcagtggc   2580
   ggcttcatcc gcagggtaac aaatgatgcc cgagaaaatg aaatggatga aaacctagag   2640
   caggtgagcg gcatcatcgg gaacctccgt cacatggccc tggatatggg caatgagatc   2700
   gatacacaga atcgccagat cgacaggatc atggagaagg ctgattccaa caaaaccaga   2760
   attgatgagg ccaaccaacg tgcaacaaag atgctgggaa gtggttaatc taga         2814
<210> 4
   <211> 1179
   <212> PRT
   <213> artificial
<220>
   <223> SV2-SNAP25-tTA (3) (pCMV-Tet3G)
<400> 4
<210> 5
   <211> 1725
   <212> DNA
   <213> artificial
<220>
   <223> Cholinetransporter-SNAP25-tTA (1)(pCMV-Tet3G)
<400> 5
   gaattcacca tggctttcca tgtggaagga ctgatagcta tcatcgtgtt ctaccttcta     60
   attttgctgg ttggaatatg ggctgcctgg agaaccaaaa acagtggcag cgcagaagag    120
   cgcagcgaag ccatcatagt tggtggccga gatattggtt tattggttgg tggatttacc    180
   atgacagcta cctgggtcgg aggagggtat atcaatggca cagctgaagc agtttatgta    240
   ccaggttatg gcctagcttg ggctcaggca ccaattggat attctcttag tctgatttta    300
   ggtggcctgt tctttgcaaa acctatgcgt tcaaaggggt atgtgaccat gttagacccg    360
   tttcagcaaa tctatggaaa acgcatgggc ggactcctgt ttattcctgc actgatggga    420
   gaaatgttct gggctgcagc aattttctct gctttgggag ccaccatcag cgtgatcatc    480
   gatgtggata tgcacatttc tgtcatcatc tctgcactca ttgccactct gtacacactg    540
   gtgggagggc tctattctgt ggcctacact gatgtcgttc agctcttttg catttttgta    600
   gggctgtgga tcagcgtccc ctttgcattg tcacatcctg cagtcgcaga catcgggttc    660
   actgctgtgc atgccaaata ccaaaagccg tggctgggaa ctgttgactc atctgaagtc    720
   tactcttggc ttgatagttt tctgttgttg atgctgggtg gaatcccatg gcaagcatac    780
   tttcagaggg ttctctcttc ttcctcagcc acctatgctc aagtgctgtc cttcctggca    840
   gctttcgggt gcctggtgat ggccatccca gccatactca ttggggccat tggagcatca    900
   acagactgga accagactgc atatgggctt ccagatccca agactacaga agaggcagac    960
   atgattttac caattgttct gcagtatctc tgccctgtgt atatttcttt ctttggtctt   1020
   ggtgcagttt ctgctgctgt tatgtcatca gcagattctt ccatcttgtc agcaagttcc   1080
   atgtttgcac ggaacatcta ccagctttcc ttcagacaaa atgcttcgga caaagaaatc   1140
   gtttgggtta tgcgaatcac agtgtttgtg tttggagcat ctgcaacagc catggccttg   1200
   ctgacgaaaa ctgtgtatgg gctctggtac ctcagttctg accttgttta catcgttatc   1260
   ttcccccagc tgctttgtgt actctttgtt aagggaacca acacctatgg ggccgtggca   1320
   ggttatgttt ctggcctctt cctgagaata actggagggg agccatatct gtatcttaac   1380
   aagcttaaat caagtgatgc ttacaaaaaa gcctggggca ataatcagga cggagtggtg   1440
   gccagccagc ctgctcgtgt agtggacgaa cgggagcaga tggccatcag tggcggcttc   1500
   atccgcaggg taacaaatga tgcccgagaa aatgaaatgg atgaaaacct agagcaggtg   1560
   agcggcatca tcgggaacct ccgtcacatg gccctggata tgggcaatga gatcgataca   1620
   cagaatcgcc agatcgacag gatcatggag aaggctgatt ccaacaaaac cagaattgat   1680
   gaggccaacc aacgtgcaac aaagatgctg ggaagtggtt ctaga                    1725
<210> 6
   <211> 817
   <212> PRT
   <213> artificial
<220>
   <223> Cholinetransporter-SNAP25-tTA (1)(pCMV-Tet3G)
<400> 6
   Met Ala Phe His Val Glu Gly Leu Ile Ala Ile Ile Val Phe Tyr Leu
   1               5                   10                  15
   Leu Ile Leu Leu Val Gly Ile Trp Ala Ala Trp Arg Thr Lys Asn Ser
               20                  25                  30
   Gly Ser Ala Glu Glu Arg Ser Glu Ala Ile Ile Val Gly Gly Arg Asp
           35                  40                  45
   Ile Gly Leu Leu Val Gly Gly Phe Thr Met Thr Ala Thr Trp Val Gly
       50                  55                  60
   Gly Gly Tyr Ile Asn Gly Thr Ala Glu Ala Val Tyr Val Pro Gly Tyr
   65                  70                  75                  80
   Gly Leu Ala Trp Ala Gln Ala Pro Ile Gly Tyr Ser Leu Ser Leu Ile
                   85                  90                  95
   Leu Gly Gly Leu Phe Phe Ala Lys Pro Met Arg Ser Lys Gly Tyr Val
               100                 105                 110
   Thr Met Leu Asp Pro Phe Gln Gln Ile Tyr Gly Lys Arg Met Gly Gly
           115                 120                 125
   Leu Leu Phe Ile Pro Ala Leu Met Gly Glu Met Phe Trp Ala Ala Ala
       130                 135                 140
   Ile Phe Ser Ala Leu Gly Ala Thr Ile Ser Val Ile Ile Asp Val Asp
   145                 150                 155                 160
   Met His Ile Ser Val Ile Ile Ser Ala Leu Ile Ala Thr Leu Tyr Thr
   165 170 175
   Leu Val Gly Gly Leu Tyr Ser Val Ala Tyr Thr Asp Val Val Gln Leu
   180 185 190
   Phe Cys Ile Phe Val Gly Leu Trp Ile Ser Val Pro Phe Ala Leu Ser
   195 200 205
   His Pro Ala Val Ala Asp Ile Gly Phe Thr Ala Val His Ala Lys Tyr
   210 215 220
   Gln Lys Pro Trp Leu Gly Thr Val Asp Ser Ser Glu Val Tyr Ser Trp
   225 230 235 240
   Leu Asp Ser Phe Leu Leu Leu Met Leu Gly Gly Ile Pro Trp Gln Ala
   245 250 255
   Tyr Phe Gln Arg Val Leu Ser Ser Ser Ser Ala Thr Tyr Ala Gln Val
   260 265 270
   Leu Ser Phe Leu Ala Ala Phe Gly Cys Leu Val Met Ala Ile Pro Ala
   275 280 285
   Ile Leu Ile Gly Ala Ile Gly Ala Ser Thr Asp Trp Asn Gln Thr Ala
   290 295 300
   Tyr Gly Leu Pro Asp Pro Lys Thr Thr Glu Glu Ala Asp Met Ile Leu
   305 310 315 320
   Pro Ile Val Leu Gln Tyr Leu Cys Pro Val Tyr Ile Ser Phe Phe Gly
   325 330 335
   Leu Gly Ala Val Ser Ala Ala Val Met Ser Ser Ala Asp Ser Ser Ile
   340 345 350
   Leu Ser Ala Ser Ser Met Phe Ala Arg Asn Ile Tyr Gln Leu Ser Phe
   355 360 365
   Arg Gln Asn Ala Ser Asp Lys Glu Ile Val Trp Val Met Arg Ile Thr
   370 375 380
   Val Phe Val Phe Gly Ala Ser Ala Thr Ala Met Ala Leu Leu Thr Lys
   385 390 395 400
   Thr Val Tyr Gly Leu Trp Tyr Leu Ser Ser Asp Leu Val Tyr Ile Val
   405 410 415
   Ile Phe Pro Gln Leu Leu Cys Val Leu Phe Val Lys Gly Thr Asn Thr
   420 425 430
   Tyr Gly Ala Val Ala Gly Tyr Val Ser Gly Leu Phe Leu Arg Ile Thr
   435 440 445
   Gly Gly Glu Pro Tyr Leu Tyr Leu Asn Lys Leu Lys Ser Ser Asp Ala
   450 455 460
   Tyr Lys Lys Ala Trp Gly Asn Asn Gln Asp Gly Val Val Ala Ser Gln
   465 470 475 480
   Pro Ala Arg Val Val Asp Glu Arg Glu Gln Met Ala Ile Ser Gly Gly
   485 490 495
   Phe Ile Arg Arg Val Thr Asn Asp Ala Arg Glu Asn Glu Met Asp Glu
   500 505 510
   Asn Leu Glu Gln Val Ser Gly Ile Ile Gly Asn Leu Arg His Met Ala
   515 520 525
   Leu Asp Met Gly Asn Glu Ile Asp Thr Gln Asn Arg Gln Ile Asp Arg
   530 535 540
   Ile Met Glu Lys Ala Asp Ser Asn Lys Thr Arg Ile Asp Glu Ala Asn
   545 550 555 560
   Gln Arg Ala Thr Lys Met Leu Gly Ser Gly Ser Arg Leu Asp Lys Ser
   565 570 575
   Lys Val Ile Asn Ser Ala Leu Glu Leu Leu Asn Gly Val Gly Ile Glu
   580 585 590
   Gly Leu Thr Thr Arg Lys Leu Ala Gln Lys Leu Gly Val Glu Gln Pro
   595 600 605
   Thr Leu Tyr Trp His Val Lys Asn Lys Arg Ala Leu Leu Asp Ala Leu
   610 615 620
   Pro Ile Glu Met Leu Asp Arg His His Thr His Ser Cys Pro Leu Glu
   625 630 635 640
   Gly Glu Ser Trp Gln Asp Phe Leu Arg Asn Asn Ala Lys Ser Tyr Arg
   645 650 655
   Cys Ala Leu Leu Ser His Arg Asp Gly Ala Lys Val His Leu Gly Thr
               660                 665                 670
   Arg Pro Thr Glu Lys Gln Tyr Glu Thr Leu Glu Asn Gln Leu Ala Phe
           675                 680                 685
   Leu Cys Gln Gln Gly Phe Ser Leu Glu Asn Ala Leu Tyr Ala Leu Ser
       690                 695                 700
   Ala Val Gly His Phe Thr Leu Gly Cys Val Leu Glu Glu Gln Glu His
   705                 710                 715                 720
   Gln Val Ala Lys Glu Glu Arg Glu Thr Pro Thr Thr Asp Ser Met Pro
                   725                 730                 735
   Pro Leu Leu Lys Gln Ala Ile Glu Leu Phe Asp Arg Gln Gly Ala Glu
               740                 745                 750
   Pro Ala Phe Leu Phe Gly Leu Glu Leu Ile Ile Cys Gly Leu Glu Lys
           755                 760                 765
   Gln Leu Lys Cys Glu Ser Gly Gly Pro Thr Asp Ala Leu Asp Asp Phe
       770                 775                 780
   Asp Leu Asp Met Leu Pro Ala Asp Ala Leu Asp Asp Phe Asp Leu Asp
   785                 790                 795                 800
   Met Leu Pro Ala Asp Ala Leu Asp Asp Phe Asp Leu Asp Met Leu Pro
                   805                 810                 815
   Gly
<210> 7
   <211> 2370
   <212> DNA
   <213> artificial
<220>
   <223> Cholinetransporter-SNAP25-tTA (3)(pCMV-Tet3G)
<400> 7
   gaattcacca tggctttcca tgtggaagga ctgatagcta tcatcgtgtt ctaccttcta     60
   attttgctgg ttggaatatg ggctgcctgg agaaccaaaa acagtggcag cgcagaagag    120
   cgcagcgaag ccatcatagt tggtggccga gatattggtt tattggttgg tggatttacc    180
   atgacagcta cctgggtcgg aggagggtat atcaatggca cagctgaagc agtttatgta    240
   ccaggttatg gcctagcttg ggctcaggca ccaattggat attctcttag tctgatttta    300
   ggtggcctgt tctttgcaaa acctatgcgt tcaaaggggt atgtgaccat gttagacccg    360
   tttcagcaaa tctatggaaa acgcatgggc ggactcctgt ttattcctgc actgatggga    420
   gaaatgttct gggctgcagc aattttctct gctttgggag ccaccatcag cgtgatcatc    480
   gatgtggata tgcacatttc tgtcatcatc tctgcactca ttgccactct gtacacactg    540
   gtgggagggc tctattctgt ggcctacact gatgtcgttc agctcttttg catttttgta    600
   gggctgtgga tcagcgtccc ctttgcattg tcacatcctg cagtcgcaga catcgggttc    660
   actgctgtgc atgccaaata ccaaaagccg tggctgggaa ctgttgactc atctgaagtc    720
   tactcttggc ttgatagttt tctgttgttg atgctgggtg gaatcccatg gcaagcatac    780
   tttcagaggg ttctctcttc ttcctcagcc acctatgctc aagtgctgtc cttcctggca    840
   gctttcgggt gcctggtgat ggccatccca gccatactca ttggggccat tggagcatca    900
   acagactgga accagactgc atatgggctt ccagatccca agactacaga agaggcagac    960
   atgattttac caattgttct gcagtatctc tgccctgtgt atatttcttt ctttggtctt   1020
   ggtgcagttt ctgctgctgt tatgtcatca gcagattctt ccatcttgtc agcaagttcc   1080
   atgtttgcac ggaacatcta ccagctttcc ttcagacaaa atgcttcgga caaagaaatc   1140
   gtttgggtta tgcgaatcac agtgtttgtg tttggagcat ctgcaacagc catggccttg   1200
   ctgacgaaaa ctgtgtatgg gctctggtac ctcagttctg accttgttta catcgttatc   1260
   ttcccccagc tgctttgtgt actctttgtt aagggaacca acacctatgg ggccgtggca   1320
   ggttatgttt ctggcctctt cctgagaata actggagggg agccatatct gtatcttcag   1380
   cccttgatct tctaccctgg ctattaccct gatgataatg gtatatataa tcagaaattt   1440
   ccatttaaaa cacttgccat ggttacatca ttcttaacca acatttgcat ctcctatcta   1500
   gccaagtatc tatttgaaag tggaaccttg ccacctaaat tagatgtatt tgatgctgtt   1560
   gttgcaagac acagtgaaga aaacatggat aagacaattc ttgtcaaaaa tgaaaatatt   1620
   aaattagatg aacttgcact tgtgaagcca cgacagagca tgaccctcag ctcaactttc   1680
   accaataaag aggccttcct tgatgttgat tccagtccag aagggtctgg gactgaagat   1740
   aatttacagg ccgaagacgc agacatgcgc aatgagctgg aggagatgca gcgaagggct   1800
   gaccagttgg ctgatgagtc gctggaaagc acccgtcgta tgctgcaact ggttgaagag   1860
   agtaaagatg ctggtatcag gactttggtt atgttggatg aacaaggaga acaactggaa   1920
   cgcattgagg aagggatgga ccaaatcaat aaggacatga aagaagcaga aaagaatttg   1980
   acggacctag gaaaattctg cgggctttgt gtgtgtccct gtaacaagct taaatcaagt   2040
   gatgcttaca aaaaagcctg ggcaataaat caggacggag tggtggccag ccagcctgct   2100
   cgtgtagtgg acgaacggga gcagatggcc atcagtggcg gcttcatccg cagggtaaca   2160
   aatgatgccc gagaaaatga aatggatgaa aacctagagc aggtgagcgg catcatcggg   2220
   aacctccgtc acatggccct ggatatgggc aatgagatcg atacacagaa tcgccagatc 2280
   gacaggatca tggagaaggc tgattccaac aaaaccagaa ttgatgaggc caaccaacgt 2340
   gcaacaaaga tgctgggaag tggttctaga 2370
<210> 8
   <211> 1032
   <212> PRT
   <213> artificial
<220>
   <223> Cholinetransporter-SNAP25-tTA (3)(pCMV-Tet3G)
<400> 8
<210> 9
   <211> 603
   <212> DNA
   <213> artificial
<220>
   <223> Synaptotagmin-SNAP25-tTA(pCMV-Tet3G)
<400> 9
   gaattcacca tgaggaacat tttcaagagg aaccaggagc ctattgtggc tcctgccacc     60
   accaccgcca cgatgcccat tggacccgtg gacaactcca ctgagagtgg gggtgctggg    120
   gagagccagg aggacatgtt tgccaaactg aaggagaagt tattcaatga gataaacaag    180
   attcccttac caccctgggc actgatcgcc attgctgtgg ttgctgggct cctgcttctc    240
   acctgctgct tctgcatcaa gcttaaatca agtgatgctt acaaaaaagc ctggggcaat    300
   aatcaggacg gagtggtggc cagccagcct gctcgtgtag tggacgaacg ggagcagatg    360
   gccatcagtg gcggcttcat ccgcagggta acaaatgatg cccgagaaaa tgaaatggat    420
   gaaaacctag agcaggtgag cggcatcatc gggaacctcc gtcacatggc cctggatatg    480
   ggcaatgaga tcgatacaca gaatcgccag atcgacagga tcatggagaa ggctgattcc    540
   aacaaaacca gaattgatga ggccaaccaa cgtgcaacaa agatgctggg aagtggttct    600
   aga                                                                  603
<210> 10
   <211> 443
   <212> PRT
   <213> artificial
<220>
   <223> Synaptotagmin-SNAP25-tTA(pCMV-Tet3G)
<400> 10
   Met Arg Asn Ile Phe Lys Arg Asn Gln Glu Pro Ile Val Ala Pro Ala
   1               5                   10                  15
   Thr Thr Thr Ala Thr Met Pro Ile Gly Pro Val Asp Asn Ser Thr Glu
               20                  25                  30
   Ser Gly Gly Ala Gly Glu Ser Gln Glu Asp Met Phe Ala Lys Leu Lys
           35                  40                  45
   Glu Lys Leu Phe Asn Glu Ile Asn Lys Ile Pro Leu Pro Pro Trp Ala
       50                  55                  60
   Leu Ile Ala Ile Ala Val Val Ala Gly Leu Leu Leu Leu Thr Cys Cys
   65                  70                  75                  80
   Phe Cys Ile Lys Leu Lys Ser Ser Asp Ala Tyr Lys Lys Ala Trp Gly
                   85                  90                  95
   Asn Asn Gln Asp Gly Val Val Ala Ser Gln Pro Ala Arg Val Val Asp
               100                 105                 110
   Glu Arg Glu Gln Met Ala Ile Ser Gly Gly Phe Ile Arg Arg Val Thr
           115                 120                 125
   Asn Asp Ala Arg Glu Asn Glu Met Asp Glu Asn Leu Glu Gln Val Ser
       130                 135                 140
   Gly Ile Ile Gly Asn Leu Arg His Met Ala Leu Asp Met Gly Asn Glu
   145                 150                 155                 160
   Ile Asp Thr Gln Asn Arg Gln Ile Asp Arg Ile Met Glu Lys Ala Asp
                   165                 170                 175
   Ser Asn Lys Thr Arg Ile Asp Glu Ala Asn Gln Arg Ala Thr Lys Met
               180                 185                 190
   Leu Gly Ser Gly Ser Arg Leu Asp Lys Ser Lys Val Ile Asn Ser Ala
           195                 200                 205
   Leu Glu Leu Leu Asn Gly Val Gly Ile Glu Gly Leu Thr Thr Arg Lys
       210                 215                 220
   Leu Ala Gln Lys Leu Gly Val Glu Gln Pro Thr Leu Tyr Trp His Val
   225                 230                 235                 240
   Lys Asn Lys Arg Ala Leu Leu Asp Ala Leu Pro Ile Glu Met Leu Asp
                   245                 250                 255
   Arg His His Thr His Ser Cys Pro Leu Glu Gly Glu Ser Trp Gln Asp
               260                 265                 270
   Phe Leu Arg Asn Asn Ala Lys Ser Tyr Arg Cys Ala Leu Leu Ser His
           275                 280                 285
   Arg Asp Gly Ala Lys Val His Leu Gly Thr Arg Pro Thr Glu Lys Gln
       290                 295                 300
   Tyr Glu Thr Leu Glu Asn Gln Leu Ala Phe Leu Cys Gln Gln Gly Phe
   305                 310                 315                 320
   Ser Leu Glu Asn Ala Leu Tyr Ala Leu Ser Ala Val Gly His Phe Thr
                   325                 330                 335
   Leu Gly Cys Val Leu Glu Glu Gln Glu His Gln Val Ala Lys Glu Glu
               340                 345                 350
   Arg Glu Thr Pro Thr Thr Asp Ser Met Pro Pro Leu Leu Lys Gln Ala
           355                 360                 365
   Ile Glu Leu Phe Asp Arg Gln Gly Ala Glu Pro Ala Phe Leu Phe Gly
       370                 375                 380
   Leu Glu Leu Ile Ile Cys Gly Leu Glu Lys Gln Leu Lys Cys Glu Ser
   385                 390                 395                 400
   Gly Gly Pro Thr Asp Ala Leu Asp Asp Phe Asp Leu Asp Met Leu Pro
                   405                 410                 415
   Ala Asp Ala Leu Asp Asp Phe Asp Leu Asp Met Leu Pro Ala Asp Ala
               420                 425                 430
   Leu Asp Asp Phe Asp Leu Asp Met Leu Pro Gly
           435                 440
<210> 11
   <211> 3600
   <212> DNA
   <213> artificial
<220>
   <223> mAN02-SNAP25 (pcDNA3.1)
<400> 11
   cttaagatgc ccgccctgcg ccccgctctg ctgtgggcgc tgctggcgct ctggctgtgc    60
   tgcgcggccc ccgcgcatgc attgcagtgt cgagatcctg ccacctgtct gagccagtat   120
   tgtgccgaca aagctcggga tggcgtctgt gatgaggcct gcaacagcca tgcctgccag   180
   tgggatgggg gtgactgttc tctcaccatg gagaacccct gggccaactg ctcctcccca   240
   cttccctgct gggattatat caacaaccag tgtgatgagc tgtgcaacac ggtcgagtgc   300
   ctgtttgaca actttgaatg ccaggggaac agcaagacat gcaagtatga caaatactgt   360
   gcagaccact tcaaagacaa ccactgtgac caggggtgca acagtgagga gtgtggttgg   420
   gatgggctgg actgtgctgc tgaccaacct gagaacctgg cagaaggtac cctggttatt   480
   gtggtattga tgccacctga acaactgctc caggatgctc gcagcttctt gcgggcactg   540
   ggtaccctgc tccacaccaa cctgcgcatt aagcgggact cccaggggga actcatggtg   600
   tacccctatt atggtgagaa gtcagctgct atgaagaaac agaggatgac acgcagatcc   660
   cttcctggtg aacaagaaca ggaggtggct ggctctaaag tctttctgga aattgacaac   720
   cgccagtgtg ttcaagactc agaccactgc ttcaagaaca cggatgcagc agcagctctc   780
   ctggcctctc acgccataca ggggaccctg tcataccctc ttgtgtctgt cgtcagtgaa   840
   tccctgactc cagaacgcac tcagctcctc tatctccttg ctgttgctgt tgtcatcatt   900
   ctgtttatta ttctgctggg ggtaatcaac aagcttaaat caagtgatgc ttacaaaaaa   960
   gcctggggca ataatcagga cggagtggtg gccagccagc ctgctcgtgt agtggacgaa  1020
   cgggagcaga tggccatcag tggcggcttc atccgcaggg taacaaatga tgcccgagaa  1080
   aatgaaatgg atgaaaacct agagcaggtg agcggcatca tcgggaacct ccgtcacatg  1140
   gccctggata tgggcaatga gatcgataca cagaatcgcc agatcgacag gatcatggag  1200
   aaggctgatt ccaacaaaac cagaattgat gaggccaacc aacgtgcaac aaagatgctg  1260
   ggaagtggta tggcaaaacg aaagcgtaag catggctctc tctggctgcc tgaaggtttc  1320
   actcttcgcc gagatgcaag caatcacaag cgtcgtgagc cagtgggaca ggatgctgtg  1380
   gggctgaaaa atctctcagt gcaagtctca gaagctaacc taattggtac tggaacaagt  1440
   gaacactggg tcgatgatga agggccccag ccaaagaaag taaaggctga agatgaggcc  1500
   ttactctcag aagaagatga ccccattgat cgacggccat ggacacagca gcaccttgaa  1560
   gctgcagaca tccgtaggac accatcgctg gctctcaccc ctcctcaggc agagcaggag  1620
   gtggatgtgt tagatgtgaa tgtccgtggc ccagatggct gcaccccatt gatgttggct  1680
   tctctccgag gaggcagctc agatttgagt gatgaagatg aagatgcaga ggactcttct  1740
   gctaacatca tcacagactt ggtctaccag ggtgccagcc tccaggccca gacagaccgg  1800
   actggtgaga tggccctgca ccttgcagcc cgctactcac gggctgatgc tgccaagcgt  1860
   ctcctggatg caggtgcaga tgccaatgcc caggacaaca tgggccgctg tccactccat  1920
   gctgcagtgg cagctgatgc ccaaggtgtc ttccagattc tgattcgcaa ccgagtaact 1980
   gatctagatg ccaggatgaa tgatggtact acacccctga tcctggctgc ccgcctggct 2040
   gtggagggaa tggtggcaga actgatcaac tgccaagcgg atgtgaatgc agtggatgac 2100
   catggaaaat ctgctcttca ctgggcagct gctgtcaata atgtggaggc aactcttttg 2160
   ttgttgaaaa atggggccaa ccgagacatg caggacaaca aggaagagac acctctgttt 2220
   cttgctgccc gggaggggag ctatgaagca gccaagatcc tgttagacca ttttgccaat 2280
   cgagacatca cagaccatat ggatcgtctt ccccgggatg tggctcggga tcgcatgcac 2340
   catgacattg tgcgccttct ggatgaatac aatgtgaccc caagccctcc aggcaccgtg 2400
   ttgacttctg ctctctcacc tgtcatctgt gggcccaaca gatctttcct cagcctgaag 2460
   cacaccccaa tgggcaagaa gtctagacgg cccagtgcca agagtaccat gcctactagc 2520
   ctccctaacc ttgccaagga ggcaaaggat gccaagggta gtaggaggaa gaagtctctg 2580
   agtgagaagg tccaactgtc tgagagttca gtaactttat cccctgttga ttccctagaa 2640
   tctcctcaca cgtatgtttc cgacaccaca tcctctccaa tgattacatc ccctgggatc 2700
   ttacaggcct cacccaaccc tatgttggcc actgccgccc ctcctgcccc agtccatgcc 2760
   cagcatgcac tatctttttc taaccttcat gaaatgcagc ctttggcaca tggggccagc 2820
   actgtgcttc cctcagtgag ccagttgcta tcccaccacc acattgtgtc tccaggcagt 2880
   ggcagtgctg gaagcttgag taggctccat ccagtcccag tcccagcaga ttggatgaac 2940
   cgcatggagg tgaatgagac ccagtacaat gagatgtttg gtatggtcct ggctccagct 3000
   gagggcaccc atcctggcat agctccccag agcaggccac ctgaagggaa gcacataacc 3060
   acccctcggg agcccttgcc ccccattgtg actttccagc tcatccctaa aggcagtatt 3120
   gcccaaccag cgggggctcc ccagcctcag tccacctgcc ctccagctgt tgcgggcccc 3180
   ctgcccacca tgtaccagat tccagaaatg gcccgtttgc ccagtgtggc tttccccact 3240
   gccatgatgc cccagcagga cgggcaggta gctcagacca ttctcccagc ctatcatcct 3300
   ttcccagcct ctgtgggcaa gtaccccaca cccccttcac agcacagtta tgcttcctca 3360
   aatgctgctg agcgaacacc cagtcacagt ggtcacctcc agggtgagca tccctacctg 3420
   acaccatccc cagagtctcc tgaccagtgg tcaagttcat caccccactc tgcttctgac 3480
   tggtcagatg tgaccaccag ccctacccct gggggtgctg gaggaggtca gcggggacct 3540
   gggacacaca tgtctgagcc accacacaac aacatgcagg tttatgcgtg ataatctaga 3600
<210> 12
   <211> 1194
   <212> PRT
   <213> artificial
<220>
   <223> mAN02-SNAP25 (pcDNA3.1)
<400> 12
   Met Pro Ala Leu Arg Pro Ala Leu Leu Trp Ala Leu Leu Ala Leu Trp
   1               5                   10                  15
   Leu Cys Cys Ala Ala Pro Ala His Ala Leu Gln Cys Arg Asp Pro Ala
               20                  25                  30
   Thr Cys Leu Ser Gln Tyr Cys Ala Asp Lys Ala Arg Asp Gly Val Cys
           35                  40                  45
   Asp Glu Ala Cys Asn Ser His Ala Cys Gln Trp Asp Gly Gly Asp Cys
       50                  55                  60
   Ser Leu Thr Met Glu Asn Pro Trp Ala Asn Cys Ser Ser Pro Leu Pro
   65                  70                  75                  80
   Cys Trp Asp Tyr Ile Asn Asn Gln Cys Asp Glu Leu Cys Asn Thr Val
                   85                  90                  95
   Glu Cys Leu Phe Asp Asn Phe Glu Cys Gln Gly Asn Ser Lys Thr Cys
               100                 105                 110
   Lys Tyr Asp Lys Tyr Cys Ala Asp His Phe Lys Asp Asn His Cys Asp
           115                 120                 125
   Gln Gly Cys Asn Ser Glu Glu Cys Gly Trp Asp Gly Leu Asp Cys Ala
       130                 135                 140
   Ala Asp Gln Pro Glu Asn Leu Ala Glu Gly Thr Leu Val Ile Val Val
   145                 150                 155                 160
   Leu Met Pro Pro Glu Gln Leu Leu Gln Asp Ala Arg Ser Phe Leu Arg
                   165                 170                 175
   Ala Leu Gly Thr Leu Leu His Thr Asn Leu Arg Ile Lys Arg Asp Ser
               180                 185                 190
   Gln Gly Glu Leu Met Val Tyr Pro Tyr Tyr Gly Glu Lys Ser Ala Ala
           195                 200                 205
   Met Lys Lys Gln Arg Met Thr Arg Arg Ser Leu Pro Gly Glu Gln Glu
       210                 215                 220
   Gln Glu Val Ala Gly Ser Lys Val Phe Leu Glu Ile Asp Asn Arg Gln
   225                 230                 235                 240
   Cys Val Gln Asp Ser Asp His Cys Phe Lys Asn Thr Asp Ala Ala Ala
   245 250 255
   Ala Leu Leu Ala Ser His Ala Ile Gln Gly Thr Leu Ser Tyr Pro Leu
   260 265 270
   Val Ser Val Val Ser Glu Ser Leu Thr Pro Glu Arg Thr Gln Leu Leu
   275 280 285
   Tyr Leu Leu Ala Val Ala Val Val Ile Ile Leu Phe Ile Ile Leu Leu
   290 295 300
   Gly Val Ile Asn Lys Leu Lys Ser Ser Asp Ala Tyr Lys Lys Ala Trp
   305 310 315 320
   Gly Asn Asn Gln Asp Gly Val Val Ala Ser Gln Pro Ala Arg Val Val
   325 330 335
   Asp Glu Arg Glu Gln Met Ala Ile Ser Gly Gly Phe Ile Arg Arg Val
   340 345 350
   Thr Asn Asp Ala Arg Glu Asn Glu Met Asp Glu Asn Leu Glu Gln Val
   355 360 365
   Ser Gly Ile Ile Gly Asn Leu Arg His Met Ala Leu Asp Met Gly Asn
   370 375 380
   Glu Ile Asp Thr Gln Asn Arg Gln Ile Asp Arg Ile Met Glu Lys Ala
   385 390 395 400
   Asp Ser Asn Lys Thr Arg Ile Asp Glu Ala Asn Gln Arg Ala Thr Lys
   405 410 415
   Met Leu Gly Ser Gly Met Ala Lys Arg Lys Arg Lys His Gly Ser Leu
   420 425 430
   Trp Leu Pro Glu Gly Phe Thr Leu Arg Arg Asp Ala Ser Asn His Lys
   435 440 445
   Arg Arg Glu Pro Val Gly Gln Asp Ala Val Gly Leu Lys Asn Leu Ser
   450 455 460
   Val Gln Val Ser Glu Ala Asn Leu Ile Gly Thr Gly Thr Ser Glu His
   465 470 475 480
   Trp Val Asp Asp Glu Gly Pro Gln Pro Lys Lys Val Lys Ala Glu Asp
   485 490 495
   Glu Ala Leu Leu Ser Glu Glu Asp Asp Pro Ile Asp Arg Arg Pro Trp
   500 505 510
   Thr Gln Gln His Leu Glu Ala Ala Asp Ile Arg Arg Thr Pro Ser Leu
   515 520 525
   Ala Leu Thr Pro Pro Gln Ala Glu Gln Glu Val Asp Val Leu Asp Val
   530 535 540
   Asn Val Arg Gly Pro Asp Gly Cys Thr Pro Leu Met Leu Ala Ser Leu
   545 550 555 560
   Arg Gly Gly Ser Ser Asp Leu Ser Asp Glu Asp Glu Asp Ala Glu Asp
   565 570 575
   Ser Ser Ala Asn Ile Ile Thr Asp Leu Val Tyr Gln Gly Ala Ser Leu
   580 585 590
   Gln Ala Gln Thr Asp Arg Thr Gly Glu Met Ala Leu His Leu Ala Ala
   595 600 605
   Arg Tyr Ser Arg Ala Asp Ala Ala Lys Arg Leu Leu Asp Ala Gly Ala
   610 615 620
   Asp Ala Asn Ala Gln Asp Asn Met Gly Arg Cys Pro Leu His Ala Ala
   625 630 635 640
   Val Ala Ala Asp Ala Gln Gly Val Phe Gln Ile Leu Ile Arg Asn Arg
   645 650 655
   Val Thr Asp Leu Asp Ala Arg Met Asn Asp Gly Thr Thr Pro Leu Ile
   660 665 670
   Leu Ala Ala Arg Leu Ala Val Glu Gly Met Val Ala Glu Leu Ile Asn
   675 680 685
   Cys Gln Ala Asp Val Asn Ala Val Asp Asp His Gly Lys Ser Ala Leu
   690 695 700
   His Trp Ala Ala Ala Val Asn Asn Val Glu Ala Thr Leu Leu Leu Leu
   705 710 715 720
   Lys Asn Gly Ala Asn Arg Asp Met Gln Asp Asn Lys Glu Glu Thr Pro
   725 730 735
   Leu Phe Leu Ala Ala Arg Glu Gly Ser Tyr Glu Ala Ala Lys Ile Leu
               740                 745                 750
   Leu Asp His Phe Ala Asn Arg Asp Ile Thr Asp His Met Asp Arg Leu
           755                 760                 765
   Pro Arg Asp Val Ala Arg Asp Arg Met His His Asp Ile Val Arg Leu
       770                 775                 780
   Leu Asp Glu Tyr Asn Val Thr Pro Ser Pro Pro Gly Thr Val Leu Thr
   785                 790                 795                 800
   Ser Ala Leu Ser Pro Val Ile Cys Gly Pro Asn Arg Ser Phe Leu Ser
                   805                 810                 815
   Leu Lys His Thr Pro Met Gly Lys Lys Ser Arg Arg Pro Ser Ala Lys
               820                 825                 830
   Ser Thr Met Pro Thr Ser Leu Pro Asn Leu Ala Lys Glu Ala Lys Asp
           835                 840                 845
   Ala Lys Gly Ser Arg Arg Lys Lys Ser Leu Ser Glu Lys Val Gln Leu
       850                 855                 860
   Ser Glu Ser Ser Val Thr Leu Ser Pro Val Asp Ser Leu Glu Ser Pro
   865                 870                 875                 880
   His Thr Tyr Val Ser Asp Thr Thr Ser Ser Pro Met Ile Thr Ser Pro
                   885                 890                 895
   Gly Ile Leu Gln Ala Ser Pro Asn Pro Met Leu Ala Thr Ala Ala Pro
               900                 905                 910
   Pro Ala Pro Val His Ala Gln His Ala Leu Ser Phe Ser Asn Leu His
           915                 920                 925
   Glu Met Gln Pro Leu Ala His Gly Ala Ser Thr Val Leu Pro Ser Val
       930                 935                 940
   Ser Gln Leu Leu Ser His His His Ile Val Ser Pro Gly Ser Gly Ser
   945                 950                 955                 960
   Ala Gly Ser Leu Ser Arg Leu His Pro Val Pro Val Pro Ala Asp Trp
                   965                 970                 975
   Met Asn Arg Met Glu Val Asn Glu Thr Gln Tyr Asn Glu Met Phe Gly
               980                 985                 990
   Met Val Leu Ala Pro Ala Glu Gly  Thr His Pro Gly Ile  Ala Pro Gln
           995                 1000                1005
   Ser Arg  Pro Pro Glu Gly Lys  His Ile Thr Thr Pro  Arg Glu Pro
       1010                 1015                 1020
   Leu Pro  Pro Ile Val Thr Phe  Gln Leu Ile Pro Lys  Gly Ser Ile
       1025                 1030                 1035
   Ala Gln  Pro Ala Gly Ala Pro  Gln Pro Gln Ser Thr  Cys Pro Pro
       1040                 1045                 1050
   Ala Val  Ala Gly Pro Leu Pro  Thr Met Tyr Gln Ile  Pro Glu Met
       1055                 1060                 1065
   Ala Arg  Leu Pro Ser Val Ala  Phe Pro Thr Ala Met  Met Pro Gln
       1070                 1075                 1080
   Gln Asp  Gly Gln Val Ala Gln  Thr Ile Leu Pro Ala  Tyr His Pro
       1085                 1090                 1095
   Phe Pro  Ala Ser Val Gly Lys  Tyr Pro Thr Pro Pro  Ser Gln His
       1100                 1105                 1110
   Ser Tyr  Ala Ser Ser Asn Ala  Ala Glu Arg Thr Pro  Ser His Ser
       1115                 1120                 1125
   Gly His  Leu Gln Gly Glu His  Pro Tyr Leu Thr Pro  Ser Pro Glu
       1130                 1135                 1140
   Ser Pro  Asp Gln Trp Ser Ser  Ser Ser Pro His Ser  Ala Ser Asp
       1145                 1150                 1155
   Trp Ser  Asp Val Thr Thr Ser  Pro Thr Pro Gly Gly  Ala Gly Gly
       1160                 1165                 1170
   Gly Gln  Arg Gly Pro Gly Thr  His Met Ser Glu Pro  Pro His Asn
       1175                 1180                 1185
   Asn Met  Gln Val Tyr Ala
       1190
<210> 13
   <211> 633
   <212> DNA
   <213> artificial
<220>
   <223> SNAP25-tTA(pCMV-Tet3G)
<400> 13
   gaattcacca tggccgaaga cgcagacatg cgcaatgagc tggaggagat gcagcgaagg     60
   gctgaccagt tggctgatga gtcgctggaa agcacccgtc gtatgctgca actggttgaa    120
   gagagtaaag atgctggtat caggactttg gttatgttgg atgaacaagg agaacaactg    180
   gaacgcattg aggaagggat ggaccaaatc aataaggaca tgaaagaagc agaaaagaat    240
   ttgacggacc taggaaaatt ctgcgggctt tgtgtgtgtc cctgtaacaa gcttaaatca    300
   agtgatgctt acaaaaaagc ctggggcaat aatcaggacg gagtggtggc cagccagcct    360
   gctcgtgtag tggacgaacg ggagcagatg gccatcagtg gcggcttcat ccgcagggta    420
   acaaatgatg cccgagaaaa tgaaatggat gaaaacctag agcaggtgag cggcatcatc    480
   gggaacctcc gtcacatggc cctggatatg ggcaatgaga tcgatacaca gaatcgccag    540
   atcgacagga tcatggagaa ggctgattcc aacaaaacca gaattgatga ggccaaccaa    600
   cgtgcaacaa agatgctggg aagtggttct aga                                 633
<210> 14
   <211> 453
   <212> PRT
   <213> artificial
<220>
   <223> SNAP25-tTA(pCMV-Tet3G)
<400> 14
   Met Ala Glu Asp Ala Asp Met Arg Asn Glu Leu Glu Glu Met Gln Arg
   1               5                   10                  15
   Arg Ala Asp Gln Leu Ala Asp Glu Ser Leu Glu Ser Thr Arg Arg Met
               20                  25                  30
   Leu Gln Leu Val Glu Glu Ser Lys Asp Ala Gly Ile Arg Thr Leu Val
           35                  40                  45
   Met Leu Asp Glu Gln Gly Glu Gln Leu Glu Arg Ile Glu Glu Gly Met
       50                  55                  60
   Asp Gln Ile Asn Lys Asp Met Lys Glu Ala Glu Lys Asn Leu Thr Asp
   65                  70                  75                  80
   Leu Gly Lys Phe Cys Gly Leu Cys Val Cys Pro Cys Asn Lys Leu Lys
                   85                  90                  95
   Ser Ser Asp Ala Tyr Lys Lys Ala Trp Gly Asn Asn Gln Asp Gly Val
               100                 105                 110
   Val Ala Ser Gln Pro Ala Arg Val Val Asp Glu Arg Glu Gln Met Ala
           115                 120                 125
   Ile Ser Gly Gly Phe Ile Arg Arg Val Thr Asn Asp Ala Arg Glu Asn
       130                 135                 140
   Glu Met Asp Glu Asn Leu Glu Gln Val Ser Gly Ile Ile Gly Asn Leu
   145                 150                 155                 160
   Arg His Met Ala Leu Asp Met Gly Asn Glu Ile Asp Thr Gln Asn Arg
                   165                 170                 175
   Gln Ile Asp Arg Ile Met Glu Lys Ala Asp Ser Asn Lys Thr Arg Ile
               180                 185                 190
   Asp Glu Ala Asn Gln Arg Ala Thr Lys Met Leu Gly Ser Gly Ser Arg
           195                 200                 205
   Leu Asp Lys Ser Lys Val Ile Asn Ser Ala Leu Glu Leu Leu Asn Gly
       210                 215                 220
   Val Gly Ile Glu Gly Leu Thr Thr Arg Lys Leu Ala Gln Lys Leu Gly
   225                 230                 235                 240
   Val Glu Gln Pro Thr Leu Tyr Trp His Val Lys Asn Lys Arg Ala Leu
                   245                 250                 255
   Leu Asp Ala Leu Pro Ile Glu Met Leu Asp Arg His His Thr His Ser
               260                 265                 270
   Cys Pro Leu Glu Gly Glu Ser Trp Gln Asp Phe Leu Arg Asn Asn Ala
           275                 280                 285
   Lys Ser Tyr Arg Cys Ala Leu Leu Ser His Arg Asp Gly Ala Lys Val
       290                 295                 300
   His Leu Gly Thr Arg Pro Thr Glu Lys Gln Tyr Glu Thr Leu Glu Asn
   305                 310                 315                 320
   Gln Leu Ala Phe Leu Cys Gln Gln Gly Phe Ser Leu Glu Asn Ala Leu
                   325                 330                 335
   Tyr Ala Leu Ser Ala Val Gly His Phe Thr Leu Gly Cys Val Leu Glu
               340                 345                 350
   Glu Gln Glu His Gln Val Ala Lys Glu Glu Arg Glu Thr Pro Thr Thr
           355                 360                 365
   Asp Ser Met Pro Pro Leu Leu Lys Gln Ala Ile Glu Leu Phe Asp Arg
   370 375 380
   Gln Gly Ala Glu Pro Ala Phe Leu Phe Gly Leu Glu Leu Ile Ile Cys
   385 390 395 400
   Gly Leu Glu Lys Gln Leu Lys Cys Glu Ser Gly Gly Pro Thr Asp Ala
   405 410 415
   Leu Asp Asp Phe Asp Leu Asp Met Leu Pro Ala Asp Ala Leu Asp Asp
   420 425 430
   Phe Asp Leu Asp Met Leu Pro Ala Asp Ala Leu Asp Asp Phe Asp Leu
   435 440 445
   Asp Met Leu Pro Gly
   450
<210> 15
   <211> 1290
   <212> DNA
   <213> artificial
<220>
   <223> GST-SNAP25-tTA (2)(pCMV-Tet3G)
<400> 15
   gaattcacca tgtcccctat actaggttat tggaaaatta agggccttgt gcaacccact 60
   cgacttcttt tggaatatct tgaagaaaaa tatgaagagc atttgtatga gcgcgatgaa 120
   ggtgataaat ggcgaaacaa aaagtttgaa ttgggtttgg agtttcccaa tcttccttat 180
   tatattgatg gtgatgttaa attaacacag tctatggcca tcatacgtta tatagctgac 240
   aagcacaaca tgttgggtgg ttgtccaaaa gagcgtgcag agatttcaat gcttgaagga 300
   gcggttttgg atattagata cggtgtttcg agaattgcat atagtaaaga ctttgaaact 360
   ctcaaagttg attttcttag caagctacct gaaatgctga aaatgttcga agatcgttta 420
   tgtcataaaa catatttaaa tggtgatcat gtaacccatc ctgacttcat gttgtatgac 480
   gctcttgatg ttgttttata catggaccca atgtgcctgg atgcgttccc aaaattagtt 540
   tgttttaaaa aacgtattga agctatccca caaattgata agtacttgaa atccagcaag 600
   tatatagcat ggcctttgca gggctggcaa gccacgtttg gtggtggcga ccatcctcca 660
   aaatcggatg ccgaagacgc agacatgcgc aatgagctgg aggagatgca gcgaagggct 720
   gaccagttgg ctgatgagtc gctggaaagc acccgtcgta tgctgcaact ggttgaagag 780
   agtaaagatg ctggtatcag gactttggtt atgttggatg aacaaggaga acaactggaa 840
   cgcattgagg aagggatgga ccaaatcaat aaggacatga aagaagcaga aaagaatttg     900
   acggacctag gaaaattctg cgggctttgt gtgtgtccct gtaacaagct taaatcaagt     960
   gatgcttaca aaaaagcctg gggcaataat caggacggag tggtggccag ccagcctgct    1020
   cgtgtagtgg acgaacggga gcagatggcc atcagtggcg gcttcatccg cagggtaaca    1080
   aatgatgccc gagaaaatga aatggatgaa aacctagagc aggtgagcgg catcatcggg    1140
   aacctccgtc acatggccct ggatatgggc aatgagatcg atacacagaa tcgccagatc    1200
   gacaggatca tggagaaggc tgattccaac aaaaccagaa ttgatgaggc caaccaacgt    1260
   gcaacaaaga tgctgggaag tggttctaga                                    1290
<210> 16
   <211> 672
   <212> PRT
   <213> artificial
<220>
   <223> GST-SNAP25-tTA (2)(pCMV-Tet3G)
<400> 16
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Ala Glu Asp Ala
       210                 215                 220
   Asp Met Arg Asn Glu Leu Glu Glu Met Gln Arg Arg Ala Asp Gln Leu
   225                 230                 235                 240
   Ala Asp Glu Ser Leu Glu Ser Thr Arg Arg Met Leu Gln Leu Val Glu
                   245                 250                 255
   Glu Ser Lys Asp Ala Gly Ile Arg Thr Leu Val Met Leu Asp Glu Gln
               260                 265                 270
   Gly Glu Gln Leu Glu Arg Ile Glu Glu Gly Met Asp Gln Ile Asn Lys
           275                 280                 285
   Asp Met Lys Glu Ala Glu Lys Asn Leu Thr Asp Leu Gly Lys Phe Cys
       290                 295                 300
   Gly Leu Cys Val Cys Pro Cys Asn Lys Leu Lys Ser Ser Asp Ala Tyr
   305                 310                 315                 320
   Lys Lys Ala Trp Gly Asn Asn Gln Asp Gly Val Val Ala Ser Gln Pro
                   325                 330                 335
   Ala Arg Val Val Asp Glu Arg Glu Gln Met Ala Ile Ser Gly Gly Phe
               340                 345                 350
   Ile Arg Arg Val Thr Asn Asp Ala Arg Glu Asn Glu Met Asp Glu Asn
           355                 360                 365
   Leu Glu Gln Val Ser Gly Ile Ile Gly Asn Leu Arg His Met Ala Leu
       370                 375                 380
   Asp Met Gly Asn Glu Ile Asp Thr Gln Asn Arg Gln Ile Asp Arg Ile
   385                 390                 395                 400
   Met Glu Lys Ala Asp Ser Asn Lys Thr Arg Ile Asp Glu Ala Asn Gln
                   405                 410                 415
   Arg Ala Thr Lys Met Leu Gly Ser Gly Ser Arg Leu Asp Lys Ser Lys
               420                 425                 430
   Val Ile Asn Ser Ala Leu Glu Leu Leu Asn Gly Val Gly Ile Glu Gly
           435                 440                 445
   Leu Thr Thr Arg Lys Leu Ala Gln Lys Leu Gly Val Glu Gln Pro Thr
       450                 455                 460
   Leu Tyr Trp His Val Lys Asn Lys Arg Ala Leu Leu Asp Ala Leu Pro
   465                 470                 475                 480
   Ile Glu Met Leu Asp Arg His His Thr His Ser Cys Pro Leu Glu Gly
                   485                 490                 495
   Glu Ser Trp Gln Asp Phe Leu Arg Asn Asn Ala Lys Ser Tyr Arg Cys
               500                 505                 510
   Ala Leu Leu Ser His Arg Asp Gly Ala Lys Val His Leu Gly Thr Arg
           515                 520                 525
   Pro Thr Glu Lys Gln Tyr Glu Thr Leu Glu Asn Gln Leu Ala Phe Leu
       530                 535                 540
   Cys Gln Gln Gly Phe Ser Leu Glu Asn Ala Leu Tyr Ala Leu Ser Ala
   545                 550                 555                 560
   Val Gly His Phe Thr Leu Gly Cys Val Leu Glu Glu Gln Glu His Gln
                   565                 570                 575
   Val Ala Lys Glu Glu Arg Glu Thr Pro Thr Thr Asp Ser Met Pro Pro
               580                 585                 590
   Leu Leu Lys Gln Ala Ile Glu Leu Phe Asp Arg Gln Gly Ala Glu Pro
           595                 600                 605
   Ala Phe Leu Phe Gly Leu Glu Leu Ile Ile Cys Gly Leu Glu Lys Gln
       610                 615                 620
   Leu Lys Cys Glu Ser Gly Gly Pro Thr Asp Ala Leu Asp Asp Phe Asp
   625                 630                 635                 640
   Leu Asp Met Leu Pro Ala Asp Ala Leu Asp Asp Phe Asp Leu Asp Met
                   645                 650                 655
   Leu Pro Ala Asp Ala Leu Asp Asp Phe Asp Leu Asp Met Leu Pro Gly
               660                 665                 670
<210> 17
   <211> 2094
   <212> DNA
   <213> artificial
<220>
   <223> GFP-SNAP25-tTA(pcDNA3.1)
<400> 17
   cttaaggcca ccatggccag caaaggagaa gaacttttca ctggagttgt cccaattctt     60
   gttgaattag atggtgatgt taatgggcac aaattttctg tcagtggaga gggtgaaggt    120
   gatgctacat acggaaagct tacccttaaa tttatttgca ctactggaaa actacctgtt    180
   ccatggccaa cacttgtcac tactttctct tatggtgttc aatgcttttc ccgttatccg    240
   gatcatatga aacggcatga ctttttcaag agtgccatgc ccgaaggtta tgtacaggaa    300
   cgcactatat ctttcaaaga tgacgggaac tacaagacgc gtgctgaagt caagtttgaa    360
   ggtgataccc ttgttaatcg tatcgagtta aaaggtattg attttaaaga agatggaaac    420
   attctcggac acaaactgga gtacaactat aactcacaca atgtatacat cacggcagac    480
   aaacaaaaga atggaatcaa agctaacttc aaaattcgcc acaacattga agatggatcc    540
   gttcaactag cagaccatta tcaacaaaat actccaattg gcgatggccc tgtcctttta    600
   ccagacaacc attacctgtc gacacaatct gccctttcga aagatcccaa cgaaaagcgt    660
   gaccacatgg tccttcttga gtttgtaact gctgctggga ttacacatgg catggatgag    720
   ctctacaaag ccgaagacgc agacatgcgc aatgagctgg aggagatgca gcgaagggct    780
   gaccagttgg ctgatgagtc gctggaaagc acccgtcgta tgctgcaact ggttgaagag    840
   agtaaagatg ctggtatcag gactttggtt atgttggatg aacaaggaga acaactggaa    900
   cgcattgagg aagggatgga ccaaatcaat aaggacatga aagaagcaga aaagaatttg    960
   acggacctag gaaaattctg cgggctttgt gtgtgtccct gtaacaagct taaatcaagt   1020
   gatgcttaca aaaaagcctg gggcaataat caggacggag tggtggccag ccagcctgct   1080
   cgtgtagtgg acgaacggga gcagatggcc atcagtggcg gcttcatccg cagggtaaca   1140
   aatgatgccc gagaaaatga aatggatgaa aacctagagc aggtgagcgg catcatcggg   1200
   aacctccgtc acatggccct ggatatgggc aatgagatcg atacacagaa tcgccagatc   1260
   gacaggatca tggagaaggc tgattccaac aaaaccagaa ttgatgaggc caaccaacgt   1320
   gcaacaaaga tgctgggaag tggttctaga ctggacaaga gcaaagtcat aaactctgct   1380
   ctggaattac tcaatggagt cggtatcgaa ggcctgacga caaggaaact cgctcaaaag   1440
   ctgggagttg agcagcctac cctgtactgg cacgtgaaga acaagcgggc cctgctcgat   1500
   gccctgccaa tcgagatgct ggacaggcat catacccact cctgccccct ggaaggcgag   1560
   tcatggcaag actttctgcg gaacaacgcc aagtcatacc gctgtgctct cctctcacat   1620
   cgcgacgggg ctaaagtgca tctcggcacc cgcccaacag agaaacagta cgaaaccctg   1680
   gaaaatcagc tcgcgttcct gtgtcagcaa ggcttctccc tggagaacgc actgtacgct   1740
   ctgtccgccg tgggccactt tacactgggc tgcgtattgg aggaacagga gcatcaagta   1800
   gcaaaagagg aaagagagac acctaccacc gattctatgc ccccacttct gaaacaagca   1860
   attgagctgt tcgaccggca gggagccgaa cctgccttcc ttttcggcct ggaactaatc   1920
   atatgtggcc tggagaaaca gctaaagtgc gaaagcggcg ggccgaccga cgcccttgac   1980
   gattttgact tagacatgct cccagccgat gcccttgacg actttgacct tgatatgctg   2040
   cctgctgacg ctcttgacga ttttgacctt gacatgctcc ccgggtaact cgag         2094
<210> 18
   <211> 691
   <212> PRT
   <213> artificial
<220>
   <223> GFP-SNAP25-tTA(pcDNA3.1)
<400> 18
   Met Ala Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Ser Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Ser Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Thr Ala Asp Lys Gln Lys Asn
   145                 150                 155                 160
   Gly Ile Lys Ala Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Lys Ala
   225                 230                 235                 240
   Glu Asp Ala Asp Met Arg Asn Glu Leu Glu Glu Met Gln Arg Arg Ala
                   245                 250                 255
   Asp Gln Leu Ala Asp Glu Ser Leu Glu Ser Thr Arg Arg Met Leu Gln
               260                 265                 270
   Leu Val Glu Glu Ser Lys Asp Ala Gly Ile Arg Thr Leu Val Met Leu
           275                 280                 285
   Asp Glu Gln Gly Glu Gln Leu Glu Arg Ile Glu Glu Gly Met Asp Gln
       290                 295                 300
   Ile Asn Lys Asp Met Lys Glu Ala Glu Lys Asn Leu Thr Asp Leu Gly
   305                 310                 315                 320
   Lys Phe Cys Gly Leu Cys Val Cys Pro Cys Asn Lys Leu Lys Ser Ser
                   325                 330                 335
   Asp Ala Tyr Lys Lys Ala Trp Gly Asn Asn Gln Asp Gly Val Val Ala
               340                 345                 350
   Ser Gln Pro Ala Arg Val Val Asp Glu Arg Glu Gln Met Ala Ile Ser
           355                 360                 365
   Gly Gly Phe Ile Arg Arg Val Thr Asn Asp Ala Arg Glu Asn Glu Met
   370 375 380
   Asp Glu Asn Leu Glu Gln Val Ser Gly Ile Ile Gly Asn Leu Arg His
   385 390 395 400
   Met Ala Leu Asp Met Gly Asn Glu Ile Asp Thr Gln Asn Arg Gln Ile
   405 410 415
   Asp Arg Ile Met Glu Lys Ala Asp Ser Asn Lys Thr Arg Ile Asp Glu
   420 425 430
   Ala Asn Gln Arg Ala Thr Lys Met Leu Gly Ser Gly Ser Arg Leu Asp
   435 440 445
   Lys Ser Lys Val Ile Asn Ser Ala Leu Glu Leu Leu Asn Gly Val Gly
   450 455 460
   Ile Glu Gly Leu Thr Thr Arg Lys Leu Ala Gln Lys Leu Gly Val Glu
   465 470 475 480
   Gln Pro Thr Leu Tyr Trp His Val Lys Asn Lys Arg Ala Leu Leu Asp
   485 490 495
   Ala Leu Pro Ile Glu Met Leu Asp Arg His His Thr His Ser Cys Pro
   500 505 510
   Leu Glu Gly Glu Ser Trp Gln Asp Phe Leu Arg Asn Asn Ala Lys Ser
   515 520 525
   Tyr Arg Cys Ala Leu Leu Ser His Arg Asp Gly Ala Lys Val His Leu
   530 535 540
   Gly Thr Arg Pro Thr Glu Lys Gln Tyr Glu Thr Leu Glu Asn Gln Leu
   545 550 555 560
   Ala Phe Leu Cys Gln Gln Gly Phe Ser Leu Glu Asn Ala Leu Tyr Ala
   565 570 575
   Leu Ser Ala Val Gly His Phe Thr Leu Gly Cys Val Leu Glu Glu Gln
   580 585 590
   Glu His Gln Val Ala Lys Glu Glu Arg Glu Thr Pro Thr Thr Asp Ser
   595 600 605
   Met Pro Pro Leu Leu Lys Gln Ala Ile Glu Leu Phe Asp Arg Gln Gly
   610 615 620
   Ala Glu Pro Ala Phe Leu Phe Gly Leu Glu Leu Ile Ile Cys Gly Leu
   625                 630                 635                 640
   Glu Lys Gln Leu Lys Cys Glu Ser Gly Gly Pro Thr Asp Ala Leu Asp
                   645                 650                 655
   Asp Phe Asp Leu Asp Met Leu Pro Ala Asp Ala Leu Asp Asp Phe Asp
               660                 665                 670
   Leu Asp Met Leu Pro Ala Asp Ala Leu Asp Asp Phe Asp Leu Asp Met
           675                 680                 685
   Leu Pro Gly
       690
<210> 19
   <211> 1731
   <212> DNA
   <213> artificial
<220>
   <223> MBP-SNAP25-tTA (2)(pCMV-Tet3G)
<400> 19
   gaattcacca tgaagaccga ggagggcaag ctggtgatct ggatcaacgg cgacaagggc      60
   tacaacggcc tggccgaggt gggcaagaag ttcgagaagg acaccggcat caaggtgacc     120
   gtggagcacc ccgacaagct ggaggagaag ttcccccagg tggccgccac cggcgacggc     180
   cccgacatca tcttctgggc ccacgacaga ttcggcggct acgcccagag cggcctgctg     240
   gccgagatca cccccgacaa ggccttccag gacaagctgt accccttcac ctgggacgcc     300
   gtgagataca acggcaagct gatcgcctac cccatcgccg tggaggccct gagcctgatc     360
   tacaacaagg acctgctgcc caaccccccc aagacctggg aggagatccc cgccctggac     420
   aaggagctga aggccaaggg caagagcgcc ctgatgttca acctgcagga gccctacttc     480
   acctggcccc tgatcgccgc cgacggcggc tacgccttca agtacgagaa cggcaagtac     540
   gacatcaagg acgtgggcgt ggacaacgcc ggcgccaagg ccggcctgac cttcctggtg     600
   gacctgatca agaacaagca catgaacgcc gacaccgact acagcatcgc cgaggccgcc     660
   ttcaacaagg gcgagaccgc catgaccatc aacggcccct gggcctggag caacatcgac     720
   accagcaagg tgaactacgg cgtgaccgtg ctgcccacct tcaagggcca gcccagcaag     780
   cccttcgtgg gcgtgctgag cgccggcatc aacgccgcca gccccaacaa ggagctggcc     840
   aaggagttcc tggagaacta cctgctgacc gacgagggcc tggaggccgt gaacaaggac     900
   aagcccctgg gcgccgtggc cctgaagagc tacgaggagg agctggccaa ggaccccaga     960
   atcgccgcca ccatggagaa cgcccagaag ggcgagatca tgcccaacat cccccagatg    1020
   agcgccttct ggtacgccgt gagaaccgcc gtgatcaacg ccgccagcgg cagacagacc    1080
   gtggacgagg ccctgaagga cgcccagacc gccgaagacg cagacatgcg caatgagctg    1140
   gaggagatgc agcgaagggc tgaccagttg gctgatgagt cgctggaaag cacccgtcgt    1200
   atgctgcaac tggttgaaga gagtaaagat gctggtatca ggactttggt tatgttggat    1260
   gaacaaggag aacaactgga acgcattgag gaagggatgg accaaatcaa taaggacatg    1320
   aaagaagcag aaaagaattt gacggaccta ggaaaattct gcgggctttg tgtgtgtccc    1380
   tgtaacaagc ttaaatcaag tgatgcttac aaaaaagcct ggggcaataa tcaggacgga    1440
   gtggtggcca gccagcctgc tcgtgtagtg gacgaacggg agcagatggc catcagtggc    1500
   ggcttcatcc gcagggtaac aaatgatgcc cgagaaaatg aaatggatga aaacctagag    1560
   caggtgagcg gcatcatcgg gaacctccgt cacatggccc tggatatggg caatgagatc    1620
   gatacacaga atcgccagat cgacaggatc atggagaagg ctgattccaa caaaaccaga    1680
   attgatgagg ccaaccaacg tgcaacaaag atgctgggaa gtggttctag a             1731
<210> 20
   <211> 819
   <212> PRT
   <213> artificial
<220>
   <223> MBP-SNAP25-tTA (2) (pCMV-Tet3G)
<400> 20
   Met Lys Thr Glu Glu Gly Lys Leu Val Ile Trp Ile Asn Gly Asp Lys
   1               5                   10                  15
   Gly Tyr Asn Gly Leu Ala Glu Val Gly Lys Lys Phe Glu Lys Asp Thr
               20                  25                  30
   Gly Ile Lys Val Thr Val Glu His Pro Asp Lys Leu Glu Glu Lys Phe
           35                  40                  45
   Pro Gln Val Ala Ala Thr Gly Asp Gly Pro Asp Ile Ile Phe Trp Ala
       50                  55                  60
   His Asp Arg Phe Gly Gly Tyr Ala Gln Ser Gly Leu Leu Ala Glu Ile
   65                  70                  75                  80
   Thr Pro Asp Lys Ala Phe Gln Asp Lys Leu Tyr Pro Phe Thr Trp Asp
                   85                  90                  95
   Ala Val Arg Tyr Asn Gly Lys Leu Ile Ala Tyr Pro Ile Ala Val Glu
               100                 105                 110
   Ala Leu Ser Leu Ile Tyr Asn Lys Asp Leu Leu Pro Asn Pro Pro Lys
           115                 120                 125
   Thr Trp Glu Glu Ile Pro Ala Leu Asp Lys Glu Leu Lys Ala Lys Gly
       130                 135                 140
   Lys Ser Ala Leu Met Phe Asn Leu Gln Glu Pro Tyr Phe Thr Trp Pro
   145                 150                 155                 160
   Leu Ile Ala Ala Asp Gly Gly Tyr Ala Phe Lys Tyr Glu Asn Gly Lys
                   165                 170                 175
   Tyr Asp Ile Lys Asp Val Gly Val Asp Asn Ala Gly Ala Lys Ala Gly
               180                 185                 190
   Leu Thr Phe Leu Val Asp Leu Ile Lys Asn Lys His Met Asn Ala Asp
           195                 200                 205
   Thr Asp Tyr Ser Ile Ala Glu Ala Ala Phe Asn Lys Gly Glu Thr Ala
       210                 215                 220
   Met Thr Ile Asn Gly Pro Trp Ala Trp Ser Asn Ile Asp Thr Ser Lys
   225                 230                 235                 240
   Val Asn Tyr Gly Val Thr Val Leu Pro Thr Phe Lys Gly Gln Pro Ser
                   245                 250                 255
   Lys Pro Phe Val Gly Val Leu Ser Ala Gly Ile Asn Ala Ala Ser Pro
               260                 265                 270
   Asn Lys Glu Leu Ala Lys Glu Phe Leu Glu Asn Tyr Leu Leu Thr Asp
           275                 280                 285
   Glu Gly Leu Glu Ala Val Asn Lys Asp Lys Pro Leu Gly Ala Val Ala
       290                 295                 300
   Leu Lys Ser Tyr Glu Glu Glu Leu Ala Lys Asp Pro Arg Ile Ala Ala
   305                 310                 315                 320
   Thr Met Glu Asn Ala Gln Lys Gly Glu Ile Met Pro Asn Ile Pro Gln
                   325                 330                 335
   Met Ser Ala Phe Trp Tyr Ala Val Arg Thr Ala Val Ile Asn Ala Ala
               340                 345                 350
   Ser Gly Arg Gln Thr Val Asp Glu Ala Leu Lys Asp Ala Gln Thr Ala
           355                 360                 365
   Glu Asp Ala Asp Met Arg Asn Glu Leu Glu Glu Met Gln Arg Arg Ala
       370                 375                 380
   Asp Gln Leu Ala Asp Glu Ser Leu Glu Ser Thr Arg Arg Met Leu Gln
   385                 390                 395                 400
   Leu Val Glu Glu Ser Lys Asp Ala Gly Ile Arg Thr Leu Val Met Leu
                   405                 410                 415
   Asp Glu Gln Gly Glu Gln Leu Glu Arg Ile Glu Glu Gly Met Asp Gln
               420                 425                 430
   Ile Asn Lys Asp Met Lys Glu Ala Glu Lys Asn Leu Thr Asp Leu Gly
           435                 440                 445
   Lys Phe Cys Gly Leu Cys Val Cys Pro Cys Asn Lys Leu Lys Ser Ser
       450                 455                 460
   Asp Ala Tyr Lys Lys Ala Trp Gly Asn Asn Gln Asp Gly Val Val Ala
   465                 470                 475                 480
   Ser Gln Pro Ala Arg Val Val Asp Glu Arg Glu Gln Met Ala Ile Ser
                   485                 490                 495
   Gly Gly Phe Ile Arg Arg Val Thr Asn Asp Ala Arg Glu Asn Glu Met
               500                 505                 510
   Asp Glu Asn Leu Glu Gln Val Ser Gly Ile Ile Gly Asn Leu Arg His
           515                 520                 525
   Met Ala Leu Asp Met Gly Asn Glu Ile Asp Thr Gln Asn Arg Gln Ile
       530                 535                 540
   Asp Arg Ile Met Glu Lys Ala Asp Ser Asn Lys Thr Arg Ile Asp Glu
   545                 550                 555                 560
   Ala Asn Gln Arg Ala Thr Lys Met Leu Gly Ser Gly Ser Arg Leu Asp
                   565                 570                 575
   Lys Ser Lys Val Ile Asn Ser Ala Leu Glu Leu Leu Asn Gly Val Gly
               580                 585                 590
   Ile Glu Gly Leu Thr Thr Arg Lys Leu Ala Gln Lys Leu Gly Val Glu
           595                 600                 605
   Gln Pro Thr Leu Tyr Trp His Val Lys Asn Lys Arg Ala Leu Leu Asp
       610                 615                 620
   Ala Leu Pro Ile Glu Met Leu Asp Arg His His Thr His Ser Cys Pro
   625                 630                 635                 640
   Leu Glu Gly Glu Ser Trp Gln Asp Phe Leu Arg Asn Asn Ala Lys Ser
                   645                 650                 655
   Tyr Arg Cys Ala Leu Leu Ser His Arg Asp Gly Ala Lys Val His Leu
               660                 665                 670
   Gly Thr Arg Pro Thr Glu Lys Gln Tyr Glu Thr Leu Glu Asn Gln Leu
           675                 680                 685
   Ala Phe Leu Cys Gln Gln Gly Phe Ser Leu Glu Asn Ala Leu Tyr Ala
       690                 695                 700
   Leu Ser Ala Val Gly His Phe Thr Leu Gly Cys Val Leu Glu Glu Gln
   705                 710                 715                 720
   Glu His Gln Val Ala Lys Glu Glu Arg Glu Thr Pro Thr Thr Asp Ser
                   725                 730                 735
   Met Pro Pro Leu Leu Lys Gln Ala Ile Glu Leu Phe Asp Arg Gln Gly
               740                 745                 750
   Ala Glu Pro Ala Phe Leu Phe Gly Leu Glu Leu Ile Ile Cys Gly Leu
           755                 760                 765
   Glu Lys Gln Leu Lys Cys Glu Ser Gly Gly Pro Thr Asp Ala Leu Asp
       770                 775                 780
   Asp Phe Asp Leu Asp Met Leu Pro Ala Asp Ala Leu Asp Asp Phe Asp
   785                 790                 795                 800
   Leu Asp Met Leu Pro Ala Asp Ala Leu Asp Asp Phe Asp Leu Asp Met
                   805                 810                 815
   Leu Pro Gly
<210> 21
   <211> 1089
   <212> DNA
   <213> artificial
<220>
   <223> CLAT-SNAP25-tTA (1)(pCMV-Tet3G)
<400> 21
   gaattcacca tggctgagct ggatccgttc ggcgcccctg ccggcgcccc tggcggtccc      60
   gcgctgggga acggagtggc cggcgccggc gaagaagacc cggctgcggc cttcttggcg     120
   cagcaagaga gcgagattgc gggcatcgag aacgacgagg ccttcgccat cctggacggc     180
   ggcgcccccg ggccccagcc gcacggcgag ccgccggggg gtccggatgc tgttgatgga     240
   gtaatgaatg gtgaatacta ccaggaaagt aatggtccaa cagacagtta tgcagctatt     300
   tcacaagtgg atcgattgca gtcagagcct gaaagtatcc gtaaatggag agaagaacaa     360
   atggaacgct tggaagccct tgatgccaat tctcggaagc aagaagcaga gtggaaagaa     420
   aaggcaataa aggagctaga agaatggtat gcaagacagg acgagcagct acagaaaaca     480
   aaagcaaaca acagggtggc agatgaagct ttctacaaac aacccttcgc tgacgtgatt     540
   ggttatgtca caaacataaa ccatccttgc tacagcctag aacaggcagc agaagaagcc     600
   tttgtaaatg acattgacga gtcgtcccca ggcactgagt gggaacgggt ggcccggctg     660
   tgtgacttta accccaagtc tagcaagcag gccaaagatg tctcccgcat gcgctcagtc     720
   ctcatctccc tcaagcaggc cccgctggtg cactcaagtg atgcttacaa aaaagcctgg     780
   ggcaataatc aggacggagt ggtggccagc cagcctgctc gtgtagtgga cgaacgggag     840
   cagatggcca tcagtggcgg cttcatccgc agggtaacaa atgatgcccg agaaaatgaa     900
   atggatgaaa acctagagca ggtgagcggc atcatcggga acctccgtca catggccctg     960
   gatatgggca atgagatcga tacacagaat cgccagatcg acaggatcat ggagaaggct    1020
   gattccaaca aaaccagaat tgatgaggcc aaccaacgtg caacaaagat gctgggaagt    1080
   ggttctaga                                                            1089
<210> 22
   <211> 605
   <212> PRT
   <213> artificial
<220>
   <223> CLAT-SNAP25-tTA (1)(pCMV-Tet3G)
<400> 22
   Met Ala Glu Leu Asp Pro Phe Gly Ala Pro Ala Gly Ala Pro Gly Gly
   1               5                   10                  15
   Pro Ala Leu Gly Asn Gly Val Ala Gly Ala Gly Glu Glu Asp Pro Ala
               20                  25                  30
   Ala Ala Phe Leu Ala Gln Gln Glu Ser Glu Ile Ala Gly Ile Glu Asn
           35                  40                  45
   Asp Glu Ala Phe Ala Ile Leu Asp Gly Gly Ala Pro Gly Pro Gln Pro
       50                  55                  60
   His Gly Glu Pro Pro Gly Gly Pro Asp Ala Val Asp Gly Val Met Asn
   65                  70                  75                  80
   Gly Glu Tyr Tyr Gln Glu Ser Asn Gly Pro Thr Asp Ser Tyr Ala Ala
                   85                  90                  95
   Ile Ser Gln Val Asp Arg Leu Gln Ser Glu Pro Glu Ser Ile Arg Lys
               100                 105                 110
   Trp Arg Glu Glu Gln Met Glu Arg Leu Glu Ala Leu Asp Ala Asn Ser
           115                 120                 125
   Arg Lys Gln Glu Ala Glu Trp Lys Glu Lys Ala Ile Lys Glu Leu Glu
       130                 135                 140
   Glu Trp Tyr Ala Arg Gln Asp Glu Gln Leu Gln Lys Thr Lys Ala Asn
   145                 150                 155                 160
   Asn Arg Val Ala Asp Glu Ala Phe Tyr Lys Gln Pro Phe Ala Asp Val
                   165                 170                 175
   Ile Gly Tyr Val Thr Asn Ile Asn His Pro Cys Tyr Ser Leu Glu Gln
               180                 185                 190
   Ala Ala Glu Glu Ala Phe Val Asn Asp Ile Asp Glu Ser Ser Pro Gly
           195                 200                 205
   Thr Glu Trp Glu Arg Val Ala Arg Leu Cys Asp Phe Asn Pro Lys Ser
       210                 215                 220
   Ser Lys Gln Ala Lys Asp Val Ser Arg Met Arg Ser Val Leu Ile Ser
   225                 230                 235                 240
   Leu Lys Gln Ala Pro Leu Val His Ser Ser Asp Ala Tyr Lys Lys Ala
                   245                 250                 255
   Trp Gly Asn Asn Gln Asp Gly Val Val Ala Ser Gln Pro Ala Arg Val
               260                 265                 270
   Val Asp Glu Arg Glu Gln Met Ala Ile Ser Gly Gly Phe Ile Arg Arg
           275                 280                 285
   Val Thr Asn Asp Ala Arg Glu Asn Glu Met Asp Glu Asn Leu Glu Gln
       290                 295                 300
   Val Ser Gly Ile Ile Gly Asn Leu Arg His Met Ala Leu Asp Met Gly
   305 310 315 320
   Asn Glu Ile Asp Thr Gln Asn Arg Gln Ile Asp Arg Ile Met Glu Lys
   325 330 335
   Ala Asp Ser Asn Lys Thr Arg Ile Asp Glu Ala Asn Gln Arg Ala Thr
   340 345 350
   Lys Met Leu Gly Ser Gly Ser Arg Leu Asp Lys Ser Lys Val Ile Asn
   355 360 365
   Ser Ala Leu Glu Leu Leu Asn Gly Val Gly Ile Glu Gly Leu Thr Thr
   370 375 380
   Arg Lys Leu Ala Gln Lys Leu Gly Val Glu Gln Pro Thr Leu Tyr Trp
   385 390 395 400
   His Val Lys Asn Lys Arg Ala Leu Leu Asp Ala Leu Pro Ile Glu Met
   405 410 415
   Leu Asp Arg His His Thr His Ser Cys Pro Leu Glu Gly Glu Ser Trp
   420 425 430
   Gln Asp Phe Leu Arg Asn Asn Ala Lys Ser Tyr Arg Cys Ala Leu Leu
   435 440 445
   Ser His Arg Asp Gly Ala Lys Val His Leu Gly Thr Arg Pro Thr Glu
   450 455 460
   Lys Gln Tyr Glu Thr Leu Glu Asn Gln Leu Ala Phe Leu Cys Gln Gln
   465 470 475 480
   Gly Phe Ser Leu Glu Asn Ala Leu Tyr Ala Leu Ser Ala Val Gly His
   485 490 495
   Phe Thr Leu Gly Cys Val Leu Glu Glu Gln Glu His Gln Val Ala Lys
   500 505 510
   Glu Glu Arg Glu Thr Pro Thr Thr Asp Ser Met Pro Pro Leu Leu Lys
   515 520 525
   Gln Ala Ile Glu Leu Phe Asp Arg Gln Gly Ala Glu Pro Ala Phe Leu
   530 535 540
   Phe Gly Leu Glu Leu Ile Ile Cys Gly Leu Glu Lys Gln Leu Lys Cys
   545 550 555 560
   Glu Ser Gly Gly Pro Thr Asp Ala Leu Asp Asp Phe Asp Leu Asp Met
                   565                 570                 575
   Leu Pro Ala Asp Ala Leu Asp Asp Phe Asp Leu Asp Met Leu Pro Ala
               580                 585                 590
   Asp Ala Leu Asp Asp Phe Asp Leu Asp Met Leu Pro Gly
           595                 600                 605
<210> 23
   <211> 3731
   <212> DNA
   <213> artificial
<220>
   <223> tTA-SNAP25-SV2 (pCMV-Tet3G)
<400> 23
   gaattcacca tgtctagact ggacaagagc aaagtcataa actctgctct ggaattactc      60
   aatggagtcg gtatcgaagg cctgacgaca aggaaactcg ctcaaaagct gggagttgag     120
   cagcctaccc tgtactggca cgtgaagaac aagcgggccc tgctcgatgc cctgccaatc     180
   gagatgctgg acaggcatca tacccactcc tgccccctgg aaggcgagtc atggcaagac    240
   tttctgcgga acaacgccaa gtcataccgc tgtgctctcc tctcacatcg cgacggggct     300
   aaagtgcatc tcggcacccg cccaacagag aaacagtacg aaaccctgga aaatcagctc     360
   gcgttcctgt gtcagcaagg cttctccctg gagaacgcac tgtacgctct gtccgccgtg     420
   ggccacttta cactgggctg cgtattggag gaacaggagc atcaagtagc aaaagaggaa     480
   agagagacac ctaccaccga ttctatgccc ccacttctga aacaagcaat tgagctgttc     540
   gaccggcagg gagccgaacc tgccttcctt ttcggcctgg aactaatcat atgtggcctg     600
   gagaaacagc taaagtgcga aagcggcggg ccgaccgacg cccttgacga ttttgactta     660
   gacatgctcc cagccgatgc ccttgacgac tttgaccttg atatgctgcc tgctgacgct     720
   cttgacgatt ttgaccttga catgctcccc ggggccggcg ccggcagcgg agtggtggcc     780
   agccagcctg ctcgtgtagt ggacgaacgg gagcagatgg ccatcagtgg cggcttcatc     840
   cgcagggtaa caaatgatgc ccgagaaaat gaaatggatg aaaacctaga gcaggtgagc     900
   ggcatcatcg ggaacctccg tcacatggcc ctggatatgg gcaatgagat cgatacacag     960
   aatcgccaga tcgacaggat catggagaag gctgattcca acaaaaccag aattgatgag    1020
   gccaaccaac gtgcaacaaa gatgctggga agtggtgccg gcgccggcag cgaagactct    1080
   tacaaggata ggacttcact gatgaagggt gccaaggaca ttgccagaga ggtgaagaaa    1140
   caaacagtaa agaaggtgaa tcaagctgtg gaccgagccc aggatgaata cacccagagg    1200
   tcctacagtc ggttccaaga tgaagaagat gatgatgact actacccggc tggagaaacc    1260
   tataatggtg aggccaacga tgacgaaggc tcaagtgaag ccactgaggg gcatgatgaa 1320
   gatgatgaga tctatgaggg ggagtatcag ggcatcccca gtatgaacca agcgaaggac 1380
   agcatcgtgt cagtggggca gcccaagggc gatgagtaca aggaccgacg ggagctggaa 1440
   tcagaaagga gagctgacga ggaagagtta gcccagcagt atgagctgat aatccaagaa 1500
   tgcggtcatg gtcgttttca gtgggccctt ttcttcgtcc tgggcatggc tcttatggca 1560
   gacggtgtag aggtgtttgt cgttggcttc gtgttaccca gtgctgagac agacctctgc 1620
   atcccaaatt caggatctgg atggctaggc agcatagtgt acctcgggat gatggtgggg 1680
   gcgttcttct ggggaggact ggcagacaaa gtgggaagga aacagtctct tctgatttgc 1740
   atgtctgtca acggattctt tgccttcctt tcttcatttg tccaaggtta tggcttcttt 1800
   ctcttctgtc gcttactttc tggattcggg attggaggag ccatacccac tgtgttctcg 1860
   tactttgctg aagtcctggc ccgggaaaag cggggcgaac acttgagctg gctctgcatg 1920
   ttctggatga tcggtggcat ctacgcctct gccatggcct gggccatcat cccgcactac 1980
   gggtggagct tcagcatggg atcggcctac cagtttcaca gttggcgtgt gtttgtcatc 2040
   gtctgtgcac tcccctgtgt ctcctccgtg gtggccctca cattcatgcc tgaaagccca 2100
   cgattcttgt tggaggttgg aaaacatgat gaagcttgga tgattctgaa gttaattcat 2160
   gacaccaaca tgagagcccg gggtcagcct gagaaggtct tcacggtaaa caaaataaaa 2220
   actcctaaac aaatagatga gctgattgaa attgagagtg acacaggaac atggtatagg 2280
   aggtgttttg ttcggatccg caccgagctg tacggaattt ggttgacttt tatgagatgt 2340
   ttcaactacc cagtcaggga taatacaata aagcttacaa ttgtttggtt caccctgtcc 2400
   tttgggtact atggattatc cgtttggttc cctgatgtca ttaaacctct gcagtccgat 2460
   gaatatgcat tgctaaccag aaatgtggag agagataaat atgcaaattt cactattaac 2520
   tttacaatgg aaaatcagat tcatactgga atggaatacg acaatggcag attcataggg 2580
   gtcaagttca aatctgtaac tttcaaagac tctgttttta agtcctgcac ctttgaggat 2640
   gtaacttcag tgaacaccta cttcaagaac tgcacattta ttgacactgt ttttgacaac 2700
   acagattttg agccatataa attcattgac agtgaattta aaaactgctc gttttttcac 2760
   aacaagacgg gatgtcagat tacctttgat gatgactata gtgcctactg gatttatttt 2820
   gtcaactttc tggggacatt ggcagtattg ccagggaaca ttgtgtctgc tctgctgatg 2880
   gacagaattg ggcgcttaac aatgctaggt ggctctatgg tgctttcggg gatcagctgt 2940
   ttcttccttt ggttcggcac cagtgaatcc atgatgatag gcatgctgtg tctgtacaat 3000
   ggattgacca tctcagcctg gaactctctt gacgtggtca ctgtggaact gtaccccaca 3060
   gaccggaggg caacaggctt tggcttctta aatgcgctat gcaaggcagc agccgtcctg 3120
   ggaaacttaa tatttggctc tctggtcagc atcaccaaat caatccccat cctgctggct 3180
   tctactgtgc tcgtgtgtgg aggactcgtt gggctgtgcc tgcctgacac acgaacccag 3240
   gttctgtaac taagtaagga tccagacatg ataagataca ttgatgagtt tggacaaacc 3300
   acaactagaa tgcagtgaaa aaaatgcttt atttgtgaaa tttgtgatgc tattgcttta 3360
   tttgtaacca ttataagctg caataaacaa gttaacaaca acaattgcat tcattttatg 3420
   tttcaggttc agggggaggt gtgggaggtt ttttaaagca agtaaaacct ctacaaatgt 3480
   ggtatggctg attatgatcc tgcaagcctc gtcgtcctgg ccggaccacg ctatctgtgc 3540
   aaggtccccg gccccggacg cgcgctccat gagcagagcg cccgccgccg aggcgaagac 3600
   tcgggcggcg ccctgcccgt cccaccaggt caacaggcgg taaccggcct cttcatcggg 3660
   aatgcgcgcg accttcagca tcgccggcat gtccccctgg cggacgggaa gtatccagct 3720
   cgaccaagct t 3731
<210> 24
   <211> 1079
   <212> PRT
   <213> artificial
<220>
   <223> tTA-SNAP25-SV2(pCMV-TEt3G)
<400> 24
<210> 25
   <211> 3311
   <212> DNA
   <213> artificial
<220>
   <223> tTA-SNAP25-Cholinetransporter(pCMV-Tet3G)
<400> 25
<210> 26
   <211> 939
   <212> PRT
   <213> artificial
<220>
   <223> tTA-SNAP25-Cholinetransporter(pCMV-Tet3G)
<400> 26
   Met Ser Arg Leu Asp Lys Ser Lys Val Ile Asn Ser Ala Leu Glu Leu
   1               5                   10                  15
   Leu Asn Gly Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Pro Ile Glu Met Leu Asp Arg His His
       50                  55                  60
   Thr His Ser Cys Pro Leu Glu Gly Glu Ser Trp Gln Asp Phe Leu Arg
   65                  70                  75                  80
   Asn Asn Ala Lys Ser Tyr Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Glu Gln Glu His Gln Val Ala Lys Glu Glu Arg Glu Thr
   145                 150                 155                 160
   Pro Thr Thr Asp Ser Met Pro Pro Leu Leu Lys Gln Ala Ile Glu Leu
                   165                 170                 175
   Phe Asp Arg Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Gly Pro
           195                 200                 205
   Thr Asp Ala Leu Asp Asp Phe Asp Leu Asp Met Leu Pro Ala Asp Ala
       210                 215                 220
   Leu Asp Asp Phe Asp Leu Asp Met Leu Pro Ala Asp Ala Leu Asp Asp
   225                 230                 235                 240
   Phe Asp Leu Asp Met Leu Pro Gly Ala Gly Ala Gly Ser Gly Val Val
                   245                 250                 255
   Ala Ser Gln Pro Ala Arg Val Val Asp Glu Arg Glu Gln Met Ala Ile
               260                 265                 270
   Ser Gly Gly Phe Ile Arg Arg Val Thr Asn Asp Ala Arg Glu Asn Glu
   275 280 285
   Met Asp Glu Asn Leu Glu Gln Val Ser Gly Ile Ile Gly Asn Leu Arg
   290 295 300
   His Met Ala Leu Asp Met Gly Asn Glu Ile Asp Thr Gln Asn Arg Gln
   305 310 315 320
   Ile Asp Arg Ile Met Glu Lys Ala Asp Ser Asn Lys Thr Arg Ile Asp
   325 330 335
   Glu Ala Asn Gln Arg Ala Thr Lys Met Leu Gly Ser Gly Ala Gly Ala
   340 345 350
   Gly Ser Gly Ala Gly Ala Gly Ser Ala Phe His Val Glu Gly Leu Ile
   355 360 365
   Ala Ile Ile Val Phe Tyr Leu Leu Ile Leu Leu Val Gly Ile Trp Ala
   370 375 380
   Ala Trp Arg Thr Lys Asn Ser Gly Ser Ala Glu Glu Arg Ser Glu Ala
   385 390 395 400
   Ile Ile Val Gly Gly Arg Asp Ile Gly Leu Leu Val Gly Gly Phe Thr
   405 410 415
   Met Thr Ala Thr Trp Val Gly Gly Gly Tyr Ile Asn Gly Thr Ala Glu
   420 425 430
   Ala Val Tyr Val Pro Gly Tyr Gly Leu Ala Trp Ala Gln Ala Pro Ile
   435 440 445
   Gly Tyr Ser Leu Ser Leu Ile Leu Gly Gly Leu Phe Phe Ala Lys Pro
   450 455 460
   Met Arg Ser Lys Gly Tyr Val Thr Met Leu Asp Pro Phe Gln Gln Ile
   465 470 475 480
   Tyr Gly Lys Arg Met Gly Gly Leu Leu Phe Ile Pro Ala Leu Met Gly
   485 490 495
   Glu Met Phe Trp Ala Ala Ala Ile Phe Ser Ala Leu Gly Ala Thr Ile
   500 505 510
   Ser Val Ile Ile Asp Val Asp Met His Ile Ser Val Ile Ile Ser Ala
   515 520 525
   Leu Ile Ala Thr Leu Tyr Thr Leu Val Gly Gly Leu Tyr Ser Val Ala
       530                 535                 540
   Tyr Thr Asp Val Val Gln Leu Phe Cys Ile Phe Val Gly Leu Trp Ile
   545                 550                 555                 560
   Ser Val Pro Phe Ala Leu Ser His Pro Ala Val Ala Asp Ile Gly Phe
                   565                 570                 575
   Thr Ala Val His Ala Lys Tyr Gln Lys Pro Trp Leu Gly Thr Val Asp
               580                 585                 590
   Ser Ser Glu Val Tyr Ser Trp Leu Asp Ser Phe Leu Leu Leu Met Leu
           595                 600                 605
   Gly Gly Ile Pro Trp Gln Ala Tyr Phe Gln Arg Val Leu Ser Ser Ser
       610                 615                 620
   Ser Ala Thr Tyr Ala Gln Val Leu Ser Phe Leu Ala Ala Phe Gly Cys
   625                 630                 635                 640
   Leu Val Met Ala Ile Pro Ala Ile Leu Ile Gly Ala Ile Gly Ala Ser
                   645                 650                 655
   Thr Asp Trp Asn Gln Thr Ala Tyr Gly Leu Pro Asp Pro Lys Thr Thr
               660                 665                 670
   Glu Glu Ala Asp Met Ile Leu Pro Ile Val Leu Gln Tyr Leu Cys Pro
           675                 680                 685
   Val Tyr Ile Ser Phe Phe Gly Leu Gly Ala Val Ser Ala Ala Val Met
       690                 695                 700
   Ser Ser Ala Asp Ser Ser Ile Leu Ser Ala Ser Ser Met Phe Ala Arg
   705                 710                 715                 720
   Asn Ile Tyr Gln Leu Ser Phe Arg Gln Asn Ala Ser Asp Lys Glu Ile
                   725                 730                 735
   Val Trp Val Met Arg Ile Thr Val Phe Val Phe Gly Ala Ser Ala Thr
               740                 745                 750
   Ala Met Ala Leu Leu Thr Lys Thr Val Tyr Gly Leu Trp Tyr Leu Ser
           755                 760                 765
   Ser Asp Leu Val Tyr Ile Val Ile Phe Pro Gln Leu Leu Cys Val Leu
       770                 775                 780
   Phe Val Lys Gly Thr Asn Thr Tyr Gly Ala Val Ala Gly Tyr Val Ser
   785                 790                 795                 800
   Gly Leu Phe Leu Arg Ile Thr Gly Gly Glu Pro Tyr Leu Tyr Leu Gln
                   805                 810                 815
   Pro Leu Ile Phe Tyr Pro Gly Tyr Tyr Pro Asp Asp Asn Gly Ile Tyr
               820                 825                 830
   Asn Gln Lys Phe Pro Phe Lys Thr Leu Ala Met Val Thr Ser Phe Leu
           835                 840                 845
   Thr Asn Ile Cys Ile Ser Tyr Leu Ala Lys Tyr Leu Phe Glu Ser Gly
       850                 855                 860
   Thr Leu Pro Pro Lys Leu Asp Val Phe Asp Ala Val Val Ala Arg His
   865                 870                 875                 880
   Ser Glu Glu Asn Met Asp Lys Thr Ile Leu Val Lys Asn Glu Asn Ile
                   885                 890                 895
   Lys Leu Asp Glu Leu Ala Leu Val Lys Pro Arg Gln Ser Met Thr Leu
               900                 905                 910
   Ser Ser Thr Phe Thr Asn Lys Glu Ala Phe Leu Asp Val Asp Ser Ser
           915                 920                 925
   Pro Glu Gly Ser Gly Thr Glu Asp Asn Leu Gln
       930                 935
<210> 27
   <211> 2198
   <212> DNA
   <213> artificial
<220>
   <223> tTA-SNAP25-GST(pCMV-Tet3G)
<400> 27
   gaattcacca tgtctagact ggacaagagc aaagtcataa actctgctct ggaattactc      60
   aatggagtcg gtatcgaagg cctgacgaca aggaaactcg ctcaaaagct gggagttgag     120
   cagcctaccc tgtactggca cgtgaagaac aagcgggccc tgctcgatgc cctgccaatc     180
   gagatgctgg acaggcatca tacccactcc tgccccctgg aaggcgagtc atggcaagac     240
   tttctgcgga acaacgccaa gtcataccgc tgtgctctcc tctcacatcg cgacggggct     300
   aaagtgcatc tcggcacccg cccaacagag aaacagtacg aaaccctgga aaatcagctc     360
   gcgttcctgt gtcagcaagg cttctccctg gagaacgcac tgtacgctct gtccgccgtg 420
   ggccacttta cactgggctg cgtattggag gaacaggagc atcaagtagc aaaagaggaa 480
   agagagacac ctaccaccga ttctatgccc ccacttctga aacaagcaat tgagctgttc 540
   gaccggcagg gagccgaacc tgccttcctt ttcggcctgg aactaatcat atgtggcctg 600
   gagaaacagc taaagtgcga aagcggcggg ccgaccgacg cccttgacga ttttgactta 660
   gacatgctcc cagccgatgc ccttgacgac tttgaccttg atatgctgcc tgctgacgct 720
   cttgacgatt ttgaccttga catgctcccc ggggccggcg ccggcagcgg agtggtggcc 780
   agccagcctg ctcgtgtagt ggacgaacgg gagcagatgg ccatcagtgg cggcttcatc 840
   cgcagggtaa caaatgatgc ccgagaaaat gaaatggatg aaaacctaga gcaggtgagc 900
   ggcatcatcg ggaacctccg tcacatggcc ctggatatgg gcaatgagat cgatacacag 960
   aatcgccaga tcgacaggat catggagaag gctgattcca acaaaaccag aattgatgag 1020
   gccaaccaac gtgcaacaaa gatgctggga agtggttccc ctatactagg ttattggaaa 1080
   attaagggcc ttgtgcaacc cactcgactt cttttggaat atcttgaaga aaaatatgaa 1140
   gagcatttgt atgagcgcga tgaaggtgat aaatggcgaa acaaaaagtt tgaattgggt 1200
   ttggagtttc ccaatcttcc ttattatatt gatggtgatg ttaaattaac acagtctatg 1260
   gccatcatac gttatatagc tgacaagcac aacatgttgg gtggttgtcc aaaagagcgt 1320
   gcagagattt caatgcttga aggagcggtt ttggatatta gatacggtgt ttcgagaatt 1380
   gcatatagta aagactttga aactctcaaa gttgattttc ttagcaagct acctgaaatg 1440
   ctgaaaatgt tcgaagatcg tttatgtcat aaaacatatt taaatggtga tcatgtaacc 1500
   catcctgact tcatgttgta tgacgctctt gatgttgttt tatacatgga cccaatgtgc 1560
   ctggatgcgt tcccaaaatt agtttgtttt aaaaaacgta ttgaagctat cccacaaatt 1620
   gataagtact tgaaatccag caagtatata gcatggcctt tgcagggctg gcaagccacg 1680
   tttggtggtg gcgaccatcc tccaaaatcg gattaactaa gtaaggatcc agacatgata 1740
   agatacattg atgagtttgg acaaaccaca actagaatgc agtgaaaaaa atgctttatt 1800
   tgtgaaattt gtgatgctat tgctttattt gtaaccatta taagctgcaa taaacaagtt 1860
   aacaacaaca attgcattca ttttatgttt caggttcagg gggaggtgtg ggaggttttt 1920
   taaagcaagt aaaacctcta caaatgtggt atggctgatt atgatcctgc aagcctcgtc 1980
   gtcctggccg gaccacgcta tctgtgcaag gtccccggcc ccggacgcgc gctccatgag 2040
   cagagcgccc gccgccgagg cgaagactcg ggcggcgccc tgcccgtccc accaggtcaa 2100
   caggcggtaa ccggcctctt catcgggaat gcgcgcgacc ttcagcatcg ccggcatgtc 2160
   cccctggcgg acgggaagta tccagctcga ccaagctt 2198
<210> 28
   <211> 569
   <212> PRT
   <213> artificial
<220>
   <223> tTA-SNAP25-GST(pCMV-Tet3G)
<400> 28
   Met Ser Arg Leu Asp Lys Ser Lys Val Ile Asn Ser Ala Leu Glu Leu
   1               5                   10                  15
   Leu Asn Gly Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Pro Ile Glu Met Leu Asp Arg His His
       50                  55                  60
   Thr His Ser Cys Pro Leu Glu Gly Glu Ser Trp Gln Asp Phe Leu Arg
   65                  70                  75                  80
   Asn Asn Ala Lys Ser Tyr Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Glu Gln Glu His Gln Val Ala Lys Glu Glu Arg Glu Thr
   145                 150                 155                 160
   Pro Thr Thr Asp Ser Met Pro Pro Leu Leu Lys Gln Ala Ile Glu Leu
                   165                 170                 175
   Phe Asp Arg Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Gly Pro
           195                 200                 205
   Thr Asp Ala Leu Asp Asp Phe Asp Leu Asp Met Leu Pro Ala Asp Ala
       210                 215                 220
   Leu Asp Asp Phe Asp Leu Asp Met Leu Pro Ala Asp Ala Leu Asp Asp
   225                 230                 235                 240
   Phe Asp Leu Asp Met Leu Pro Gly Ala Gly Ala Gly Ser Gly Val Val
                   245                 250                 255
   Ala Ser Gln Pro Ala Arg Val Val Asp Glu Arg Glu Gln Met Ala Ile
               260                 265                 270
   Ser Gly Gly Phe Ile Arg Arg Val Thr Asn Asp Ala Arg Glu Asn Glu
           275                 280                 285
   Met Asp Glu Asn Leu Glu Gln Val Ser Gly Ile Ile Gly Asn Leu Arg
       290                 295                 300
   His Met Ala Leu Asp Met Gly Asn Glu Ile Asp Thr Gln Asn Arg Gln
   305                 310                 315                 320
   Ile Asp Arg Ile Met Glu Lys Ala Asp Ser Asn Lys Thr Arg Ile Asp
                   325                 330                 335
   Glu Ala Asn Gln Arg Ala Thr Lys Met Leu Gly Ser Gly Met Ser Pro
               340                 345                 350
   Ile Leu Gly Tyr Trp Lys Ile Lys Gly Leu Val Gln Pro Thr Arg Leu
           355                 360                 365
   Leu Leu Glu Tyr Leu Glu Glu Lys Tyr Glu Glu His Leu Tyr Glu Arg
       370                 375                 380
   Asp Glu Gly Asp Lys Trp Arg Asn Lys Lys Phe Glu Leu Gly Leu Glu
   385                 390                 395                 400
   Phe Pro Asn Leu Pro Tyr Tyr Ile Asp Gly Asp Val Lys Leu Thr Gln
                   405                 410                 415
   Ser Met Ala Ile Ile Arg Tyr Ile Ala Asp Lys His Asn Met Leu Gly
               420                 425                 430
   Gly Cys Pro Lys Glu Arg Ala Glu Ile Ser Met Leu Glu Gly Ala Val
           435                 440                 445
   Leu Asp Ile Arg Tyr Gly Val Ser Arg Ile Ala Tyr Ser Lys Asp Phe
       450                 455                 460
   Glu Thr Leu Lys Val Asp Phe Leu Ser Lys Leu Pro Glu Met Leu Lys
   465                 470                 475                 480
   Met Phe Glu Asp Arg Leu Cys His Lys Thr Tyr Leu Asn Gly Asp His
                   485                 490                 495
   Val Thr His Pro Asp Phe Met Leu Tyr Asp Ala Leu Asp Val Val Leu
               500                 505                 510
   Tyr Met Asp Pro Met Cys Leu Asp Ala Phe Pro Lys Leu Val Cys Phe
           515                 520                 525
   Lys Lys Arg Ile Glu Ala Ile Pro Gln Ile Asp Lys Tyr Leu Lys Ser
       530                 535                 540
   Ser Lys Tyr Ile Ala Trp Pro Leu Gln Gly Trp Gln Ala Thr Phe Gly
   545                 550                 555                 560
   Gly Gly Asp His Pro Pro Lys Ser Asp
                   565
<210> 29
   <211> 2255
   <212> DNA
   <213> artificial
<220>
   <223> tTA-SNAP25-GFP(pCMV-Tet3G)
<400> 29
   gaattcacca tgtctagact ggacaagagc aaagtcataa actctgctct ggaattactc     60
   aatggagtcg gtatcgaagg cctgacgaca aggaaactcg ctcaaaagct gggagttgag    120
   cagcctaccc tgtactggca cgtgaagaac aagcgggccc tgctcgatgc cctgccaatc    180
   gagatgctgg acaggcatca tacccactcc tgccccctgg aaggcgagtc atggcaagac    240
   tttctgcgga acaacgccaa gtcataccgc tgtgctctcc tctcacatcg cgacggggct    300
   aaagtgcatc tcggcacccg cccaacagag aaacagtacg aaaccctgga aaatcagctc    360
   gcgttcctgt gtcagcaagg cttctccctg gagaacgcac tgtacgctct gtccgccgtg    420
   ggccacttta cactgggctg cgtattggag gaacaggagc atcaagtagc aaaagaggaa    480
   agagagacac ctaccaccga ttctatgccc ccacttctga aacaagcaat tgagctgttc    540
   gaccggcagg gagccgaacc tgccttcctt ttcggcctgg aactaatcat atgtggcctg    600
   gagaaacagc taaagtgcga aagcggcggg ccgaccgacg cccttgacga ttttgactta    660
   gacatgctcc cagccgatgc ccttgacgac tttgaccttg atatgctgcc tgctgacgct    720
   cttgacgatt ttgaccttga catgctcccc ggggccggcg ccggcagcgg agtggtggcc    780
   agccagcctg ctcgtgtagt ggacgaacgg gagcagatgg ccatcagtgg cggcttcatc 840
   cgcagggtaa caaatgatgc ccgagaaaat gaaatggatg aaaacctaga gcaggtgagc 900
   ggcatcatcg ggaacctccg tcacatggcc ctggatatgg gcaatgagat cgatacacag 960
   aatcgccaga tcgacaggat catggagaag gctgattcca acaaaaccag aattgatgag 1020
   gccaaccaac gtgcaacaaa gatgctggga agtggtgcca gcaaaggaga agaacttttc 1080
   actggagttg tcccaattct tgttgaatta gatggtgatg ttaatgggca caaattttct 1140
   gtcagtggag agggtgaagg tgatgctaca tacggaaagc ttacccttaa atttatttgc 1200
   actactggaa aactacctgt tccatggcca acacttgtca ctactttctc ttatggtgtt 1260
   caatgctttt cccgttatcc ggatcatatg aaacggcatg actttttcaa gagtgccatg 1320
   cccgaaggtt atgtacagga acgcactata tctttcaaag atgacgggaa ctacaagacg 1380
   cgtgctgaag tcaagtttga aggtgatacc cttgttaatc gtatcgagtt aaaaggtatt 1440
   gattttaaag aagatggaaa cattctcgga cacaaactcg agtacaacta taactcacac 1500
   aatgtataca tcacggcaga caaacaaaag aatggaatca aagctaactt caaaattcgc 1560
   cacaacattg aagatggatc cgttcaacta gcagaccatt atcaacaaaa tactccaatt 1620
   ggcgatggcc ctgtcctttt accagacaac cattacctgt cgacacaatc tgccctttcg 1680
   aaagatccca acgaaaagcg tgaccacatg gtccttcttg agtttgtaac tgctgctggg 1740
   attacacatg gcatggatga gctctacaaa taactaagta aggatccaga catgataaga 1800
   tacattgatg agtttggaca aaccacaact agaatgcagt gaaaaaaatg ctttatttgt 1860
   gaaatttgtg atgctattgc tttatttgta accattataa gctgcaataa acaagttaac 1920
   aacaacaatt gcattcattt tatgtttcag gttcaggggg aggtgtggga ggttttttaa 1980
   agcaagtaaa acctctacaa atgtggtatg gctgattatg atcctgcaag cctcgtcgtc 2040
   ctggccggac cacgctatct gtgcaaggtc cccggccccg gacgcgcgct ccatgagcag 2100
   agcgcccgcc gccgaggcga agactcgggc ggcgccctgc ccgtcccacc aggtcaacag 2160
   gcggtaaccg gcctcttcat cgggaatgcg cgcgaccttc agcatcgccg gcatgtcccc 2220
   ctggcggacg ggaagtatcc agctcgacca agctt 2255
<210> 30
   <211> 696
   <212> PRT
   <213> artificial
<220>
   <223> tTA-SNAP25-GFP(pCMV-Tet3G)
<400> 30
   Met Ser Arg Leu Asp Lys Ser Lys Val Ile Asn Ser Ala Leu Glu Leu
   1               5                   10                  15
   Leu Asn Gly Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Pro Ile Glu Met Leu Asp Arg His His
       50                  55                  60
   Thr His Ser Cys Pro Leu Glu Gly Glu Ser Trp Gln Asp Phe Leu Arg
   65                  70                  75                  80
   Asn Asn Ala Lys Ser Tyr Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Glu Gln Glu His Gln Val Ala Lys Glu Glu Arg Glu Thr
   145                 150                 155                 160
   Pro Thr Thr Asp Ser Met Pro Pro Leu Leu Lys Gln Ala Ile Glu Leu
                   165                 170                 175
   Phe Asp Arg Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Gly Pro
           195                 200                 205
   Thr Asp Ala Leu Asp Asp Phe Asp Leu Asp Met Leu Pro Ala Asp Ala
       210                 215                 220
   Leu Asp Asp Phe Asp Leu Asp Met Leu Pro Ala Asp Ala Leu Asp Asp
   225                 230                 235                 240
   Phe Asp Leu Asp Met Leu Pro Gly Ala Gly Ala Gly Ser Gly Val Val
                   245                 250                 255
   Ala Ser Gln Pro Ala Arg Val Val Asp Glu Arg Glu Gln Met Ala Ile
               260                 265                 270
   Ser Gly Gly Phe Ile Arg Arg Val Thr Asn Asp Ala Arg Glu Asn Glu
           275                 280                 285
   Met Asp Glu Asn Leu Glu Gln Val Ser Gly Ile Ile Gly Asn Leu Arg
       290                 295                 300
   His Met Ala Leu Asp Met Gly Asn Glu Ile Asp Thr Gln Asn Arg Gln
   305                 310                 315                 320
   Ile Asp Arg Ile Met Glu Lys Ala Asp Ser Asn Lys Thr Arg Ile Asp
                   325                 330                 335
   Glu Ala Asn Gln Arg Ala Thr Lys Met Leu Gly Ser Gly Ala Ser Lys
               340                 345                 350
   Gly Glu Glu Leu Phe Thr Gly Val Val Pro Ile Leu Val Glu Leu Asp
           355                 360                 365
   Gly Asp Val Asn Gly His Lys Phe Ser Val Ser Gly Glu Gly Glu Gly
       370                 375                 380
   Asp Ala Thr Tyr Gly Lys Leu Thr Leu Lys Phe Ile Cys Thr Thr Gly
   385                 390                 395                 400
   Lys Leu Pro Val Pro Trp Pro Thr Leu Val Thr Thr Phe Ser Tyr Gly
                   405                 410                 415
   Val Gln Cys Phe Ser Arg Tyr Pro Asp His Met Lys Arg His Asp Phe
               420                 425                 430
   Phe Lys Ser Ala Met Pro Glu Gly Tyr Val Gln Glu Arg Thr Ile Ser
           435                 440                 445
   Phe Lys Asp Asp Gly Asn Tyr Lys Thr Arg Ala Glu Val Lys Phe Glu
       450                 455                 460
   Gly Asp Thr Leu Val Asn Arg Ile Glu Leu Lys Gly Ile Asp Phe Lys
   465                 470                 475                 480
   Glu Asp Gly Asn Ile Leu Gly His Lys Leu Glu Tyr Asn Tyr Asn Ser
                   485                 490                 495
   His Asn Val Tyr Ile Thr Ala Asp Lys Gln Lys Asn Gly Ile Lys Ala
               500                 505                 510
   Asn Phe Lys Ile Arg His Asn Ile Glu Asp Gly Ser Val Gln Leu Ala
           515                 520                 525
   Asp His Tyr Gln Gln Asn Thr Pro Ile Gly Asp Gly Pro Val Leu Leu
       530                 535                 540
   Pro Asp Asn His Tyr Leu Ser Thr Gln Ser Ala Leu Ser Lys Asp Pro
   545                 550                 555                 560
   Asn Glu Lys Arg Asp His Met Val Leu Leu Glu Phe Val Thr Ala Ala
                   565                 570                 575
   Gly Ile Thr His Gly Met Asp Glu Leu Tyr Lys Ala Glu Asp Ala Asp
               580                 585                 590
   Met Arg Asn Glu Leu Glu Glu Met Gln Arg Arg Ala Asp Gln Leu Ala
           595                 600                 605
   Asp Glu Ser Leu Glu Ser Thr Arg Arg Met Leu Gln Leu Val Glu Glu
       610                 615                 620
   Ser Lys Asp Ala Gly Ile Arg Thr Leu Val Met Leu Asp Glu Gln Gly
   625                 630                 635                 640
   Glu Gln Leu Glu Arg Ile Glu Glu Gly Met Asp Gln Ile Asn Lys Asp
                   645                 650                 655
   Met Lys Glu Ala Glu Lys Asn Leu Thr Asp Leu Gly Lys Phe Cys Gly
               660                 665                 670
   Leu Cys Val Cys Pro Cys Asn Lys Leu Lys Ser Ser Asp Ala Tyr Lys
           675                 680                 685
   Lys Ala Trp Gly Asn Asn Gln Asp
       690                 695
<210> 31
   <211> 444
   <212> PRT
   <213> artificial
<220>
   <223> SNAP110-pTET-tTAK
<400> 31
   Ser Ser Asp Ala Tyr Lys Lys Ala Trp Gly Asn Asn Gln Asp Gly Val
   1               5                   10                  15
   Val Ala Ser Gln Pro Ala Arg Val Val Asp Glu Arg Glu Gln Met Ala
               20                  25                  30
   Ile Ser Gly Gly Phe Ile Arg Arg Val Thr Asn Asp Ala Arg Glu Asn
           35                  40                  45
   Glu Met Asp Glu Asn Leu Glu Gln Val Ser Gly Ile Ile Gly Asn Leu
       50                  55                  60
   Arg His Met Ala Leu Asp Met Gly Asn Glu Ile Asp Thr Gln Asn Arg
   65                  70                  75                  80
   Gln Ile Asp Arg Ile Met Glu Lys Ala Asp Ser Asn Lys Thr Arg Ile
                   85                  90                  95
   Asp Glu Ala Asn Gln Arg Ala Thr Lys Met Leu Gly Ser Gly Ser Arg
               100                 105                 110
   Leu Asp Lys Ser Lys Val Ile Asn Ser Ala Leu Glu Leu Leu Asn Glu
           115                 120                 125
   Val Gly Ile Glu Gly Leu Thr Thr Arg Lys Leu Ala Gln Lys Leu Gly
       130                 135                 140
   Val Glu Gln Pro Thr Leu Tyr Trp His Val Lys Asn Lys Arg Ala Leu
   145                 150                 155                 160
   Leu Asp Ala Leu Ala Ile Glu Met Leu Asp Arg His His Thr His Phe
                   165                 170                 175
   Cys Pro Leu Glu Gly Glu Ser Trp Gln Asp Phe Leu Arg Asn Asn Ala
               180                 185                 190
   Lys Ser Phe Arg Cys Ala Leu Leu Ser His Arg Asp Gly Ala Lys Val
           195                 200                 205
   His Leu Gly Thr Arg Pro Thr Glu Lys Gln Tyr Glu Thr Leu Glu Asn
       210                 215                 220
   Gln Leu Ala Phe Leu Cys Gln Gln Gly Phe Ser Leu Glu Asn Ala Leu
   225                 230                 235                 240
   Tyr Ala Leu Ser Ala Val Gly His Phe Thr Leu Gly Cys Val Leu Glu
                   245                 250                 255
   Asp Gln Glu His Gln Val Ala Lys Glu Glu Arg Glu Thr Pro Thr Thr
               260                 265                 270
   Asp Ser Met Pro Pro Leu Leu Arg Gln Ala Ile Glu Leu Phe Asp His
           275                 280                 285
   Gln Gly Ala Glu Pro Ala Phe Leu Phe Gly Leu Glu Leu Ile Ile Cys
       290                 295                 300
   Gly Leu Glu Lys Gln Leu Lys Cys Glu Ser Gly Ser Ala Tyr Ser Arg
   305                 310                 315                 320
   Ala Arg Thr Lys Asn Asn Tyr Gly Ser Thr Ile Glu Gly Leu Leu Asp
                   325                 330                 335
   Leu Pro Asp Asp Asp Ala Pro Glu Glu Ala Gly Leu Ala Ala Pro Arg
               340                 345                 350
   Leu Ser Phe Leu Pro Ala Gly His Thr Arg Arg Leu Ser Thr Ala Pro
           355                 360                 365
   Pro Thr Asp Val Ser Leu Gly Asp Glu Leu His Leu Asp Gly Glu Asp
       370                 375                 380
   Val Ala Met Ala His Ala Asp Ala Leu Asp Asp Phe Asp Leu Asp Met
   385                 390                 395                 400
   Leu Gly Asp Gly Asp Ser Pro Gly Pro Gly Phe Thr Pro His Asp Ser
                   405                 410                 415
   Ala Pro Tyr Gly Ala Leu Asp Met Ala Asp Phe Glu Phe Glu Gln Met
               420                 425                 430
   Phe Thr Asp Ala Leu Gly Ile Asp Glu Tyr Gly Gly
           435                 440
<210> 32
   <211> 10
   <212> PRT
   <213> artificial
<220>
   <223> leucine-rich nuclear export signal (NES)
<220>
   <221> misc_feature
   <222> (2)..(4)
   <223> Xaa can be any naturally occurring amino acid
<220>
   <221> misc_feature
   <222> (6) .. (7)
   <223> Xaa can be any naturally occurring amino acid
<220>
   <221> misc_feature
   <222> (9) .. (9)
   <223> Xaa can be any naturally occurring amino acid
<400> 32
   Leu Xaa Xaa Xaa Leu Xaa Xaa Leu Xaa Leu
   1               5                   10
<210> 33
   <211> 247
   <212> PRT
   <213> artificial
<220>
   <223> tetracycline dependent transactivator
<400> 33
   Ser Arg Leu Asp Lys Ser Lys Val Ile Asn Ser Ala Leu Glu Leu Leu
   1               5                   10                  15
   Asn Gly Val Gly Ile Glu Gly Leu Thr Thr Arg Lys Leu Ala Gln Lys
               20                  25                  30
   Leu Gly Val Glu Gln Pro Thr Leu Tyr Trp His Val Lys Asn Lys Arg
           35                  40                  45
   Ala Leu Leu Asp Ala Leu Pro Ile Glu Met Leu Asp Arg His His Thr
       50                  55                  60
   His Ser Cys Pro Leu Glu Gly Glu Ser Trp Gln Asp Phe Leu Arg Asn
   65                  70                  75                  80
   Asn Ala Lys Ser Tyr Arg Cys Ala Leu Leu Ser His Arg Asp Gly Ala
                   85                  90                  95
   Lys Val His Leu Gly Thr Arg Pro Thr Glu Lys Gln Tyr Glu Thr Leu
               100                 105                 110
   Glu Asn Gln Leu Ala Phe Leu Cys Gln Gln Gly Phe Ser Leu Glu Asn
           115                 120                 125
   Ala Leu Tyr Ala Leu Ser Ala Val Gly His Phe Thr Leu Gly Cys Val
       130                 135                 140
   Leu Glu Glu Gln Glu His Gln Val Ala Lys Glu Glu Arg Glu Thr Pro
   145                 150                 155                 160
   Thr Thr Asp Ser Met Pro Pro Leu Leu Lys Gln Ala Ile Glu Leu Phe
                   165                 170                 175
   Asp Arg Gln Gly Ala Glu Pro Ala Phe Leu Phe Gly Leu Glu Leu Ile
               180                 185                 190
   Ile Cys Gly Leu Glu Lys Gln Leu Lys Cys Glu Ser Gly Gly Pro Thr
           195                 200                 205
   Asp Ala Leu Asp Asp Phe Asp Leu Asp Met Leu Pro Ala Asp Ala Leu
       210                 215                 220
   Asp Asp Phe Asp Leu Asp Met Leu Pro Ala Asp Ala Leu Asp Asp Phe
   225                 230                 235                 240
   Asp Leu Asp Met Leu Pro Gly
                   245
<210> 34
   <211> 334
   <212> PRT
   <213> artificial
<220>
   <223> tetracycline dependent transactivator
<400> 34
   Ser Arg Leu Asp Lys Ser Lys Val Ile Asn Ser Ala Leu Glu Leu Leu
   1               5                   10                  15
   Asn Glu Val Gly Ile Glu Gly Leu Thr Thr Arg Lys Leu Ala Gln Lys
               20                  25                  30
   Leu Gly Val Glu Gln Pro Thr Leu Tyr Trp His Val Lys Asn Lys Arg
           35                  40                  45
   Ala Leu Leu Asp Ala Leu Ala Ile Glu Met Leu Asp Arg His His Thr
       50                  55                  60
   His Phe Cys Pro Leu Glu Gly Glu Ser Trp Gln Asp Phe Leu Arg Asn
   65                  70                  75                  80
   Asn Ala Lys Ser Phe Arg Cys Ala Leu Leu Ser His Arg Asp Gly Ala
                   85                  90                  95
   Lys Val His Leu Gly Thr Arg Pro Thr Glu Lys Gln Tyr Glu Thr Leu
               100                 105                 110
   Glu Asn Gln Leu Ala Phe Leu Cys Gln Gln Gly Phe Ser Leu Glu Asn
           115                 120                 125
   Ala Leu Tyr Ala Leu Ser Ala Val Gly His Phe Thr Leu Gly Cys Val
       130                 135                 140
   Leu Glu Asp Gln Glu His Gln Val Ala Lys Glu Glu Arg Glu Thr Pro
   145                 150                 155                 160
   Thr Thr Asp Ser Met Pro Pro Leu Leu Arg Gln Ala Ile Glu Leu Phe
                   165                 170                 175
   Asp His Gln Gly Ala Glu Pro Ala Phe Leu Phe Gly Leu Glu Leu Ile
               180                 185                 190
   Ile Cys Gly Leu Glu Lys Gln Leu Lys Cys Glu Ser Gly Ser Ala Tyr
           195                 200                 205
   Ser Arg Ala Arg Thr Lys Asn Asn Tyr Gly Ser Thr Ile Glu Gly Leu
       210                 215                 220
   Leu Asp Leu Pro Asp Asp Asp Ala Pro Glu Glu Ala Gly Leu Ala Ala
   225                 230                 235                 240
   Pro Arg Leu Ser Phe Leu Pro Ala Gly His Thr Arg Arg Leu Ser Thr
                   245                 250                 255
   Ala Pro Pro Thr Asp Val Ser Leu Gly Asp Glu Leu His Leu Asp Gly
               260                 265                 270
   Glu Asp Val Ala Met Ala His Ala Asp Ala Leu Asp Asp Phe Asp Leu
           275                 280                 285
   Asp Met Leu Gly Asp Gly Asp Ser Pro Gly Pro Gly Phe Thr Pro His
       290                 295                 300
   Asp Ser Ala Pro Tyr Gly Ala Leu Asp Met Ala Asp Phe Glu Phe Glu
   305                 310                 315                 320
   Gln Met Phe Thr Asp Ala Leu Gly Ile Asp Glu Tyr Gly Gly
                   325                 330
<210> 35
   <211> 22
   <212> DNA
   <213> artificial
<220>
   <223> forward primer Tet-element
<400> 35
   agctgggagt tgagcagcct ac 22
<210> 36
   <211> 22
   <212> DNA
   <213> artificial
<220>
   <223> reverse primer Tet-element
<400> 36
   ggtgccgaga tgcactttag cc 22
<210> 37
   <211> 20
   <212> DNA
   <213> artificial
<220>
   <223> forward primer Luciferase
<400> 37
   gaagagatac gccctggttc 20
<210> 38
   <211> 20
   <212> DNA
   <213> artificial
<220>
   <223> reverse primer Luciferase
<400> 38
   cgggaggtag atgagatgtg 20

## Description

The present invention is concerned with means and methods for determining neurotoxin activity. Specifically, it relates to a polynucleotide encoding a fusion polypeptide comprising (i) tetracycline dependent transactivator (tet-repressor VP16) and (ii) synaptotagmin, separated by a linker comprising a neurotoxin cleavage site as well as to a fusion polypeptide encoded by the polynucleotide of the invention. Also contemplated is a vector comprising the polynucleotide of the invention and a host cell comprising the polynucleotide, vector or fusion polypeptide of the invention. Moreover, envisaged is a method for determining neurotoxin activity in a sample. In addition, the invention pertains to the use of the polynucleotide, vector, fusion polypeptide or host cell of the invention for determining neurotoxin activity in a sample. Finally, the invention relates to a kit for determining neurotoxin activity comprising the polynucleotide, vector, fusion polypeptide or host cell of the invention.

Clostridium botulinum and Clostridium tetani produce highly potent neurotoxins, i.e. botulinum toxins (BoNTs) and tetanus toxin (TeNT), respectively. These Clostridial neurotoxins specifically bind to neuronal cells and disrupt neurotransmitter release. Each toxin is synthesized as an inactive unprocessed approximately 150 kDa single-chain protein. The posttranslational processing involves formation of disulfide bridges, and limited proteolysis (nicking) by bacterial protease(s). Active dichain neurotoxin consists of two chains, an N-terminal light chain of approx. 50 kDa and a heavy chain of approx. 100 kDa linked by a disulfide bond. Neurotoxins structurally consist of three domains, i.e. the catalytic light chain, the heavy chain encompassing the translocation domain (N-terminal half) and the receptor binding domain (C-terminal half), see Krieglstein 1990, Eur. J. Biochem. 188, 39; Krieglstein 1991, Eur. J. Biochem. 202, 41; Krieglstein 1994, J. Protein Chem. 13, 49.

Clostridium botulinum secretes seven antigenically distinct serotypes designated A to G of the BoNTs. All serotypes together with the related TeNT secreted by Clostridium tetani, are zinc (Zn²⁺)-dependent endoproteases that block synaptic exocytosis by cleaving SNARE proteins and, in particular, SNAP-25. BoNTs cause, inter alia, the flaccid muscular paralysis seen in botulism and tetanus, see Fischer 2007, PNAS 104, 10447.

Despite its toxic effects, BoNTs have been used as therapeutic agents in a large number of diseases. BoNT serotype A (BoNT/A) was approved for human use in the United States in 1989 for the treatment of strabism, blepharospasm, and other disorders. It is commercially available as a protein preparation, for example, under the tradename BOTOX (Allergan Inc) under the tradename DYSPORT (Ipsen Ltd). For therapeutic application the complex is injected directly into the muscle to be treated. At physiological pH, the toxin is released from the protein complex and the desired pharmacological effect takes place. An improved BoNT/A preparation being free of complexing proteins is available under the tradename XEOMIN (Merz Pharmaceuticals GmbH).

BoNTs, in principle, weaken voluntary muscle strength and are, therefore, effective therapeutic agents for the therapy of diseases such as strabism, focal dystonia, including cervical dystonia, and benign essential blepharospasm. They have been further shown to relief hemifacial spasm, and focal spasticity, and moreover, to be effective in a wide range of other indications, such as gastrointestinal disorders, hyperhidrosis, and cosmetic wrinkle correction, see Jost 2007, Drugs 67, 669.

The determination of the biological activity is important as a safety measure, for quality control and for quantification purposes. WO 95/33850 describes methods for quantifying the biological activity of neurotoxins based on non-competitive assays. In these methods, a substrate comprising a cleavage site for the neurotoxin is coupled to a solid phase. The addition of the neurotoxin results in the cleavage of the substrate. The cleavage product is then detected by, e.g., a specific antibody conjugated to an enzyme. One drawback of such non-competitive assays is that the test results obtained may be inhomogeneous and therefore unreliable.

Further assays which have been developed for the determination of the biological activity of Botulinum neurotoxins pertain to, e.g., the determination of the time of survival of animals after intoxication, for example "time to death" (Lamanna C, Spero L, Schantz EJ: Dependence of time to death on molecular size of botulinum toxin. Infect. Immun. 1970, 1:423-4; Kondo H, Shimizu T, Kubonoya M, Izumi N, Takahashi M, Sakaguchi G: Titration of botulinum toxins for lethal toxicity by intravenous injection into mice. Jpn. J. Med. Sci. Biol. 1984, 37:131-5; Boroff DA, Fleck U: Statistical analysis of a rapid in vivo method for the titration of the toxin of Clostridium botulinum. J. Bacteriol. 1966, 92:1580-1), the inhibition of organ functions of the living organism, such as paralysis of perspiratory glands (Ellies M: Experimental and clinical investigations on the inhibition of secretion of the major salivary glands with botulinum toxin A. Laryngorhinootologie 2003, 82:713-4; Monnier G, Tatu L, Parratte B, Cosson A, Michel F, Metton G: Sialorrhea, hyperhidrosis and botulinum toxin. Ann. Readapt. Med. Phys. 2003, 46:338-45), the determination of paralysis of isolated organs, such as HDA (Göschel H, Wohlfarth K, Frevert J, Dengler R, Bigalke H: Botulinum A toxin therapy: neutralizing and nonneutralizing antibodies - therapeutic consequences. Exp. Neurol. 1997, 147:96-102; HUGHES R, WHALER BC. Influence of nerve-ending activity and of drugs on the rate of paralysis of rat diaphragm preparations by Cl. botulinum type A toxin. J. Physiol. 1962, 160:221-33), cell based assays, such as SNAP-25 cleavage Western Blot assays using primary neurons (Pellett S, Tepp WH, Toth SI, Johnson EA: Comparison of the primary rat spinal cord cell (RSC) assay and the mouse bioassay for botulinum neurotoxin type A potency determination. J. Pharmacol. Toxicol. Methods 2010, 61:304-10) and in vitro tests, which, however, determine only single aspects of the biological activity of neurotoxins, such as binding assays or SNAP-25 cleavage assay (Evans ER, Skipper PJ, Shone CC: An assay for botulinum toxin types A, B and F that requires both functional binding and catalytic activities within the neurotoxin. J. Appl. Microbiol. 2009, 107:1384-91; Habermann E: 125I-labeled neurotoxin from Clostridium botulinum A: preparation, binding to synaptosomes and ascent to the spinal cord. Naunyn Schmiedebergs Arch. Pharmacol. 1974, 281:47-56; Ekong TA, Feavers IM, Sesardic D: Recombinant SNAP-25 is an effective substrate for Clostridium botulinum type A toxin endopeptidase activity in vitro. Microbiology 1997, 143:3337-47).

The mouse LD50 assay is thus far the only reliable assay for quantifying the biological activity of neurotoxins and for assessing their therapeutic potential and/or their toxicity. Said assay is also accepted for quality control purposes during manufacture of neurotoxin. In the mouse LD50 bioassay, lethal and sub-lethal concentrations of a sample containing the neurotoxin polypeptide have to be injected into at least 120 animals. The number of killed animals over an observation period of 72 hours allows determining the neurotoxin polypeptide concentration in the sample. Apparent drawbacks of this assay are the high number of animals which will be sacrificed and the high level of stress and pain for said animals during the test.

In vitro assays which have been proposed so far are based on determining SNAP-25 cleavage in a cell free system or on neurotoxin exposure to primary neurons. However, these assays are less reliable and/or do not take into account all of the desired neurotoxin functions. Thus, at present, the LD50 bioassay described above is the only reliable assay which is described in the monograph for BoNT/A in the European pharmacopeia. However, there is a need for reliable assays for measuring neurotoxin activity which avoid the drawbacks of the assays described in the art.

Therefore, the technical problem underlying the present invention could be seen in the provision of means and methods for complying with the aforementioned needs. The technical problem is solved by the embodiments characterized in the claims and herein described below.

The present invention relates to a polynucleotide encoding a fusion polypeptide comprising (i) tetracycline dependent transactivator (tet-repressor VP16) and (ii) synaptotagmin, separated by a linker comprising a neurotoxin cleavage site.

The term "polynucleotide" as used herein refers to single- or double-stranded DNA molecules as well as to RNA molecules. Encompassed by the said term is genomic DNA, cDNA, hnRNA, mRNA as well as all naturally occurring or artificially modified derivatives of such molecular species. The polynucleotide may be in an aspect a linear or circular molecule. Moreover, in addition to the nucleic acid sequences encoding the fusion polypeptide of the present invention, a polynucleotide of the present invention may comprise additional sequences required for proper transcription and/or translation such as 5'- or 3'-UTR sequences. In light of the degeneracy of the genetic code, optimized codons may be used in the nucleic acid sequences encoding the fusion polypeptide of the present invention. Thereby, optimal expression in, e.g., a host cell of the present invention can be achieved. In another aspect, the said polynucleotide comprises a nucleic acid sequence as shown in SEQ ID NO: 9. Moreover, encompassed is in an aspect a polynucleotide comprising a nucleic acid sequence encoding an amino acid sequence as shown in SEQ ID NO: 10.

In another aspect, the said polynucleotide is a variant of the aforementioned polynucleotides comprising one or more nucleotide substitutions, deletions and/or additions which in still another aspect may result in a fusion polypeptide having one or more amino acid substitutions, deletions and/or additions. Moreover, a variant polynucleotide of the invention shall in another aspect comprise a nucleic acid sequence variant being at least 40%, at least 50%, at least 60%, at least 70%, at least 75%, at least 80%, at least 85%, at least 90%, at least 95%, at least 98% or at least 99% identical to the nucleic acid sequence as shown in SEQ ID NO: 9 or a nucleic acid sequence variant which encodes an amino acid sequence being at least 40%, at least 50%, at least 60%, at least 70%, at least 75%, at least 80%, at least 85%, at least 90%, at least 95%, at least 98% or at least 99% identical to the amino acid sequence as shown in SEQ ID NO: 10. The term "identical" as used herein refers to sequence identity characterized by determining the number of identical amino acids between two nucleic acid sequences or amino acid sequences wherein the sequences are aligned so that the highest order match is obtained. It can be calculated using published techniques or methods codified in computer programs such as, for example, BLASTP, BLASTN or FASTA (Altschul 1990, J. Mol. Biol. 215, 403). The percent sequence identity values are, in one aspect, calculated over the entire nucleotide or amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J. Mol. Biol. 48; 443; Smith 1981, Adv. Appl. Math. 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. In an aspect, each of the aforementioned variant polynucleotides encodes a fusion polypeptide retaining one or more and, in another aspect, all of the biological properties of the respective fusion polypeptide of the invention. Preferably, the variant polynucleotide encodes a fusion polypeptide comprising (i) tetracycline dependent transactivator (tet-repressor VP16) and (ii) synaptotagmin, separated by a linker comprising a neurotoxin cleavage site.

The term "transcription factor" as used herein means a protein required to initiate or regulate transcription in eukaryotes. A transcription factor is a protein that binds to specific DNA sequences, thereby controlling the flow of genetic information from DNA to mRNA, i.e. the transcription. The DNA sequence that a transcription factor binds to is called a transcription factor-binding site or response element. An "enhancer" as used herein is a short region of DNA that can increase transcription of genes, whereas a "silencer" is a DNA sequence capable of binding transcription regulation factors termed repressors. Transcription factors perform this function alone or with other proteins in a complex, by promoting as an activator or blocking as a repressor the recruitment of RNA polymerase to specific genes. The term "domain" as used herein denotes a protein domain which is a part of protein sequence and structure that can evolve, function, and exist independently of the rest of the protein chain. Each domain forms a compact three-dimensional structure and often can be independently stable and folded. Many proteins, including transcription factors, consist of several structural domains. The term "transcription factor domain" as used herein comprises a transcription factor of a protein domain of a transcription factor. Transcription factors are modular in structure and contain several domains, including, but not limited to, a DNA binding domain, a transactivator domain or silencer domain and an optional signal sensing domain, e.g. a ligand binding domain. The portion of the transcription factor that binds to DNA is called DNA binding domain. The DNA binding domain attaches to specific sequences of DNA such as enhancers or promoters. Transcription factors interact with their binding sites using a combination of electrostatic and van der Waals forces. Due to the nature of these chemical interactions, most transcription factors bind DNA in a sequence specific manner. However, not all bases in the transcription factor-binding site may actually interact with the transcription factor. In addition, some of these interactions may be weaker than others. Thus, transcription factors do not bind just one sequence but are capable of binding a subset of closely related sequences, each with a different strength of interaction. The transactivating domain, i.e. the transactivator or silencer domain, contains binding sites for other proteins such as transcription co-regulators. Transactivator or silencer domains are named after their amino acid composition. These amino acids are either essential for the activity or simply the most abundant in the transactivator or silencer domain. A transcription factor regulates transcription by controlling the rate of gene transcription, for example, by activating or inhibiting RNA polymerase binding to DNA. The transactivating domain may increase the rate of gene transcription or decrease the rate of gene transcription. In the former case, the transactivating domain is called a transactivator domain, in the latter case, the transactivating domain is called a silencer domain. The signal sensing domain senses external signals and, in response, transmit these signals to the rest of the transcription complex, resulting in up- or down-regulation of gene expression. A transcription factor as used herein may be, for example, a tetracycline dependent transactivator (tet-repressor - VP 16), steroid hormone receptors, NOTCH, NFκB, p53, NFAT, MLL, E2A, HSF1, NF-IL6, STAT, R-SMAD, GAL4, or SP1. It is envisaged that the transcription factor as used herein may in some aspects comprise a nuclear localization sequence. Nuclear localization sequences or signals may be either signal sequences or signal patches. The nuclear localization signals direct proteins to the nucleus of the cell.

The term "cytoplasmic retention domain" as used herein denotes a protein domain which mediates a cytoplasmic localization of the fusion polypeptide encoded by the polynucleotide of the invention, prior to cleavage of the fusion protein by the neurotoxin. The cytoplasmic retention domain as used herein can be, for instance, a transmembrane protein or a transmembrane spanning domain of a transmembrane protein. For example, the transmembrane protein may be plasmalemmal neurotransmitter transporters, ion channels, G-protein coupled receptors, or membrane receptors. The cytoplasmic retention domain can also be a membrane-anchored protein or a membrane anchor domain of a membrane-anchored protein. Such a membrane-anchored protein may be, for example, SNAP-25, Syntaxin, synaptoprevin, synaptotagmin, vesicle associated membrane proteins (VAMPs), synaptic vesicle glycoproteins (SV2), high affinity choline transporters, Neurexins, voltage-gated calcium channels, acetylcholinesterase, or NOTCH. The cytoplasmic retention domain as used herein may also be a globular protein or a cytoskeleton anchor protein which is too big to be able to pass the pores of the nucleus. After expression of the fusion polypeptide of the invention, the transcription factor domain tetracycline dependent transactivator (tet-repressor VP16) is immobilized, for example, to the plasma or vesicle membrane via the cytoplasmic retention domain synaptotagmin and is, therefore, not able to translocate to the nucleus. By this way, the expression of the reporter in the nucleus by the transcription factor is avoided. Only the cleavage of the fusion protein of the invention within the linker by the neurotoxin light chain of the Botulinum neurotoxin releases the transcription factor which subsequently translocates to the cell nucleus, thereby initiating the expression of the reporter gene.

The term "linker" as used herein denotes a polylinker which is a short segment of amino acid sequence comprising a neurotoxin cleavage site. Suitable linker sequences encoded by a corresponding DNA sequence are described, for example, in Schiavo G, Matteoli M, Montecucco C: Neurotoxins affecting neuroexocytosis. Physiol. Rev. 2000, 80:717-66.

The term "neurotoxin cleavage site" as used herein refers to a cleavage site which is recognized and cleaved by the endogenous protease of a neurotoxin polypeptide. Cleavage site which are recognized by the neurotoxin proteases are well known in the art; see, e.g., EP 1 926 744 B1. In principle, a neurotoxin cleavage site can be a cleavage site which naturally occurs in a substrate or which is an artificially designed cleavage site recognized and cleaved by the neurotoxin polypeptides protease. It will be understood that the properties of the neurotoxin cleavage site govern the kind of neurotoxin which can activate the fusion polypeptide of the present invention. Neurotoxin polypeptides referred to herein, in an aspect, encompass BoNT/A, BoNT/B, BoNT/C1, BoNT/D, BoNT/E, BoNT/G, BoNT/F or TeNT all of which are well known in the art. For example, if a neurotoxin cleavage site is used which is specifically recognized and cleaved by BoNT/A, only the BoNT/A protease will be capable of activating the fusion polypeptide of the present invention, whereas if a neurotoxin cleavage site is used which is specifically recognized and cleaved by BoNT/E, only the BoNT/E protease will be capable of activating the fusion polypeptide of the present invention. In an aspect of the fusion polypeptide of the invention, the neurotoxin cleavage site is cleaved by mature BoNTs. In yet another aspect, it is cleaved by muteins of BoNTs, in an aspect, by muteins comprising or consisting of the BoNT light chain exhibiting the BoNT protease activity.

A neurotoxin cleavage site recognized and cleaved by the BoNT/A protease, in an aspect of the invention, is derived from a protein that is sensitive to cleavage by BoNT/A. In an aspect, such a protein is human SNAP25A or SNAP25B or a homolog, paralog or ortholog thereof from rat, mouse, bovine, Danio, Carassius, Xenopus, Torpedo, Strongylocentrotus, Loligo, Lymnaea or Aplysia. Suitable cleavage sites derived from said proteins are disclosed in EP 1 926 744 B1.

A neurotoxin cleavage site recognized and cleaved by the BoNT/B protease, in an aspect of the invention, is derived from a protein that is sensitive to cleavage by BoNT/B. In an aspect, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the BoNT/C1 protease, in an aspect of the invention, is derived from a protein that is sensitive to cleavage by BoNT/C1. In an aspect, such a protein is human and mouse Syntaxin 1A, Syntaxin 1B1, Syntaxin 2-1, Syntaxin 2-2, Syntaxin 2-3, Syntaxin 3A or Syntaxin 1B2, bovine or rat Syntaxin 1A, Syntaxin 1B1 or Syntaxin 1B2, rat Syntaxin 2 or Rat syntaxin 3, mouse Syntaxin 1A, Syntaxin 1B1, Syntaxin 1B2, Syntaxin 2, Syntaxin 3A, Syntaxin 3B or Syntaxin 3C, chicken Syntaxin 1A or Syntaxin 2; Xenopus Syntaxin 1A or Syntaxin 1B, Danio Syntaxin 1A, Syntaxin 1B or Syntaxin 3, Torpedo Syntaxin 1A or Syntaxin 1B, Strongylocentrotus Syntaxin 1A or Syntaxin 1B, Drosophila Syntaxin 1A or Syntaxin 1B, Hirudo Syntaxin 1A or Syntaxin 1B, Loligo Syntaxin 1A or Syntaxin 1B, Lymnaea Syntaxin 1A or Syntaxin 1B or any ortholog, paralog or homolog thereof. Suitable cleavage sites derived from said proteins are disclosed in EP 1 926 744 B1.

A neurotoxin cleavage site recognized and cleaved by the BoNT/D protease, in an aspect of the invention, is derived from a protein that is sensitive to cleavage by BoNT/D. In an aspect, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the BoNT/E protease, in an aspect of the invention, is derived from a protein that is sensitive to cleavage by BoNT/E. In an aspect, such a protein is, such a protein is human SNAP-25A or B or a homolog, paralog or ortholog thereof from rat, mouse, bovine, Danio, Carassius, Xenopus, Torpedo, Strongylocentrotus, Loligo, Lymnaea or Aplysia. Suitable cleavage sites derived from said proteins are disclosed in EP 1 926 744 B1.

A neurotoxin cleavage site recognized and cleaved by the BoNT/F protease, in an aspect of the invention, is derived from a protein that is sensitive to cleavage by BoNT/F. In an aspect, such a protein is, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the BoNT/G protease, in an aspect of the invention, is derived from a protein that is sensitive to cleavage by BoNT/G. In an aspect, such a protein is, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

A neurotoxin cleavage site recognized and cleaved by the TeNT protease, in an aspect of the invention, is derived from a protein that is sensitive to cleavage by TeNT. In an aspect, such a protein is human or mouse VAMP-1, VAMP-2 and VAMP-3/cellubrevin, bovine VAMP-2, rat VAMP-2 or VAMP-3, chicken VAMP-1, VAMP-2 or VAMP-3, Torpedo VAMP-1, Strongylocentrotus VAMP, Drosophila sybA, synB, synC, synD, or syn, Hirudo VAMP, Xenopus VAMP-2 or VAMP-3, Danio VAMP-1 or VAMP-2, Loligo VAMP, Lymnaea VAMP, Aplysia VAMP or Caenorhabditis SNB1-like or any ortholog, paralog or homolog thereof. Suitable cleavage sites derived from said proteins are disclosed in EP 1 926 744 B1.

The term "separated by a linker" in context of the polynucleotide or the fusion polypeptide of the invention means that a linker comprising a neurotoxin cleavage site is located between the transcription factor domain tetracycline dependent transactivator (tet-repressor VP16) and the cytoplasmic retention domain synaptotagmin, as set forth in more detail below.

The term "biologically active" used herein in context with a neurotoxin refers to a mature neurotoxin polypeptide exhibiting essentially the biological properties specified herein, i.e. being capable of (a) receptor binding, (b) internalization, (c) translocation across the endosomal membrane into the cytosol, and/or (d) endoproteolytic cleavage of proteins involved in synaptic vesicle membrane fusion. Preferably, the biologically activity is the proteolytic activity of neurotoxins.

It is to be understood that the definitions and explanations of the terms made above apply *mutatis mutandis* for all aspects described in the specification in the following except as otherwise indicated.

The means and methods of the invention are based on the proteolytic release of the transcription factor domain tetracycline dependent transactivator (tet-repressor VP16) from a fusion polypeptide by the Botulinum neurotoxin in cells, preferably in neuronal cells as further defined elsewhere herein. The transcription factor domain tetracycline dependent transactivator (tet-repressor VP16) can in one aspect of the polynucleotide or fusion polypeptide of the invention be fused to the cytoplasmic retention domain synaptotagmin via a linker comprising a neurotoxin cleavage site. After expression of said fusion polypeptide, the transcription factor domain tetracycline dependent transactivator (tet-repressor VP16) is first immobilized to the cytoplasm. This can be achieved by using the membrane-anchored protein synaptotagmin as a suitable cytoplasmic retention signal in the polynucleotide or fusion polypeptide of the invention. The cytoplasmic retention signal synaptotagmin prevents the transcription factor domain tetracycline dependent transactivator (tet-repressor VP16) from translocating to the cell nucleus. Only the proteolytic activity of a Botulinum neurotoxin releases the transcription factor domain tetracycline dependent transactivator (tet-repressor VP16) from the fusion polypeptide upon cleavage of the fusion protein within the linker comprising the neurotoxin cleavage site. This results in the translocation of the transcription factor domain tetracycline dependent transactivator (tet-repressor VP16) to the nucleus where it is able to initiate the expression of a reporter protein, such as luciferase, alkaline phosphatase, or horseradish peroxidase. The presence and/or the amount of the expressed reporter protein can be analyzed by assays well described in the art, for instance by an enzymatic test (Miraglia LJ, King FJ, Damoiseaux R: Seeing the light: luminescent reporter gene assays. Comb. Chem. High Throughput Screen 2011, 14:648-57; Fan F, Wood KV: Bioluminescent assays for high-throughput screening. Assay Drug Dev. Technol. 2007, 5:127-36). The amount of the expressed reporter is directly proportional to the proteolytic activity of the neurotoxin light chain in the cytoplasm and, thus, also proportional to the biological activity of Botulinum neurotoxin. By comparison of the results of such an assay with a reference Botulinum neurotoxin with a defined biological activity, the biological activity of an unknown Botulinum neurotoxin in a sample can be determined.

Advantageously, the polynucleotide and the fusion polypeptide of the invention allow for the determination of the protease activity of a given neurotoxin polypeptide in a cell culture system. Thus, expensive and ethically unnecessary animal testing can be avoided or reduced thanks to the present invention. Moreover, the polynucleotide and the fusion polypeptide of the invention can be applied in automated high throughput screening assays. The polynucleotide and the fusion polypeptide of the invention also allow for establishing assays in cell lines, such as, e.g., neuroblastoma cell lines. Accordingly, the use of primary neurons as required in other cell based neurotoxin assays can be avoided. This is another advantage of the methods and means of the invention since the preparation of primary neurons is cumbersome and inefficient. In addition, by using the means and methods described therein, the sensitivity and flow rate of cell based assays for the determination of the biological activity of Botulinum neurotoxins could be significantly increased. Therefore, the present invention provides improved assays for testing the biological activity of Botulinum neurotoxins.

In an aspect of the polynucleotide of the present disclosure, the cytoplasmic retention domain is a transmembrane protein or transmembrane spanning domain of a transmembrane protein. By using suitable cytoplasmic retention domains, the fusion protein of the present disclosure is transported to or retained in the compartment of the cell, where the neurotoxin light chain is biologically active, i.e. the cytoplasm. Put in other words: After expression of said fusion polypeptide, the transcription factor domain is, prior to the activation by neurotoxin cleavage, immobilized to the cytoplasm. Such a cytoplasmic retention domain is in this aspect of the polynucleotide encoding the fusion polypeptide of the present disclosure a transmembrane protein or transmembrane spanning domain of a transmembrane protein.

In another aspect of the polynucleotide of the present disclosure, the transmembrane protein is selected from the group consisting of: plasmalemmal neurotransmitter transporters, ion channels, G-protein coupled receptors and membrane receptors.

In a further aspect of the polynucleotide of the present disclosure, the cytoplasmic retention domain is a membrane-anchored protein or a membrane anchor domain of a membrane-anchored protein. In one aspect of the polynucleotide of the present disclosure, the membrane-anchored protein is selected from the group consisting of: SNAP-25, Syntaxin, synaptoprevin, synaptotagmin, vesicle associated membrane proteins (VAMPs), synaptic vesicle glycoproteins (SV2), high affinity choline transporters, Neurexins, voltage-gated calcium channels, acetylcholinesterase and NOTCH. In one aspect of the polynucleotide of the invention, the membrane anchored protein is synaptotagmin. In the following, the corresponding accession number of the respective protein is indicated: human SNAP-25 P60880, human Syntaxin-1A Q16623, Syntaxin-1B P61266, Syntaxin-2 P32856, Syntaxin-3 Q13277, Syntaxin-4 Q12846, Syntaxin-5 Q13190, Syntaxin-6 O43752, Syntaxin-7 O15400, Syntaxin-8 Q9UNK0, Syntaxin-10 O60499, Syntaxin-11 O75558, Syntaxin-12 Q86Y82, Syntaxin-16 O14662, Syntaxin-17 P56962, Syntaxin-18 Q9P2W9, Syntaxin-19 Q8N4C7; human Synaptobrevin-1 P23763, Synaptobrevin-2 P63027, Synaptobrevin-3 Q15836; human synaptotagmin: Synaptotagmin-1 P21579, Synaptotagmin-2 Q8N9I0, Synaptotagmin-3 Q9BQG1, Synaptotagmin-4 Q9H2B2, Synaptotagmin-5 O00445, Synaptotagmin-6 Q5T7P8, Synaptotagmin-8 Q8NBV8, Synaptotagmin-9 Q86SS6, Synaptotagmin-10 Q6XYQ8, Synaptotagmin-11 Q9BT88, Synaptotagmin-12 Q8IV01, Synaptotagmin-13 Q7L8C5, Synaptotagmin-14 Q8NB59, Synaptotagmin-15 Q9BQS2, Synaptotagmin-16 Q17RD7, Synaptotagmin-17 Q9BSW7, human vesicle associated membrane proteins (VAMPs): Vesicle-associated membrane protein 1 P23763, Vesicle-associated membrane protein 2 P63027, Vesicle-associated membrane protein 3 Q15836, Vesicle-associated membrane protein 4 O75379, Vesicle-associated membrane protein 5 095183, Vesicle-associated membrane protein 7 P51809, Vesicle-associated membrane protein 8 Q9BV40; of synaptic vesicle glycoproteins (SV2): Synaptic vesicle glycoprotein 2A Q7L0J3, Synaptic vesicle glycoprotein 2B Q7L1I2, Synaptic vesicle glycoprotein 2C Q496J9; human high affinity choline transporter Q9GZV3; human Neurexins: Neurexin-1-alpha Q9ULB1, Neurexin-1-beta P58400, Neurexin-2-alpha Q9P2S2, Neurexin-2-beta P58401, of Neurexin-3-alpha Q9Y4C0, Neurexin-3-beta Q9HDB5; human voltage-gated calcium channels: Voltage-dependent calcium channel subunit alpha-2/delta-1 P54289, Voltage-dependent calcium channel subunit Q9NY47, Voltage-dependent calcium channel subunit Q8IZS8, Voltage-dependent calcium channel subunit Q7Z3S7, Voltage-dependent P/Q-type calcium channel subunit O00555, Voltage-dependent N-type calcium channel subunit Q00975, Voltage-dependent L-type calcium channel subunit Q13936, Voltage-dependent L-type calcium channel subunit Q01668, Voltage-dependent R-type calcium channel subunit Q15878, Voltage-dependent L-type calcium channel subunit O60840, Voltage-dependent T-type calcium channel subunit O43497, Voltage-dependent T-type calcium channel subunit O95180, Voltage-dependent T-type calcium channel subunit Q9P0X4, Voltage-dependent L-type calcium channel subunit Q13698, Voltage-dependent L-type calcium channel subunit Q02641, Voltage-dependent L-type calcium channel subunit Q08289, Voltage-dependent L-type calcium channel subunit P54284, Voltage-dependent L-type calcium channel subunit O00305, Voltage-dependent calcium channel gamma-1 subunit Q06432, Voltage-dependent calcium channel gamma-2 subunit Q9Y698, Voltage-dependent calcium channel gamma-3 subunit O60359, Voltage-dependent calcium channel gamma-4 subunit Q9UBN1, Voltage-dependent calcium channel gamma-5 subunit Q9UF02, Voltage-dependent calcium channel gamma-6 subunit Q9BXT2, Voltage-dependent calcium channel gamma-7 subunit P62955, Voltage-dependent calcium channel gamma-8 subunit Q8WXS5, Voltage-dependent calcium channel gamma-like subunit Q8WXS4, Voltage gated calcium channel alpha 1F subunit F5CIQ9, Sodium leak channel non-selective protein Q8IZF0, Voltage-gated calcium channel beta 2 subunit Q5QJ99, Voltage-gated calcium channel beta 2 subunit Q5QJA0, Voltage-gated L-type calcium channel Cav1.2 Q5V9X8, Voltage-gated L-type calcium channel Cav1.2 Q5V9X9, Voltage-gated calcium channel beta 2 subunit Q6TME0, Voltage-gated calcium channel beta 2 subunit Q6TME1, Voltage-gated calcium channel beta 2 subunit Q6TME2, Voltage-gated calcium channel beta 2 subunit Q6TME3, Voltage-gated calcium channel beta 1 subunit Q6TME4, Voltage-gated calcium channel alpha 1 subunit Q71UT1, Voltage-gated calcium channel alpha 1E subunit Q9UN68; human acetylcholinesterase P22303; and human NOTCH: Neurogenic locus notch homolog protein 1 P46531, Neurogenic locus notch homolog protein 2 Q04721, Neurogenic locus notch homolog protein 3 Q9UM47, Neurogenic locus notch homolog protein 4 Q99466.

In this aspect of the polynucleotide of the present disclosure, it is advantageous to select proteins which are localized at or in the pre-synaptic membrane. It is to be understood that basically all proteins can be used which by membrane-anchor or interaction with membrane proteins are localized at the membrane, e.g. plasma membrane or vesicle membrane. Such proteins are preferably present at the compartment where the neurotoxin light chain exerts its biological activity, i.e. in the cytoplasm of a cell, e.g. a neuronal cell. The biological activity is composed of the binding to the neurotoxin receptors, the translocation of the neurotoxin light chain into the cytosol of the target cell and the proteolytic activity of the neurotoxin mediated by the light chain of the neurotoxin.

In a still further aspect of the polynucleotide of the present disclosure, the cytoplasmic retention domain is selected from a globular protein or a cytoskeleton anchor protein. In this aspect, the transcription factor is fused to a large globular protein or cytoskeleton anchor protein which is big enough to prevent the fusion polypeptide from passing the pores of the nucleus. By this way, the transport of the fusion protein (comprising the transcription activator domain) through the pores of the nucleus can be avoided. It is envisaged that a globular protein or a cytoskeleton anchor protein can be used alone or fused to other proteins or protein domains, such as exportin binding domains or IκB binding domains. The invention has been exemplified for the leucine-rich nuclear export signal (NES): (LxxxLxxLxL; SEQ ID NO: 32), with "x" representing any naturally occurring amino acid.

In another aspect of the polynucleotide of the present disclosure, the transcription factor domain comprises a transcription factor comprising a DNA binding domain and a transactivator or silencer domain. In a further aspect of the polynucleotide of the invention, the transcription factor tetracycline dependent transactivator (tet-repressor VP16) comprises, in addition to a DNA binding domain and a transactivator or silencer domain, a nuclear localization sequence. For example, in some aspects, the silencer or repressor (e.g. REST) can be positioned opposite to the transactivator domain so that the cleavage site intervenes silencer/repressor and transactivator domain. This results in an additional decrease in basal expression of the reporter. The nuclear localization signals direct proteins to the nucleus of the cell. It is to be understood that the transcription factor as used herein is able to modulate the expression of a reporter gene, i.e. the transcription factor is capable of increasing or decreasing the expression of a reporter gene.

In a still further aspect of the polynucleotide of the present disclosure, the transcription factor is selected from the group consisting of: tetracycline dependent transactivator (tet-repressor - VP 16), steroid hormone receptors, NOTCH, NFκB, p53, NFAT, MLL, E2A, HSF1, NF-IL6, STAT, R-SMAD, GAL4, and SP1. In one aspect of the polynucleotide of the invention the transcription factor is tetracycline dependent transactivator (tet-repressor VP16). The amino acid sequence for the tetracycline dependent transactivator is shown, e.g., in SEQ ID NO: 33 or 34: The amino acid sequence of the tet-repressor is shown under accession number P04483. The amino acid sequence of VP16 is shown under accession number P06492. The corresponding accession numbers for steroid hormone or other nuclear receptors are: Androgen receptor P10275, Estrogen receptor P03372, Glucocorticoid receptor P04150, Mineralocorticoid receptor P08235, Progesterone receptor P06401, Prolactin receptor P16471, Retinoic acid receptor alpha P10276, Retinoic acid receptor gamma P13631, Retinoic acid receptor RXR-alpha P19793, Steroid hormone receptor ERR1 P11474, Steroid hormone receptor ERR2 095718, Thyroid hormone receptor alpha P10827, Thyroid hormone receptor beta P10828. The corresponding accession numbers for NOTCH proteins are: Neurogenic locus notch homolog protein 1 P46531, Neurogenic locus notch homolog protein 2 Q04721, Neurogenic locus notch homolog protein 3 Q9UM47, Neurogenic locus notch homolog protein 4 Q99466. The corresponding accession number for NFκB is Q04206; for p53 P04637; for NFAT: Nuclear factor of activated T-cells 1 095644, Nuclear factor of activated T-cells 2 Q13469, Nuclear factor of activated T-cells 3 Q12968, Nuclear factor of activated T-cells 4 Q14934, Nuclear factor of activated T-cells 5 094916; for E2A (Transcription factor E2-alpha) P15923; for HSF1 Q00613; for NF-IL6 P17676; for STAT: STAT1_HUMAN P42224, STAT2_HUMAN P52630, STAT3_HUMAN P40763, STAT4_HUMAN Q14765, STA5A HUMAN P42229, STA5B HUMAN P51692, STAT6 HUMAN P42226; for R-SMAD: Mothers against decapentaplegic homolog 1 Q15797, Mothers against decapentaplegic homolog 2 Q15796, Mothers against decapentaplegic homolog 3 P84022, Mothers against decapentaplegic homolog 4 Q13485, Mothers against decapentaplegic homolog 5 Q99717, Mothers against decapentaplegic homolog 6 O43541, Mothers against decapentaplegic homolog 7 O15105, Mothers against decapentaplegic homolog 9 O15198; for GAL4, and for SP1 P08047.

The invention also pertains to a vector comprising the polynucleotide of the invention. In an aspect, the said vector is an expression vector.

The term "vector", preferably, encompasses phage, plasmid, viral or retroviral vectors as well as artificial chromosomes, such as bacterial or yeast artificial chromosomes. Moreover, the term also relates to targeting constructs which allow for random or site-directed integration of the targeting construct into genomic DNA. Such target constructs, preferably, comprise DNA of sufficient length for either homologous or heterologous recombination as described in detail below. The vector encompassing the polynucleotides of the present invention, in an aspect, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells. Moreover, in an aspect of the invention, the polynucleotide is operatively linked to expression control sequences allowing expression in prokaryotic or eukaryotic host cells or isolated fractions thereof in the said vector. Expression of the polynucleotide comprises transcription of the polynucleotide into a translatable mRNA. Regulatory elements ensuring expression in host cells are well known in the art. In an aspect, they comprise regulatory sequences ensuring initiation of transcription and/or poly-A signals ensuring termination of transcription and stabilization of the transcript. Additional regulatory elements may include transcriptional as well as translational enhancers. Possible regulatory elements permitting expression in prokaryotic host cells comprise, e.g., the lac-, trp- or tac- promoter in E. coli, and examples for regulatory elements permitting expression in eukaryotic host cells are the AOX1- or the GAL1- promoter in yeast or the CMV-, SV40-, RSV-promoter (Rous sarcoma virus), CMV-enhancer, SV40-enhancer or a globin intron in mammalian and other animal cells. Other expression systems envisaged by the invention shall permit expression in insect cells, such as polyhedrin promoter based systems.

Moreover, inducible expression control sequences may be used in an expression vector encompassed by the present invention. Inducible expression systems and suitable expression control sequences are well known in the art. For example, the tetracycline-responsive regulatory system for transcriptional transactivation is described in Zhu Z, Zheng T, Lee CG, Homer RJ, Elias JA: Tetracycline-controlled transcriptional regulation systems: advances and application in transgenic animal modeling. Semin. Cell Dev. Biol. 2002, 13:121-8; or Shockett P, Schatz D: Inducible gene expression using an autoregulatory, tetracycline-controlled system. Curr. Protoc. Mol. Biol. 2002, Chapter 16: Unit 16.14. Such inducible vectors may comprise tet or lac operator sequences or sequences inducible by heat shock or other environmental factors are described in the art. For example, two commonly used inducible expression systems are Tet-Off and Tet-On; see Bujard and Gossen, Proc. Natl. Acad. Sci. U.S.A. 89 (12): 5547-51. They consist of a fusion of the Tet repressor and a VP16 activation domain to create a transcriptional activator protein (transactivator) rather than a repressor. Gene expression is activated as a result of binding of the Tet-Off or Tet-On protein to tetracycline response elements (TREs) located within an inducible promoter. The difference relates to their respective response to doxycycline (Dox), a more stable tetracycline analogue: Tet-Off activates expression in the absence of Dox, whereas Tet-On activates in the presence of Dox. Suitable vectors are commercially available. For example, the Tet-On 3G vector set by Clontech can be used to create tightly regulated and highly responsive tetracycline (Tet)-inducible mammalian expression systems that are turned on by the addition of doxycycline to the culture medium. The pCMV-Tet3G vector expresses Tet-On 3G, a tetracycline-controlled transactivator that exhibits high activity in the presence of the inducer doxycycline, and exceptionally low activity in its absence. Tet-On 3G results from the fusion of amino acids 1-207 of a mutant Tet repressor (TetR) to 39 amino acids that form three minimal "F"-type transcriptional activation domains from the herpes simplex virus VP16 protein. Constitutive expression of Tet-On 3G is driven by the human cytomegalovirus immediate early promoter (P_{CMV IE}). Further, an EF1 alpha version is available for cell lines in which the CMV promoter is silenced. For further detailed information see Clontech catalogue number 631163 and references cited therein. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3, pcDNA3.1 (Invitrogen) or pSPORT1 (Invitrogen) or baculovirus-derived vectors. Preferably, said vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Furthermore, the invention relates to a fusion polypeptide encoded by the polynucleotide of the invention. The term "fusion polypeptide" as used herein denotes a fusion polypeptide comprising (i) tetracycline dependent transactivator (tet-repressor VP16) and (ii) synaptotagmin. The transcription factor domain tetracycline dependent transactivator (tet-repressor VP16) and the cytoplasmic retention domain synaptotagmin are separated by a linker comprising a neurotoxin cleavage site. Proteolytic cleavage in the linker occurs at the neurotoxin cleavage site in a manner such that the linker is cleaved (at least) once. The linker may comprise one or more neurotoxin cleavage site(s). The term "separated by a linker" in context of the polynucleotide encoding the fusion polypeptide or the fusion polypeptide of the invention means that a linker comprising a neurotoxin cleavage site is located between the mentioned transcription factor domain and cytoplasmic retention domain. Accordingly, the domain arrangement in the fusion polypeptide can be from the N-terminus transcription factor domain-linker-cytoplasmic retention domain to the C-terminus or from the N-terminus cytoplasmic retention domain-linker-transcription factor domain to the C-terminus. The term "fusion polypeptide" as used herein encompasses isolated or essentially purified polypeptides being essentially free of other host cell polypeptides. The term, in another aspect, includes polypeptide preparations comprising the fusion polypeptide of the present invention and other proteins in addition. Moreover, the term includes, in an aspect, chemically modified polypeptides. Such modifications may be artificial modifications or naturally occurring modifications. The fusion polypeptide of the present invention shall have the biological properties referred to above. Moreover, encompassed is in an aspect a fusion polypeptide comprising an amino acid sequence as shown in SEQ ID NO: 10. The polypeptide of the invention, in an aspect, can be manufactured by chemical synthesis or recombinant molecular biology techniques well known for the skilled artisan. In an aspect, such a method of manufacturing the fusion polypeptide of the invention comprises (a) culturing the host cell of the present invention described elsewhere herein in more detail and (b) obtaining from the said host cell the fusion polypeptide of the present invention. In an aspect of this method, the polypeptide can be obtained by conventional purification techniques from a host cell lysate including affinity chromatography, ion exchange chromatography, size exclusion chromatography and/or preparative gel electrophoresis. In an aspect of the fusion polypeptide of the invention, the fusion polypeptide comprises a purification tag.

The term "purification tag" refers to an amino acid sequence which allows for purification of the polypeptide comprising it. This can be achieved in an aspect due to the presence of individual amino acids, peptide sequences or three dimensional peptide structures which are capable of physically or chemically interacting with the purification matrix. Suitable tags include affinity tags and epitope tags. In this context, it is well known that certain amino acids are capable of forming complexes with defined matrices to be used for purification, such as histidines which are capable of physico-chemically interacting with metal ions such as nickel or cobalt embedded in a suitable matrix such as sepharose or agarose. Further peptide sequences or three dimensional structures can be found in chitin binding proteins (CBP), maltose binding proteins (MBP) or glutathione-S-transferases (GST). In a further aspect, protein-protein interaction based purification matrix/purification tag systems can be used such as streptavidin/biotin systems which are also well known in the art. In yet another aspect, the tag may confer a physic-chemical property to the polypeptide containing it which allows for purification, such as fluorescence. Suitable tags in this context include the well known fluorescent proteins, such as green fluorescent protein (GFP), blue fluorescent protein (BFP) or others. In addition to these tags which have an inherent affinity for some matrices, epitope tags can be applied in another aspect. Such epitope tags consists of short amino acid stretches which constitute a peptide epitope that can be specifically recognized by an antibody. Epitope tags are also well known in the art and include FLAG tags, MYC-tags, or HA-tags. Thus, in an aspect, the purification tag is selected from the group consisting of: His-tag, Myc-tag, FLAG-tag, strep-tag, MBP-tag, NusA tag, GST-tag, streptavidin and avidin.

The invention further pertains to a host cell comprising the polynucleotide, the vector or the fusion polypeptide of the invention.

The term "host cell" as used herein encompasses prokaryotic and eukaryotic host cells, preferably isolated prokaryotic and eukaryotic host cells. Preferably, the host cell is a eukaryotic host cell. A eukaryotic host cell as used herein is a cell of an animal cell line suitable for production of the fusion polypeptide of the invention. The polynucleotide or vector of the invention can be stably integrated into the genome of the host cell or transiently expressed by the host cell. A host cell as referred to herein, thus, encompasses in an aspect, yeast cells, mammalian cells, including human cells, plant cells or insect cells, either as primary cells or as cell lines. Preferably, the host cell is a neuronal cell or cell line or a neuroblastoma cell or cell line.

In one aspect of the host cell of the invention, the host cell is selected from the group consisting of: primary neuronal cells, neuroblastoma cell lines, cell line N1E-115, cell line Neuro2a, cell line SH-SY5Y, cell line PC12, cell line SiMa, cell line MHH-NB-11, cell line SK-N-BE(2) and induced pluripotent stem cells (iPS), preferably human induced pluripotent stem cells (hiPS). Neuroblastoma cell lines can be obtained from ATCC or DSMZ under the following ATCC or DSMZ numbers: cell line N1E-115 CRL-2263, cell line Neuro2a CCL-131, cell line SH-SY5Y CRL-2266, cell line PC12 CRL-1721, cell line SiMa ACC 164 (DSMZ), cell line MHH-NB-11 ACC 157 (DSMZ) and cell line SK-N-BE(2) CRL-2271. Specific clones of the cell line SiMa are furthermore disclosed in WO 2010/105234. Methods of generating iPS cells are described, for example, in Yu et al., Science. 2009 May 8; 324(5928): 797-801. Epub 2009, WO 2011/056971 and WO 2011/025852. In some embodiments, iPS are differentiated into neurons using suitable methods, e.g., those described in WO 2012/135621 and U.S. Patent Applications US 2010/0279403 and US 2010/0216181.

In a still further aspect of the host cell of the invention, the host cell comprises a reporter gene the expression of which is modulated by the tetracycline dependent transactivator (tet-repressor VP16) upon cleavage of the fusion polypeptide. In this aspect, the host cell comprises a reporter gene. The reporter gene can be stably integrated into the genome of the host cell or transiently expressed by the host cell. The expression of the reporter gene is modulated by the mentioned transcription factor domain only after cleavage by a neurotoxin of the fusion polypeptide of the invention within the linker. Reporter gene assays are well described in the art (Miraglia LJ, King FJ, Damoiseaux R: Seeing the light: luminescent reporter gene assays. Comb. Chem. High Throughput Screen 2011, 14:648-57; Fan F, Wood KV: Bioluminescent assays for high-throughput screening. Assay Drug Dev. Technol. 2007, 5:127-36). As a reporter gene, for example luciferase, alkaline phosphatase, horseradish peroxidase or fluorescent proteins (GFP, YFP, RFP, CFP etc.) can be used.

The invention pertains additionally to a method for determining neurotoxin activity in a sample comprising the steps of:
(a) contacting a host cell comprising the fusion polypeptide of the invention and a reporter gene the expression of which is modulated by the tetracycline dependent transactivator (tet-repressor VP16) upon cleavage of the said fusion polypeptide with a sample suspected to comprise neurotoxin activity; and
(b) measuring the reporter gene activity, whereby neurotoxin activity in the sample is determined.

The generation of a reporter gene assay as described herein facilitates and improves the determination of the biological activity of neurotoxin polypeptides such as BoNT/A. For such assays, the polynucleotides, fusion polypeptides, vectors and host cells of the invention can be utilized. Sequentially, an activator construct and a reporter gene construct are stably transfected into host cells as described herein. After intoxication and cleavage of the neurotoxin polypeptide substrate such as SNAP-25, the transactivator (tTA) comprised in the activator construct is released from the Tet system. The binding of the transactivator to regulatory elements in the reporter gene construct results in the activation of the expression of the reporter gene such as luciferase. The activity of the encoded luciferase enzyme can be detected by commercially available kits, within a few hours. The luminescence mediated by the luciferase in a redox reaction is equivalent to the amount of neurotoxin polypeptide in the sample. A standard curve with known neurotoxin polypeptide concentrations is used as a reference in parallel. As a further reference, a protein measurement can be carried out in order to match the signal by luciferase to the cell number in the sample which may vary from case to case. This method is a routine method known in the art and can be carried out in parallel to the activity assays of the invention described above. As host cells the cells described herein can be used such as, for example, the SIMA cell line (human neuroblastoma). In the present invention, various clones of the SIMA cell line have been generated from a sub-cloned SIMA cell line (SIMA no. 3B4) which comprise the activator construct mST01 (synaptotagmin-SNAP-25-tTA) (SEQ ID NO: 9) and the reporter gene construct pTRE-Luc (luciferase reporter) (Clontech), as shown in the following Example. Reporter genes that can be used for the reporter gene assay as described herein are well known in the art and include, for example, the sequences shown in accession numbers P08659, Q27758, H1AD96, A3KC01, A3KC00, A3KBZ9, A3KBZ8, A3KBZ7, A3KBZ6, A3KBZ5, D5MS63, Q2TNU5, Q5UFR2, Q2VL10, Q6SVE0, B5A991, Q27688, A1Z0H9, Q207B1, Q5USC8, G9M6I8, Q6SVE2, Q9GPF9, or Q6SVE1.
The aforementioned clones which comprise the activator construct mST01 (synaptotagmin-SNAP-25-tTA) (SEQ ID NO: 9) and the reporter gene construct pTRE-Luc (luciferase reporter) (Clontech) carry stably the activator and reporter construct in the genome. Furthermore, parental SIMA cells, i.e. not sub-cloned, human multipotent cells from embryonic tissues or the commercially available P19 cell line (murine embryonic carcinoma) can be used as well for transfection with the mentioned constructs of the invention, either as transient transfection or as stable transfection. Preferably, the transfection is a stable transfection. The, thus, generated novel double-transgenic cell line described above allows for an improved assay for the measurement of the biological activity of neurotoxin polypeptides. As a result, the sensitivity of the assay for the determination of the neurotoxin polypeptide activity could be increased. Moreover, the assays of the invention save time and costs in comparison to assays described in the art. Said assays of the invention provide an alternative to methods for the detection of the biological activity of neutrotoxin polypeptides described in the art, such as for examples Western blotting which is a laborious method requiring up to two man days.

The method of the present invention can be assisted by automation. Specifically, in an aspect, step a) and /or b) may be assisted by robotic devices and automated reader systems for mixing compounds and measuring the reporter gene activity. Suitable systems are known in the art and depend on the type of response to be determined. Moreover, the method may comprise additional steps pertaining to the sample preparation or generation of the fusion polypeptide of the present invention.
The term "contacting" as used herein refers to bringing at least two different compounds in physical proximity as to allow physical and/or chemical interaction of said compounds. In the aforementioned method, a host cell comprising the fusion polypeptide of the invention and a reporter gene the expression of which is modulated by the transcription factor domain upon cleavage of the said fusion polypeptide is contacted with a sample suspected to comprise neurotoxin activity. The contacting shall be for a time and under conditions sufficient to allow cleavage of the neurotoxin cleavage site in the linker of the fusion polypeptide of the invention by the neurotoxin polypeptide comprised by the sample. Contacting as used herein, in an aspect, occurs in a host cell of the present invention containing the fusion polypeptide of the present invention. Thus, in an aspect, said polypeptide is comprised by a host cell and, in an aspect, the host cell of the present invention. The said time and conditions will be dependent on the amount of neurotoxin polypeptide comprised by the sample as well as on the uptake of the neurotoxin polypeptide by the host cell. The person skilled in the art is well aware of which conditions need to be applied dependent on the host cell, kind of sample, and kind of neurotoxin which shall be determined. In this aspect, contacting occurs in a cell system comprising the fusion polypeptide of the invention. The cell system shall allow for measuring the activity of the neurotoxin, upon contacting the system with a sample and, thus, allows for determining the neurotoxin protease activity in said sample. The term "sample" refers to a sample suspected to comprise neurotoxin polypeptide. The sample, in an aspect, is an aqueous solution. Such a sample may be a biological sample or may be a sample of an artificially generated aqueous solution. Such solutions, in an aspect, are obtained at different stages during neurotoxin manufacture, either for quality control and/or activity determination/specification purposes or for safety control. It is envisaged that the neurotoxin present in the said sample shall exhibit at least the neurotoxin protease activity. In another aspect, the neurotoxin is fully biologically active. In an aspect the said fully biologically active neurotoxin is required for entering the cell and for activating the read out based on the fusion polypeptide of the present invention. Accordingly, such a fully biologically active neurotoxin is to be applied if a host cell is to be contacted with the sample to be analyzed by the method of the invention. In another aspect, the sample to be applied for the method of the invention comprises neurotoxin polypeptides or fragments thereof which merely exhibit neurotoxin protease activity. Such neurotoxin polypeptides or fragments are, in an aspect, muteins of neurotoxin polypeptides comprising or consisting essentially of a proteolytically active light chain.

In an aspect, the reporter gene activity is determined by measuring the enzymatic conversion of a reporter gene substrate. The latter one can be measured in an aspect by detecting the intensity of the light emitted during the conversion reaction. Suitable systems for measuring the light emission that occurs during the conversion reaction catalyzed by reporter genes are well known in the art and commercially available. Moreover, suitable substrates which can be used for the reporter genes are also well known and commercially available.

The neurotoxin polypeptide in a sample can be determined quantitatively or qualitatively. For a qualitative determination, in an aspect of the invention, the presence or absence of a neurotoxin polypeptide is determined. For a quantitative detection, in an aspect, the amount of the neurotoxin polypeptide is determined. In an aspect, the quantitative determination encompasses a determination of the absolute amount or a relative amount, i.e. an amount which is normalized such that the amount found in different samples can be compared with each other. In an aspect, this can be achieved by comparison of a measured reporter gene activity for a test sample to a calibration curve which is to be established by subjecting calibration samples having predetermined amounts of the neurotoxin polypeptide to the method of the present invention.

In one aspect of the method of the invention, the host cell is a host cell as defined elsewhere herein.

The invention further relates to the use of the polynucleotide, the vector, the fusion polypeptide or the host cell of the invention for determining neurotoxin activity in a sample.

Finally, the invention pertains to a kit for determining neurotoxin activity comprising the polynucleotide, the vector, the fusion polypeptide or the host cell of the invention and, preferably, a detection agent for detecting reporter gene activity.

The term "kit" as used herein refers to a collection of means comprising the fusion polypeptide, the polynucleotide, the vector and/or the host cell of the present invention which are provided in separate or common vials in a ready to use manner for carrying out the method of the present invention. In an aspect, the kit comprises additional means for carrying out the method of the present invention, in an aspect, calibration standard solutions comprising neurotoxin polypeptide with defined biological activity and/or means for measuring the reporter gene activity such as detection agents for the reporter gene or substrates converted by the reporter gene. Furthermore, in an aspect, the kit comprises instructions for carrying out the method of the present invention. These instructions can be provided as a manual or can be in the form of a computer-implementable algorithm on a data storage medium which upon implementation is capable of governing one or more steps of the method of the invention. In an aspect, the kit is to be used for carrying out the method of the invention specified above.

### FIGURES

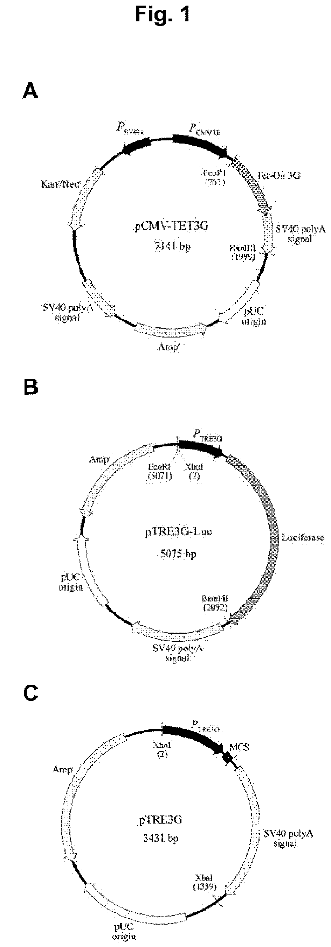

**Figure 1 A)** shows the original-regulator plasmid provided by Clontech, which was used as basis for the construction of activator-constructs; **B)** Luciferase-encoding Reporter-construct; **C)** System control plasmid for Luciferase-reporter construct (all: Clontech).

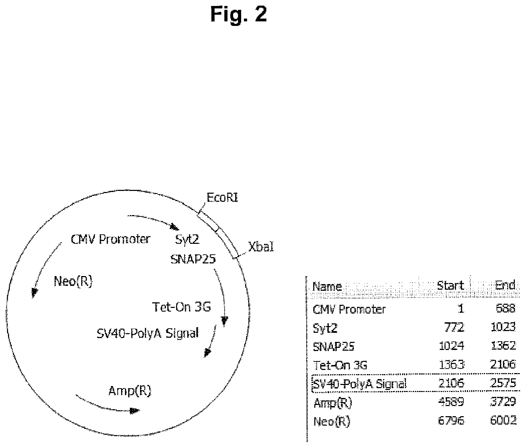

**Figure 2** shows one example for the construction of the activator-plasmid mST01 (SEQ ID NO: 9) with pCMV-Tet3G-Vector (Fig. 1A) as backbone. The claimed polynucleotide comprising the N-terminal membrane anchor domain of Synaptotagmin (87 amino acids) and the 112 C-terminal amino acids of SNAP25 is cloned via the plotted EcoRI and XbaI restriction-sites. The key-features of the vector are highlighted as arrows and denoted in the table on the right indicating their position (in bp).

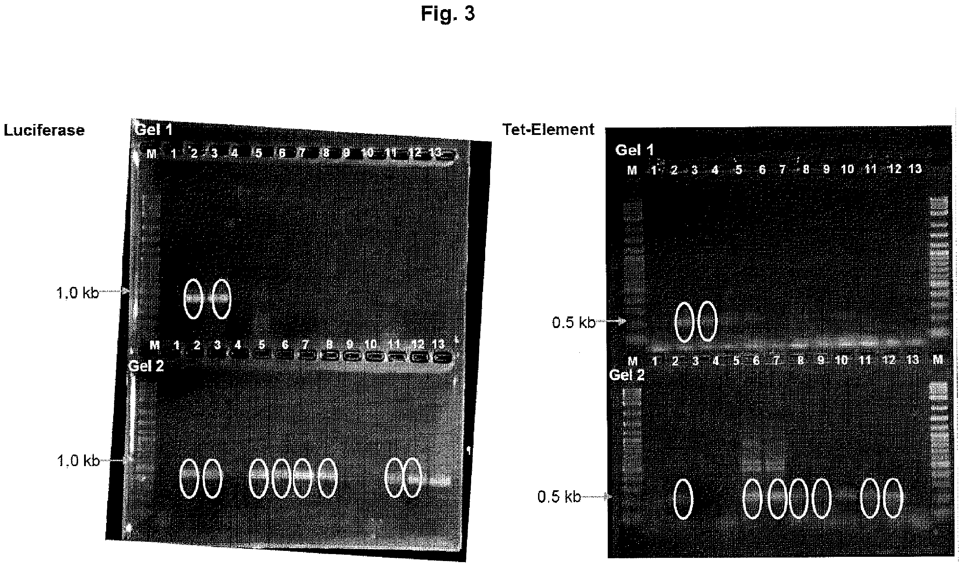

**Figure 3** shows the results of a genomic PCR as control for stable integration of the Activator- and Reporter construct. Primer designed for Luciferase yield a product of ∼1kb, those for Tet-Element ∼0.5kb. The highlighted lanes represent clones that stably integrated both plasmids. In total, 10 out of 24 generated clones were positive for Luciferase and Tet-Element, based on the results of the genomic PCR.

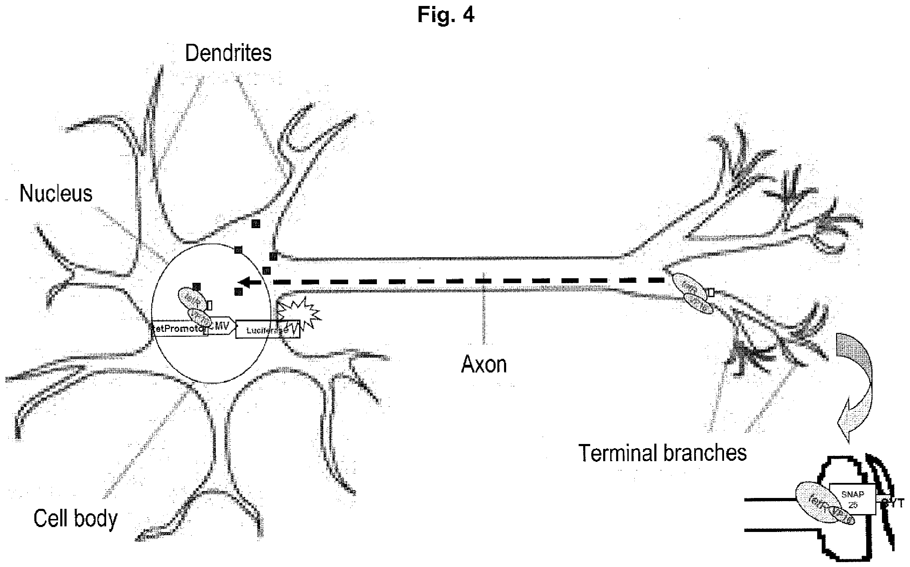

**Figure 4** shows a cartoon of the mode of action of the depicted example showing a membrane-anchored construct of Synaptotagmin-SNAP25 fusion protein.

### Abbreviations and Symbols:

- SYT:: Synaptotagmin;
- tetR:: Tetracycline-Repressor;
- VP16:: transcriptional activation domain of Herpes simplex VP16 protein;
- CMV:: Cytomegalie virus Promoter;

### Black squares: Doxycycline.

The fusion protein is constitutively expressed and preferably linked to the membrane of terminal branches of neuronal differentiated cells. Upon cleavage of BoNT within the respective linker domain, the complex consisting of TetR-Element and VP16 is transported into the nucleus. In presence of the antibiotic doxycycline, a conformational change is induced which allows for the binding of the TetR-Element/VP16 to the Tet-Promoter and its thereby regulated reporter gene luciferase. Upon induced expression of luciferase, a detection and quantification of its enzymatic activity using commercially available assay kits is the functional readout. It is also applicable for all membrane-anchored constructs. For cytosolic activator constructs, localization is not defined to a preferred domain within the cell. The fundamental mechanism of transport of the TetR-Element into the nucleus and activation of the subsequent expression/detection cascade of the reporter is consistent.

### EXAMPLE

The invention will now be described by the following example.

A sub-cloned SiMa cell line (SIMA no. 3B4), derived from parental SiMa cell line (DSMZ, Acc.No.: ACC-164) by subcloning using limited dilution, has been stably transfected using Xfect Transfection Reagent and accompanied products provided by vendor (Clontech Laboratories, Inc., Cat. No.: 631317). Transfection was performed in 6-well plates, at the time of transfection cells showed 90% confluency. The respective polynucleotide-DNA was used at 2µg per well. In case of transfection with the Luciferase-reporter-construct, said DNA was accompanied by 0.1µg linear selection marker (ratio 20:1) for Hygromycin. After 4h, medium containing transfection matrix was replaced by normal growth medium.
24h after this medium change, two 6-wells of the same preparation have been pooled and plated onto PDL-coated Petri-dishes (Ø14.5cm) and let grown for at least 48h (until density reached 70-80% confluence), before antibiotic selection (G418: 400µg/ml; Hygromycin: 150µg/ml) was applied until single colonies were formed. Those single colonies have been picked, expanded and subjected to testing for stable genomic integration of the respective construct of interest. After selection period antibiotic concentrations have been lowered to G418: 200µg/ml and Hygromycin: 100µg/ml.
Genomic PCR (cells were lysed using Lyse and Go PCR reagent, Thermo Scientific according to protocol) using specific primers for the Tet-Element (forward: AGCTGGGAGTTGAGCAGCCTAC (SEQ ID NO: 35); reverse: GGTGCCGAGATGCACTTTAGCC (SEQ ID NO: 36)) and Luciferase-Reporter (forward: GAAGAGATACGCCCTGGTTC (SEQ ID NO: 37); reverse: CGGGAGGTAGATGAGATGTG (SEQ ID NO: 38)) applying Phusion High Fidelity Kit (NEB) according to protocol and analysis by subsequent agarose gel electrophoresis.
After having been tested positively for the insertion of both constructs, cells have been expanded and cell bank stocks in liquid nitrogen tanks have been generated. Tests for stable insertion after re-thawing of stocks have been performed before using cells for experiments.
In the first place, construct mST01 (Synaptotagmin-SNAP-25-tTA) (SEQ ID NO: 9) was transfected and single cell subcloning was performed to reach a clonal background with as little genetic variation as possible. These stable clones were subjected to a second round of transfection with the reporter gene construct pTRE-Luc (luciferase reporter) and repeated subsequent single cell cloning (Fig. 3). Transfectional, viability and functional controls were made by using the empty vectors (pCMV-Tet3G as activator control plasmid and pTRE3G as reporter control plasmid) without the respective GOI and did not show any impairment in the above mentioned features (Fig. 1A, C) (Clontech).
After showing the stability of integration, the expression analysis of Tet-Element and cleavage of the BoNT linker-domain by neurotoxin is assessed. For this experiment, double-transfected clones are plated onto PDL-coated 96-well plates and differentiated to a neuronal phenotype according to the procedures disclosed in WO 2010/105234. Intoxication with BoNT/A is carried out using a concentration series with final concentrations in cell culture ranging from 1x10⁻¹⁰M to 10x10⁻¹⁴M for 24-72 hours. The stock dilution series of BoNT is performed in DPBS supplemented with 0.2% human serum albumin and applied correspondingly and directly into the cell culture medium. After intoxication, the release of Tet-Repressor protein will be detectable and distinguishable from the original polypeptide sequence. Further on the expression level of the Luciferase-reporter is assessed in time and dose-response to BoNT as well as in a time and dose response level to doxycycline as a Tet-on System is used as basis. Functional readout is done by using commercially available Luciferase-Assay-Kits. In this case, two different kits can be applied, mainly for a purely functional read out a pure Luciferase Assay System (One-Glo, Promega) and for full assay application a Luciferase Assay System, in combination with assessment of cell viability (One-Glo+Tox, Promega). This multiplexing approach allows for the normalization and functional data within the same samples and provides, therefore, for a solid basis for validation of the assay.

## Claims

1. A polynucleotide encoding a fusion polypeptide comprising (i) a tetracycline dependent transactivator (tet-repressor-VP16) and (ii) synaptotagmin, separated by a linker comprising a neurotoxin cleavage site.

2. A vector comprising a polynucleotide of claim 1.

3. A fusion polypeptide encoded by the polynucleotide of claim 1.

4. A host cell comprising the polynucleotide of claim 1, the vector of claim 2 or the fusion polypeptide of claim 3.

5. The host cell of claim 4, wherein said host cell is selected from the group consisting of: primary neuronal cells, neuroblastoma cell lines, cell line N1E-115, cell line Neuro2a, cell line SH-SY5Y, cell line PC12, cell line SiMa, cell line MHH-NB-11, cell line SK-N-BE(2) and induced pluripotent stem cells.

6. The host cell of claim 4 or 5, wherein said host cell comprises a reporter gene the expression of which is modulated by the tetracycline dependent transactivator (tet-repressor-VP16) upon cleavage of the fusion polypeptide.

7. A method for determining neurotoxin activity in a sample comprising the steps of:
(a) contacting a host cell comprising the fusion polypeptide of claim 3 and a reporter gene the expression of which is modulated by the tetracycline dependent transactivator (tet-repressor-VP16) upon cleavage of the said fusion polypeptide with a sample suspected to comprise neurotoxin activity; and
(b) measuring the reporter gene activity, whereby neurotoxin activity in the sample is determined.

8. The method of claim 7, wherein said host cell is a host cell of any one of claims 4 to 6.

9. Use of the polynucleotide of claim 1, the vector of claim 2, the fusion polypeptide of claim 3 or the host cell of claim 4 or 6 for determining neurotoxin activity in a sample.

10. A kit for determining neurotoxin activity comprising the polynucleotide of claim 1, the vector of claim 2, the fusion polypeptide of claim 3 or the host cell of claim 4 or 6 and a detection agent for detecting reporter gene activity.

## Patentansprüche

1. Polynukleotid, das ein Fusionspolypeptid kodiert, umfassend (i) einen Tetracyclin-abhängigen Transaktivator (tet-Repressor-VP16) und (ii) Synaptotagmin, die durch einen Linker getrennt sind, der eine Neurotoxin-Spaltstelle umfasst.

2. Vektor, umfassend ein Polynukleotid nach Anspruch 1.

3. Fusionspolypeptid, das von dem Polynukleotid nach Anspruch 1 kodiert wird.

4. Wirtszelle, umfassend das Polynukleotid nach Anspruch 1, den Vektor nach Anspruch 2 oder das Fusionspolypeptid nach Anspruch 3.

5. Wirtszelle nach Anspruch 4, wobei die Wirtszelle ausgewählt ist aus der Gruppe bestehend aus: primären neuronalen Zellen, Neuroblastomzelllinien, der Zelllinie N1E-115, der Zelllinie Neuro2a, der Zelllinie SH-SY5Y, der Zelllinie PC12, der Zelllinie SiMa, der Zelllinie MHH-NB-11, der Zelllinie S-N-BE(2) und induzierten pluripotenten Stammzellen.

6. Wirtszelle nach Anspruch 4 oder 5, wobei die Wirtszelle ein Reportergen umfasst, dessen Expression durch den Tetracyclin-abhängigen Transaktivator (tet-Repressor-VP16) nach der Spaltung des Fusionspolypeptids moduliert wird.

7. Verfahren zur Bestimmung der Neurotoxinaktivität in einer Probe, umfassend die Schritte:
(a) Zusammenbringen einer Wirtszelle, die das Fusionspolypeptid nach Anspruch 3 und ein Reportergen umfasst, dessen Expression durch den Tetracyclin-abhängigen Transaktivator (tet-Repressor-VP16) nach der Spaltung des Fusionspolypeptids moduliert wird, mit einer Probe, von der vermutet wird, dass sie Neurotoxinaktivität umfasst; und
(b) Messen der Reportergenaktivität, wodurch die Neurotoxinaktivität in der Probe bestimmt wird.

8. Verfahren nach Anspruch 7, wobei die Wirtszelle eine Wirtszelle nach einem der Ansprüche 4 bis 6 ist.

9. Verwendung des Polynukleotids nach Anspruch 1, des Vektors nach Anspruch 2, des Fusionspolypeptids nach Anspruch 3 oder der Wirtszelle nach Anspruch 4 oder 6 zur Bestimmung der Neurotoxinaktivität in einer Probe.

10. Kit zur Bestimmung der Neurotoxinaktivität, umfassend das Polynukleotid nach Anspruch 1, den Vektor nach Anspruch 2, das Fusionspolypeptid nach Anspruch 3 oder die Wirtszelle nach Anspruch 4 oder 6 und ein Nachweismittel zum Nachweisen einer Reportergenaktivität.

## Revendications

1. Polynucléotide codant pour un polypeptide de fusion comprenant (i) un transactivateur dépendant de la tétracycline (tet-répresseur-VP16) et (ii) la synaptotagmine, séparés par un lieur comprenant un site de clivage de neurotoxine.

2. Vecteur comprenant un polynucléotide selon la revendication 1.

3. Polypeptide de fusion codé par le polynucléotide selon la revendication 1.

4. Cellule hôte comprenant le polynucléotide selon la revendication 1, le vecteur selon la revendication 2 ou le polypeptide de fusion selon la revendication 3.

5. Cellule hôte selon la revendication 4, ladite cellule hôte étant choisie dans le groupe constitué des : cellules neuronales primaires, lignées cellulaires de neuroblastome, lignée cellulaire NIE-115, lignée cellulaire Neuro2a, lignée cellulaire SH-SY5Y, lignée cellulaire PC12, lignée cellulaire SiMa, lignée cellulaire MHH-NB-11, lignée cellulaire SK-N-BE(2) et cellules souches pluripotentes induites.

6. Cellule hôte selon la revendication 4 ou 5, ladite cellule hôte comprenant un gène rapporteur dont l'expression est modulée par le transactivateur dépendant de la tétracycline (tet-répresseur-VP16) après clivage du polypeptide de fusion.

7. Procédé de détermination de l'activité de neurotoxine dans un échantillon comprenant les étapes de :
(a) mise en contact d'une cellule hôte comprenant le polypeptide de fusion selon la revendication 3 et un gène rapporteur dont l'expression est modulée par le transactivateur dépendant de la tétracycline (tet-répresseur-VP16) après clivage dudit polypeptide de fusion avec un échantillon suspecté de comprendre une activité de neurotoxine ; et
(b) mesure de l'activité du gène rapporteur, de sorte que l'activité neurotoxine dans l'échantillon soit déterminée.

8. Procédé selon la revendication 7, dans lequel ladite cellule hôte est une cellule hôte selon l'une quelconque des revendications 4 à 6.

9. Utilisation du polynucléotide selon la revendication 1, du vecteur selon la revendication 2, du polypeptide de fusion selon la revendication 3 ou de la cellule hôte selon la revendication 4 ou 6 pour déterminer l'activité de neurotoxine dans un échantillon.

10. Kit pour déterminer l'activité de neurotoxine comprenant le polynucléotide selon la revendication 1, le vecteur selon la revendication 2, le polypeptide de fusion selon la revendication 3 ou la cellule hôte selon la revendication 4 ou 6 et un agent de détection pour détecter l'activité de gène rapporteur.
